# EUROPÄISCHE PATENTSCHRIFT

(11) **EP 0 663 941 B1**
(45) Veröffentlichungstag und Bekanntmachung des Hinweises auf die Patenterteilung: **28.10.1998**
(21) Anmeldenummer: 94925420.5
(22) Anmeldetag: 01.08.1994
(51) Int. Cl.: C09K 19/30, C09K 19/32, C09K 19/34, C07C 255/50, C07C 25/18, C07C 23/18, C07C 43/225

(54) **2-FLUORCYCLOYHEXEN-DERIVATE**
2-FLUOROCYCLOHEXENE DERIVATIVES
DERIVES DE 2-FLUOROCYCLOHEXENE

(30) Priorität: 06.08.1993 DE 4326420
(43) Veröffentlichungstag der Anmeldung: 26.07.1995
(73) Patentinhaber: MERCK PATENT GmbH, 64293 Darmstadt (DE)
(72) Erfinder: REIFFENRATH, Volker, D-64380 Rossdorf (DE); PLACH, Herbert, D-64287 Darmstadt (DE)
(86) Internationale Anmeldenummer: EP9402546
(87) Internationale Veröffentlichungsnummer: WO9504790

(56) Entgegenhaltungen:
- DE-A- 4 035 509
- DE-A- 4 227 772
- PATENT ABSTRACTS OF JAPAN vol. 18, no. 122 (C-1173) 28. Februar 1994 & JP,A,53 010 616 (TAKEHARA SADAO) 22. November 1993 in der Anmeldung erwähnt & JP,A,5 310 616 (..)
- DATABASE WPI Week 8910, Derwent Publications Ltd., London, GB; AN 89-071659 & JP,A,1 022 830 (DAINIPPON) 25. Januar 1989

## Beschreibung

Die vorliegende Erfindung betrifft 2-Fluorcyclohexen-Derivate der Formel I, 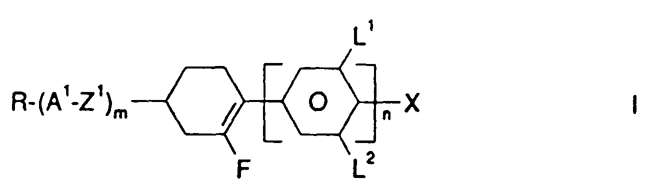 wobei
- R: ein unsubstituierter oder mit CN oder mit mindestens einem Halogen substituierter Alkyl- oder Alkenylrest mit bis zu 18 C-Atomen, worin eine oder mehrere nicht benachbarte CH₂-Gruppen durch einen Rest ausgewählt aus der Gruppe -O-, -S-, -CO-, -O-CO-, -CO-O- und -C≡C- ersetzt sein können,
- A¹: a) einen 1,4-Phenylenrest, worin eine oder zwei CH-Gruppen durch N ersetzt sein können,
b) einen 1,4-Cyclohexylenrest, worin eine oder zwei nicht benachbarte CH₂-Gruppen durch -O- oder -S- ersetzt sein können,
c) einen 1,4-Cyclohexenylen-, einen Piperidin-1,4-diyl-, einen 1,4-Bicyclo[2,2,2]-octylen- oder einen Naphthalin-2,6-diylrest
wobei die Reste a) und b) ein oder mehrfach durch Halogenatome, Cyano- und/oder Methylgruppen substituiert sein können,
- Z¹: -CH₂CH₂-, -CO-O-, -O-CO-, -CH=CH-, -OCH₂-, -CH₂O-, -C≡C-, -(CH₂)₄-, -CH=CH-CH₂CH₂- oder eine Einfachbindung,
- X: Alkyl oder Alkoxy mit 1-12 C-Atomen, NCS oder Q-Y, wobei Q -O-, -S- oder eine Einfachbindung und Y CN, F, Cl oder halogeniertes Alkyl oder Alkenyl mit 1-5 C-Atomen ist,
- L¹ und L²: jeweils unabhängig voneinander H oder F,
- m: 0, 1 oder 2 und
- n: 0, 1 oder 2, wobei m + n ≥ 1 ist,
bedeuten, mit der Maßgabe, daß im Fall m = 0, n = 1 und X = Alkyl, Alkoxy, F, Cl oder CN L¹ und L² Fluor bedeuten.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle einschließlich deren hochverdrillten Varianten, wie z.B. STN oder SBE, dem Guest-Host-Effekt, dem Effekt der Deformation aufgerichteter Phasen oder dem Effekt der dynamischen Streuung beruhen, insbesondere für Matrix-Flüssigkristallanzeigen (MFK-Anzeigen).

Der Erfindung lag die Aufgabe zugrunde, neue stabile flüssigkristalline oder mesogene Verbindungen aufzufinden, die als Komponenten flüssigkristalliner Medien geeignet sind und insbesondere eine vergleichsweise geringe Viskosität besitzen sowie eine hohe positive dielektrische Anisotropie.

Verbindungen der Formel 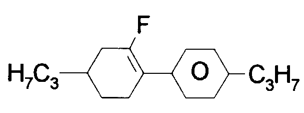 sind aus der JP 5310616 bekannt. Desweiteren offenbart die JP 5310616 Alkohole der Struktur 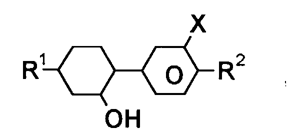 worin X H oder F bedeutet.

Es wurde nun gefunden, daß Verbindungen der Formel I als Komponenten flüssigkristalliner Phasen vorzüglich geeignet sind. Insbesondere verfügen sie über vergleichsweise niedrige Viskositäten. Mit ihrer Hilfe lassen sich stabile flüssigkristalline Phasen mit breitem Mesophasenbereich vorteilhaften Werten für die optische und dielektrische Anisotropie erhalten, welche sich gleichzeitig durch sehr günstige Werte für den spezifischen Widerstand auszeichnen. Hierdurch lassen sich insbesondere bei Medien für Matrix-Flüssigkristallanzeigen (MFK-Anzeigen) oder Supertwistdisplays deutliche Vorteile erzielen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität und/oder dessen spezifischen Widerstand zu optimieren.

Die Verbindungen der Formel I sind in reinem Zustand farblos und bilden flüssigkristalline Mesophasen in einem für die elektrooptische Verwendung günstig gelegenen Temperaturbreich. Chemisch, thermisch und gegen Licht sind sie stabil.

Gegenstand der Erfindung sind somit die Verbindungen der Formel I, insbesondere die Verbindungen der Formel I, worin n + m = 2 ist und/oder L¹ und L² F bedeuten.

Gegenstand der Erfindung ist weiterhin die Verwendung dieser Verbindungen als Komponenten flüssigkristalliner Medien.

Gegenstand der Erfindung sind ferner flüssigkristalline Medien mit einem Gehalt an mindestens einer Verbindung der Formel I sowie Flussigkristallanzeigeelemente, insbesondere elektrooptische Anzeigeelemente, insbesondere Matrix-Flüssigkristallanzeigen, die derartige Medien enthalten.

Der Einfachheit halber bedeuten im folgenden A² 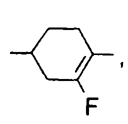 Cyc einen 1,4-Cyclohexylen- oder einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest und Bi einen Bicyclo(2,2,2)-octylenrest, wobei Cyc und/oder Phe unsubstituiert oder ein- oder zweifach durch F oder CN substituiert sein können.

Die Verbindungen der Formel I umfassen dementsprechend Verbindungen mit zwei oder drei Ringen der Teilformeln Ia bis Ip:

R-A²-Phe-X Ia

R-Cyc-A²-Phe-X Ib

R-Phe-A²-Phe-X Ic

R-Dio-A²-Phe-X Id

R-Dit-A²-Phe-X Ie

R-Pyd-A²-Phe-X If

R-Pyr-A²-Phe-X Ig

R-Bi-A²-Phe-X Ih

R-A²-Phe-Phe-X Ii

R-Phe-Z¹-A²-Phe-X Ij

R-Cyc-Z¹-A²-Phe-X Ik

R-Dio-Z¹-A²-Phe-X Il

R-Dit-Z¹-A²-Phe-X Im

R-Pyd-Z¹-A²-Phe-X In

R-Pyr-Z¹-A²-Phe-X Io

R-Bi-Z¹-A²-Phe-X Ip

Verbindungen mit vier Ringen der Teilformeln Iq-Iu:

R-A¹-A¹-A²-Phe-X Iq

R-A¹-Z¹-A¹-A²-Phe-X Ir

R-A¹-Z¹-A¹-Z¹-A²-Phe-X Is

R-A¹-A²-Phe-Phe-X It

R-A¹-Z¹-A²-Phe-Phe-X Iu

In den Verbindungen der Teilformeln Ia bis Iu, die eine oder mehrere Brücken Z¹ aufweisen, bedeuten die Brückenglieder vorzugsweise -CH₂CH₂-, -CO-O-, -O-CO-, -CH₂O-, -OCH₂- oder -C≡C-, insbesondere -CH₂CH₂-.

R bedeutet vorzugsweise geradkettiges Alkyl, ferner Alkoxy und Alkenyl.

Bevorzugt enthalten die Verbindungen der Formel I nicht mehr als einen der Reste Bi, Pyd, Pyr, Dio oder Dit.

Besonders bevorzugte Verbindungen sind die Fluorcyclohexen-Derivate der Formeln I1 bis I36:

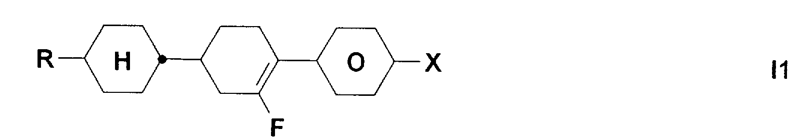

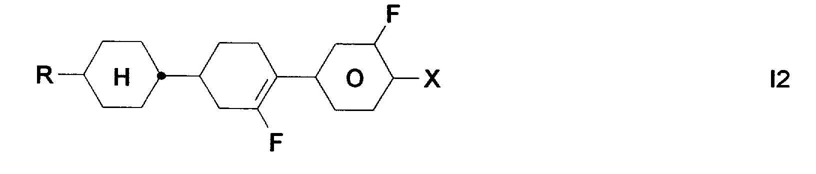

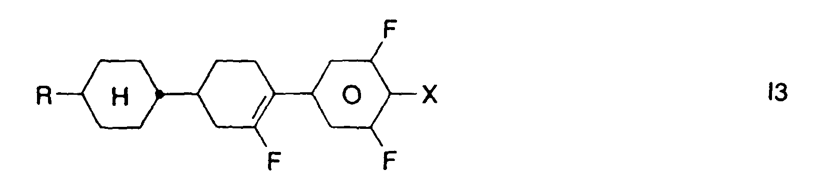

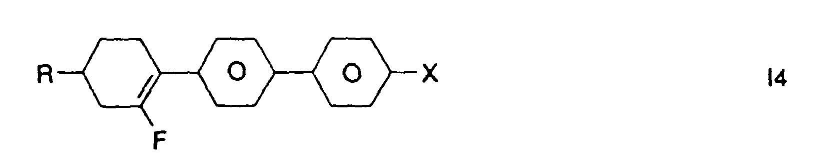

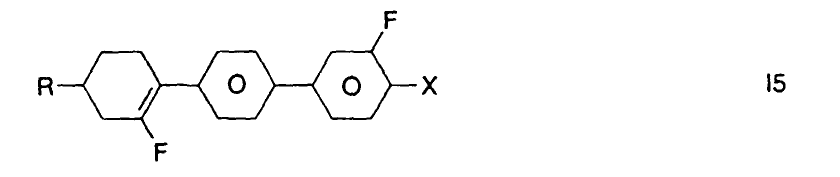

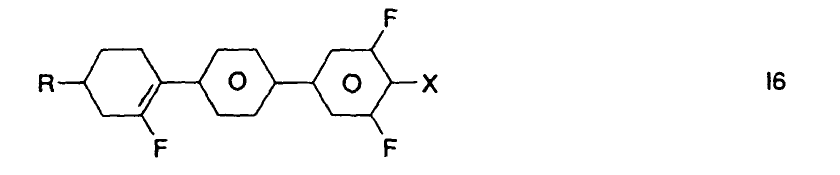

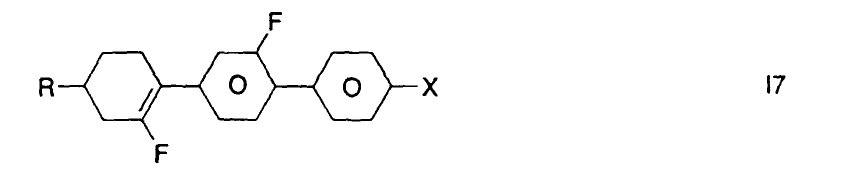

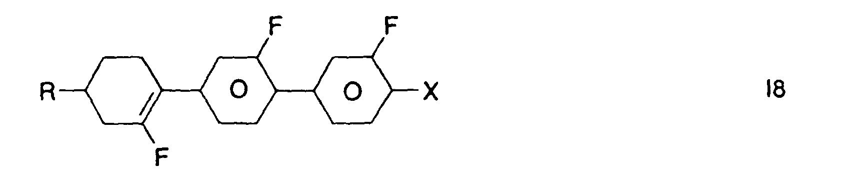

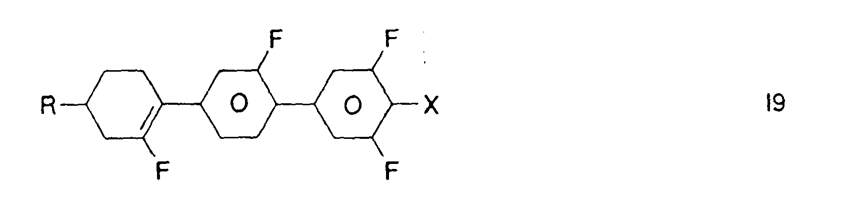

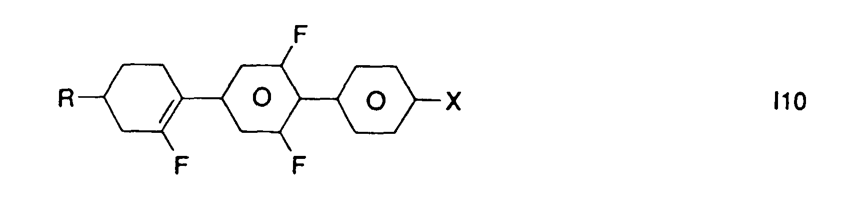

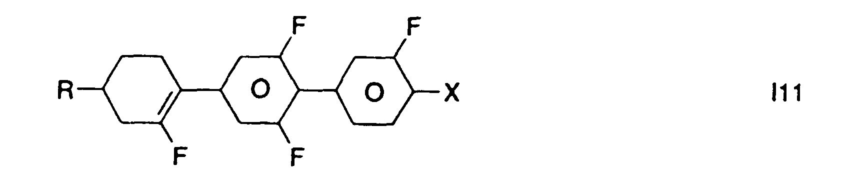

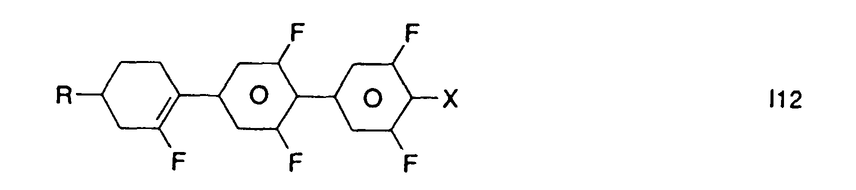

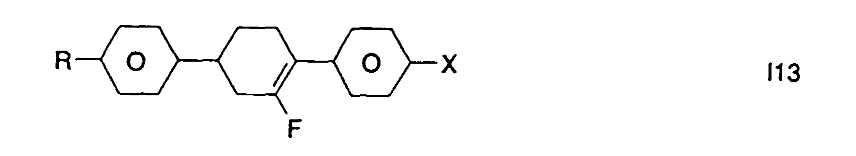

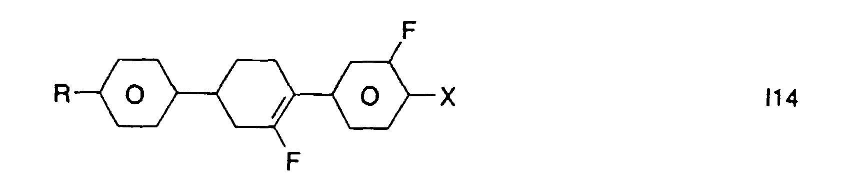

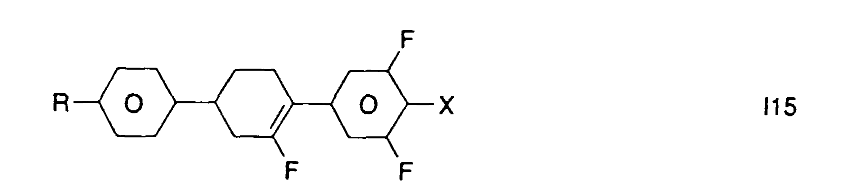

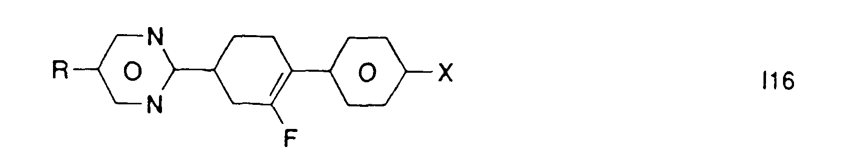

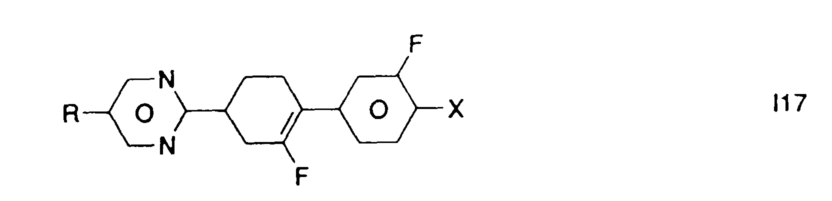

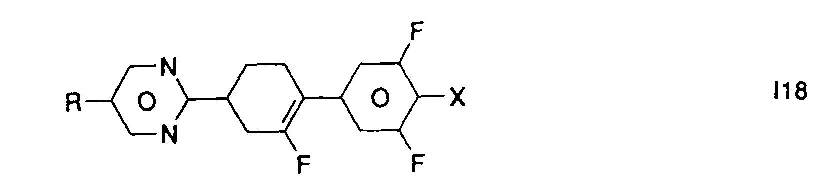

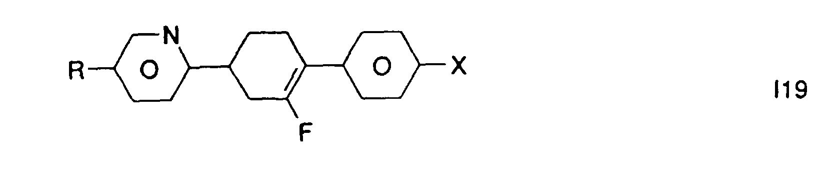

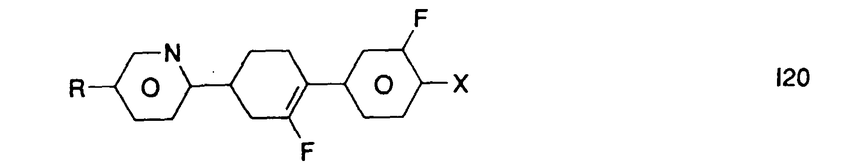

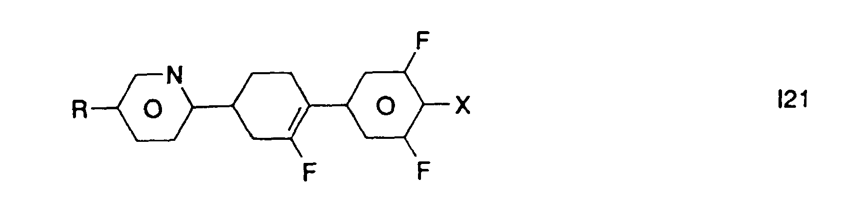

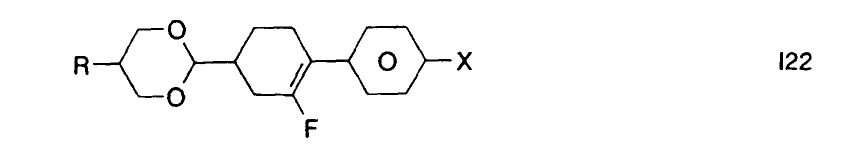

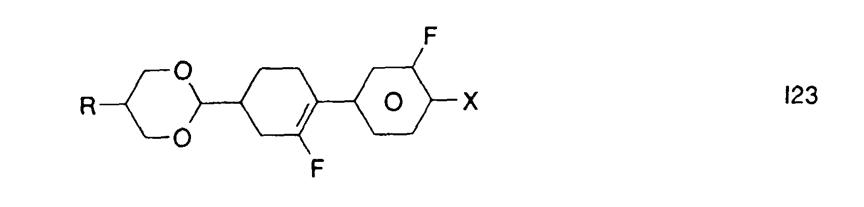

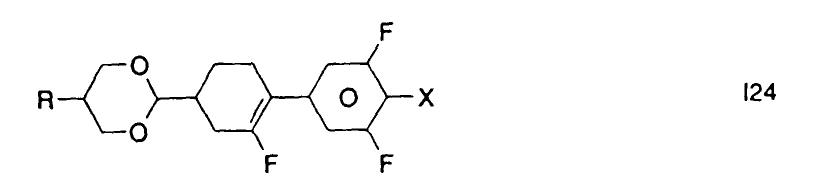

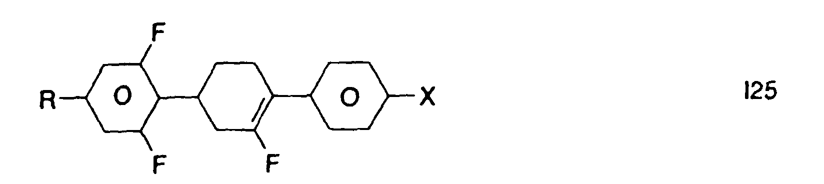

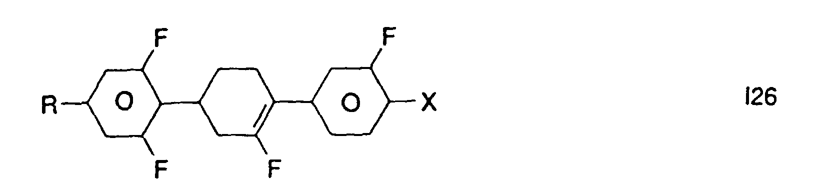

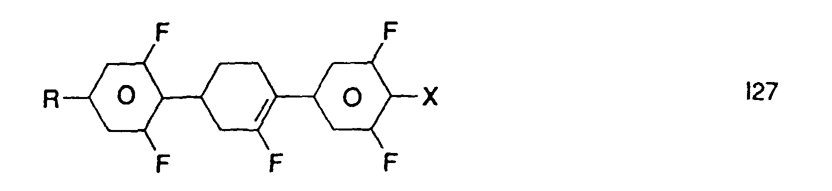

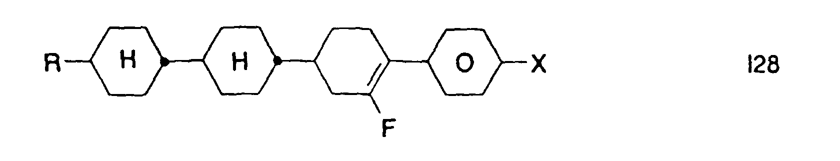

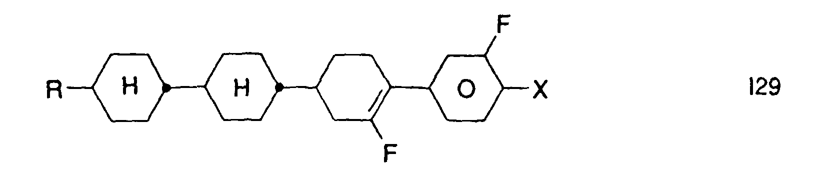

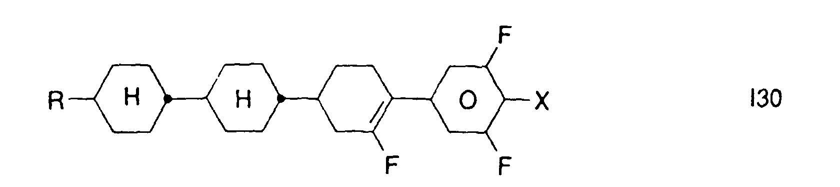

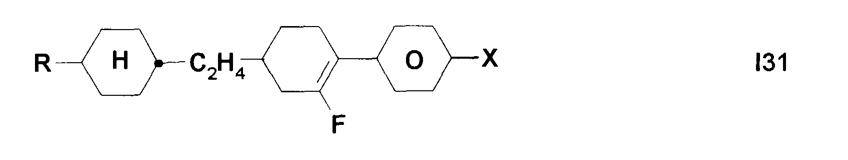

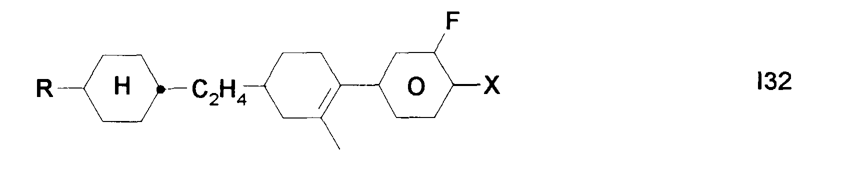

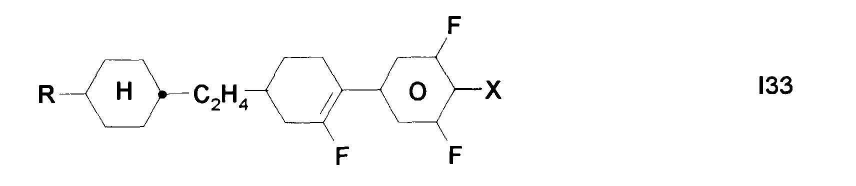

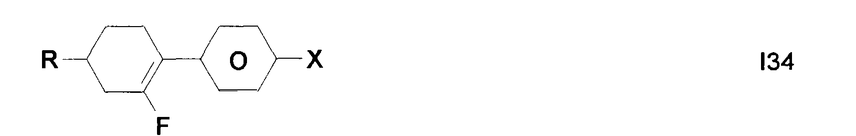

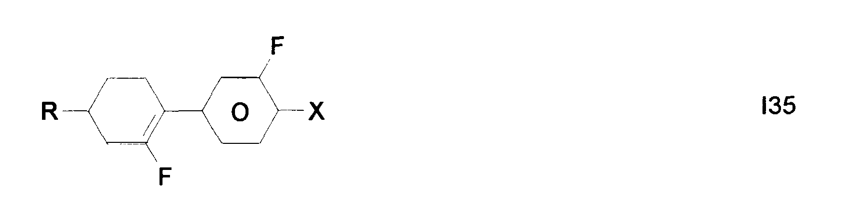

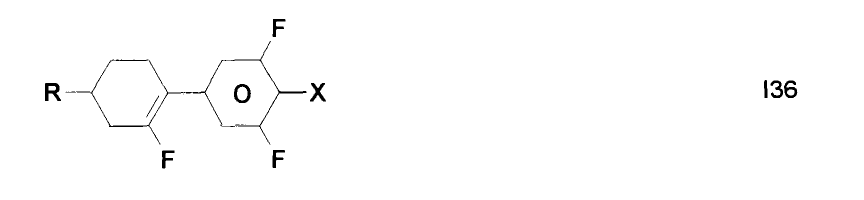

In den Verbindungen der Formel I bedeutet X vorzugsweise CN, NCS, OCN, F, Cl, CF₃, OCF₃, CF₂H, CHF₂, CF₂Cl, OCF₂H, OCF₂Cl, OCH₂CF₃, OCHFCF₃, OCF₂CF₃, OCH₂CF₂H, OCH=CF₂, OCF=CF₂, OCF=CCl₂, OCH₂CF₂Cl, CH₂CF₃, CH₂CF₂H, OCF=CH₂, CH=CF₂, CF=CF₂, CF=CH₂, OCH₃ oder C₂H₅.

Insbesondere bevorzugt sind Verbindungen worin X F, Cl, CN, OCF₃, OCHFCF₃, OCHF₂, OCH₂CF₃, OCF=CF₂ oder OCH=CF₂ bedeutet.

Bevorzugt sind auch Verbindungen der Formel I, in denen die im Molekül enthaltenen 1,4-Phenylenringe ein- oder zweifach durch Fluor substituiert sind. Insbesondere sind dies 2-Fluor-1,4-phenylen sowie 2,6-Difluor-1,4-phenylen.

Falls R einen Alkylrest oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- = Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6 oder 7-Oxaoctyl, 2-, 3-, 5-, 6-, 7- oder 8-Oxanonyl, 2-, 3-, 4-, 5-, 6-, 7-, 8 oder 9-Oxadecyl.

Falls R einen Alkenylrest bedeutet, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O- oder eine Oxycarbonylgruppe -O-CO-. Vorzugsweise sind diese geradkettig und haben 2 bis 6 C-Atome. Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkenylrest bedeutet, in dem eine CH₂-Gruppe durch CO oder CO-O oder O-CO- ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Verbindungen der Formel I, die über für Polymerisationsreaktionen geeignete Flügelgruppen R verfügen, eignen sich zur Darstellung flüssigkristalliner Polymerer.

Verbindungen der Formeln I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 4-Methylhexyl, 2-Nonyl, 2-Decyl, 2-Dodecyl, 6-Methyloctoxy, 6-Methyloctanoyloxy, 5-Methylheptyloxycarbonyl, 2-Methylbutyryloxy, 3-Methylvaleryloxy, 4-Methylhexanoyloxy, 2-Chlorpropionyloxy, 2-Chlor-3methylbutyryloxy, 2-Chlor-4-methylvaleryloxy, 2-Chlor-3-methylvaleryloxy, 2-Methyl-3-oxapentyl, 2-Methyl-3-oxahexyl.

Falls R einen Alkylrest darstellt, in dem zwei oder mehr CH₂-Gruppen durch -O- und/oder -CO-O- ersetzt sind, so kann dieser geradkettig oder verzweigt sein. Vorzugsweise ist er verzweigt und hat 3 bis 12 C-Atome.

Er bedeutet demnach besonders Bis-carboxy-methyl, 2,2-Bis-carboxyethyl, 3,3-Bis-carboxy-propyl, 4,4-Bis-carboxy-butyl, 5,5-Bis-carboxypentyl, 6,6-Bis-carboxy-hexyl, 7,7-Biscarboxy-heptyl, 8,8-Bis-carboxyoctyl, 9,9-Bis-carboxynonyl, 10,10-Bis-carboxydecyl, Bis-(methoxycarbonyl)-methyl, 2,2-Bis-(methoxycarbonyl)-ethyl, 3,3-Bis-(methyoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Verbindungen der Formel I, die über die Polykondensationen geeignete Flügelgruppen R verfügen, eignen sich zur Darstellung flüssigkristalliner Polykondensate.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Die 1,4-Cyclohexenylen-Gruppe hat vorzugsweise folgende Strukturen:

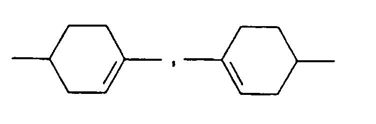

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die erfindungsgemäßen Fluorcyclohexen-Derivate der Formel I können z.B. wie folgt hergestellt werden:

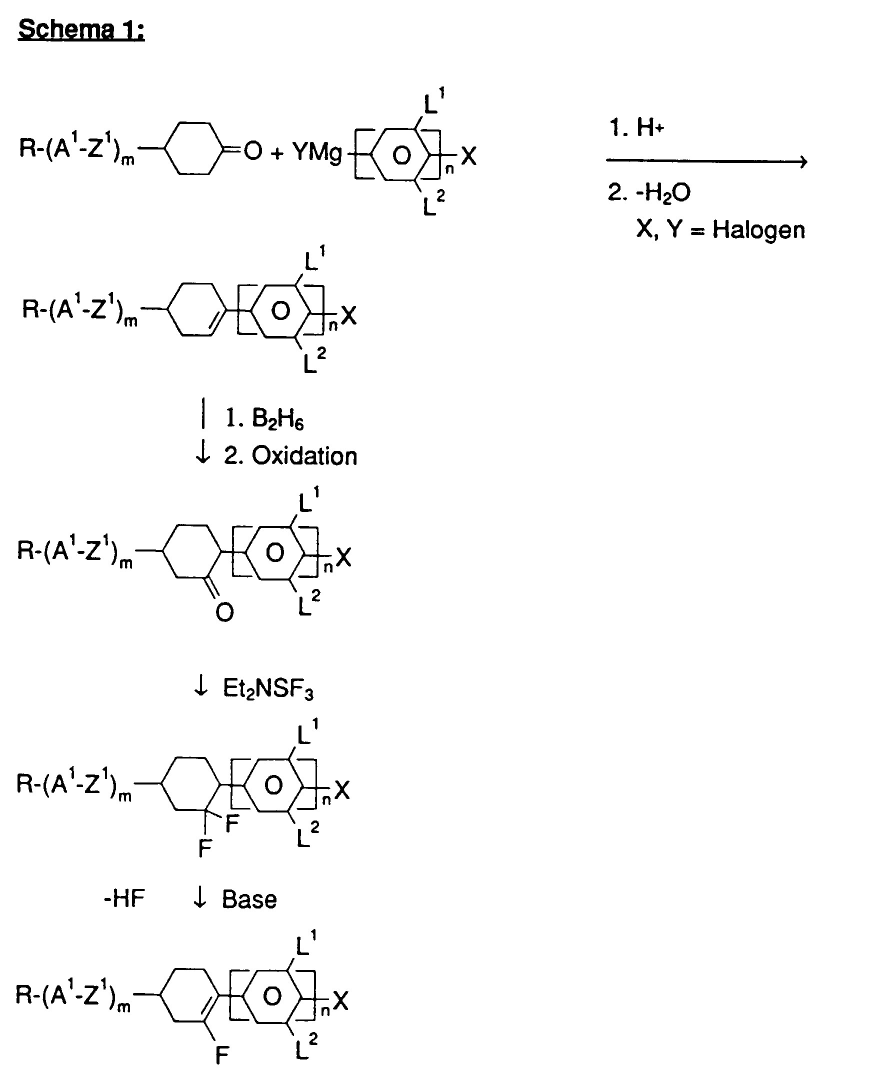

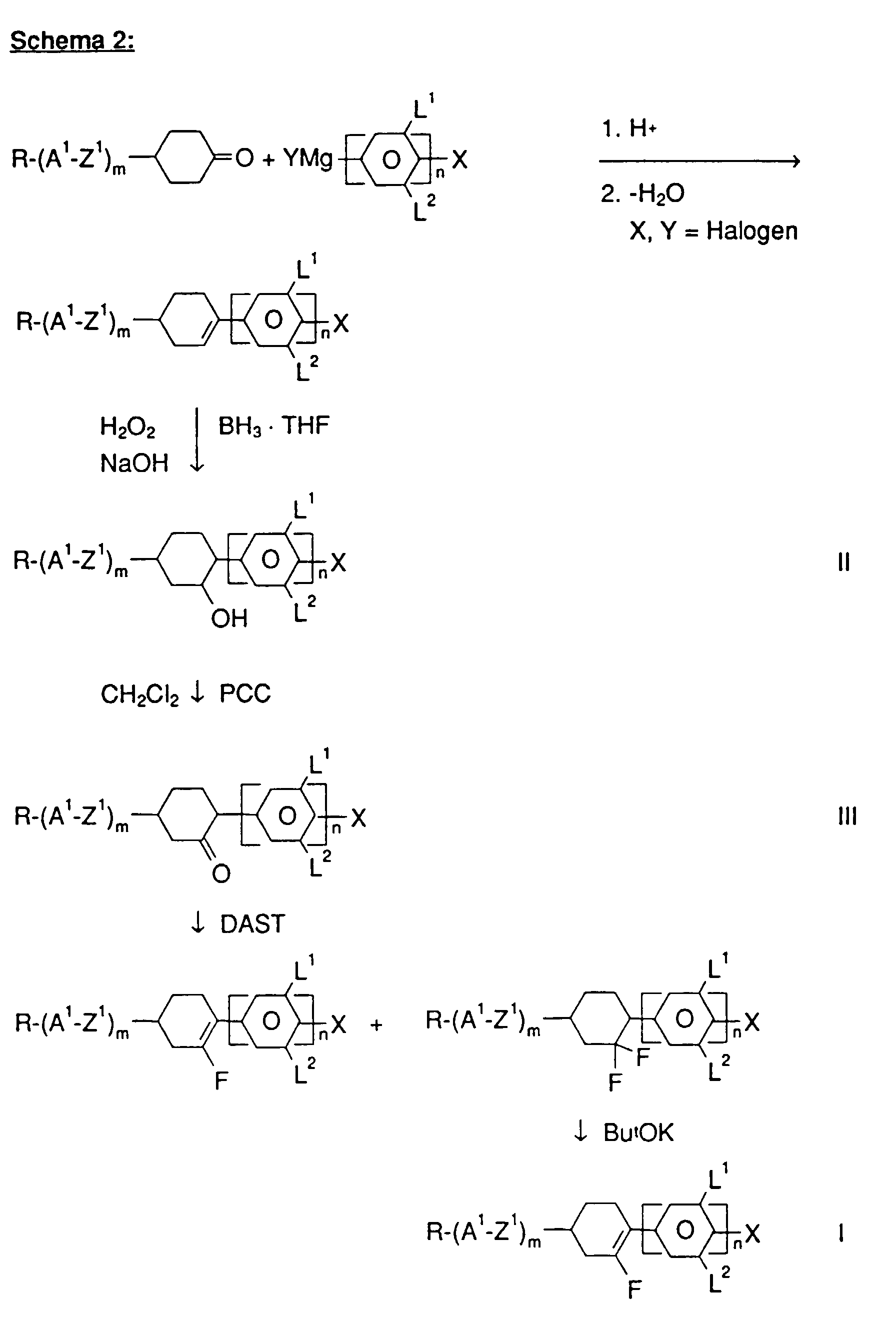

Verbindungen der Formel II und III, worin R, A¹, Z¹, L¹, L², X und m die in Anspruch 1 angegebene Bedeutung haben und n 1 oder 2 ist, sind ebenfalls Gegenstand der Erfindung.

Die Synthese einiger besonders bevorzugter Verbindungen ist im folgenden näher angegeben:

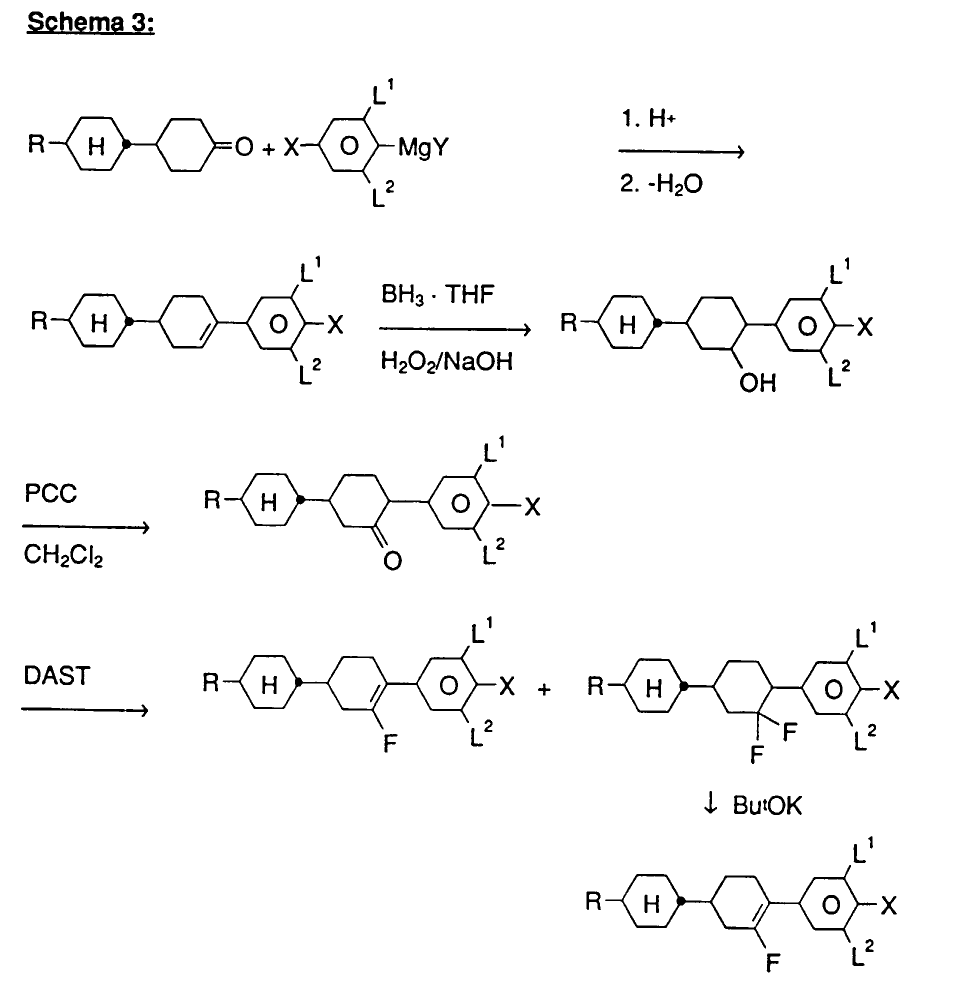

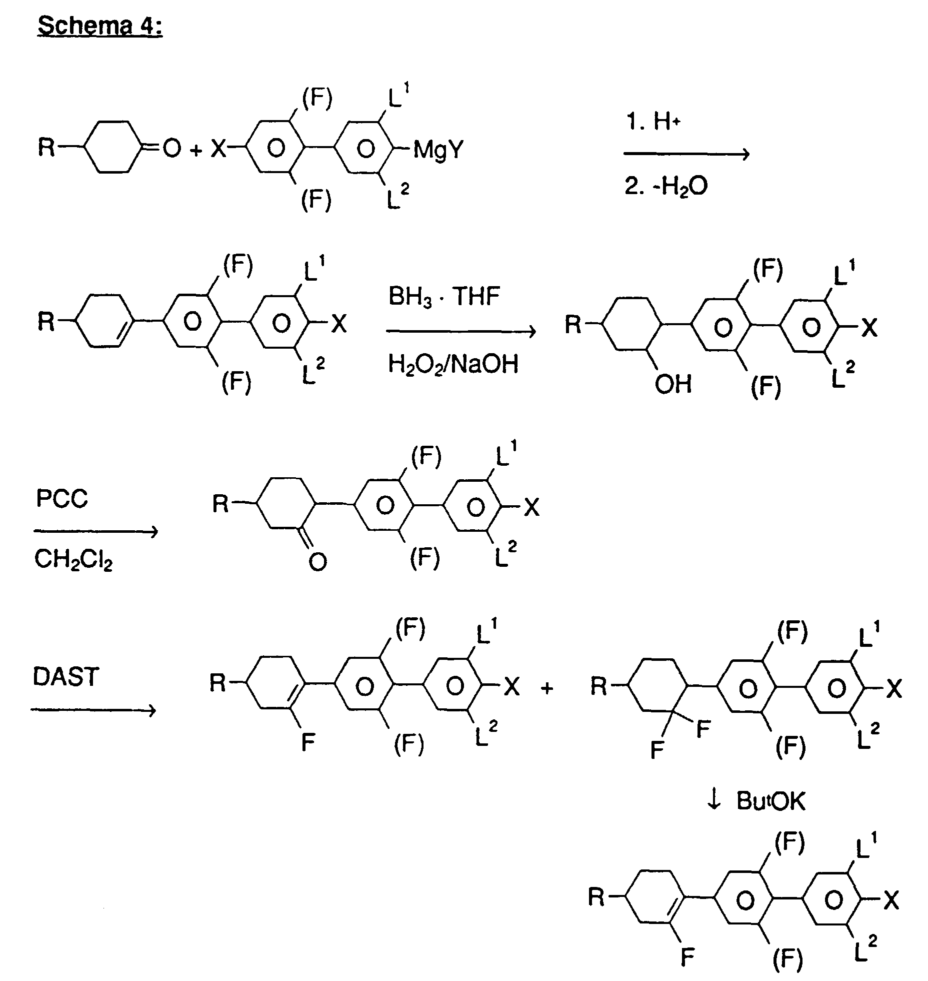

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäurephenyl- oder cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane; Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether; Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-,-Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cydohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr.

E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R" voneinander verschieden, wobei einer dieser Reste meist Alkyl oder Alkenyl ist. In den Verbindungen der Teilformeln 1b, 2b, 3b, 4b und 5b bedeutet R" -CN, -CF₃, F, Cl oder -NCS; R hat dabei die bei den Verbindungen der Teilformeln 1a bis 5a angegebene Bedeutung und ist vorzugsweise Alkyl oder Alkenyl. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90 %,
Gruppe 2: 10 bis 80 %, insbesondere 10 bis 50 %,
wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/ R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Insbesondere eignen sich die erfindungsgemäßen Medien zur Verwendung in MFK-Anzeigen.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nF.F.F | CₙH₂ₙ₊₁ | F | F | H |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-C₂H₂ₛ- | CN | H | H |
| nAm | CₙH₂ₙ₊₁ | COOCₘH₂ₘ₊₁ | H | H |

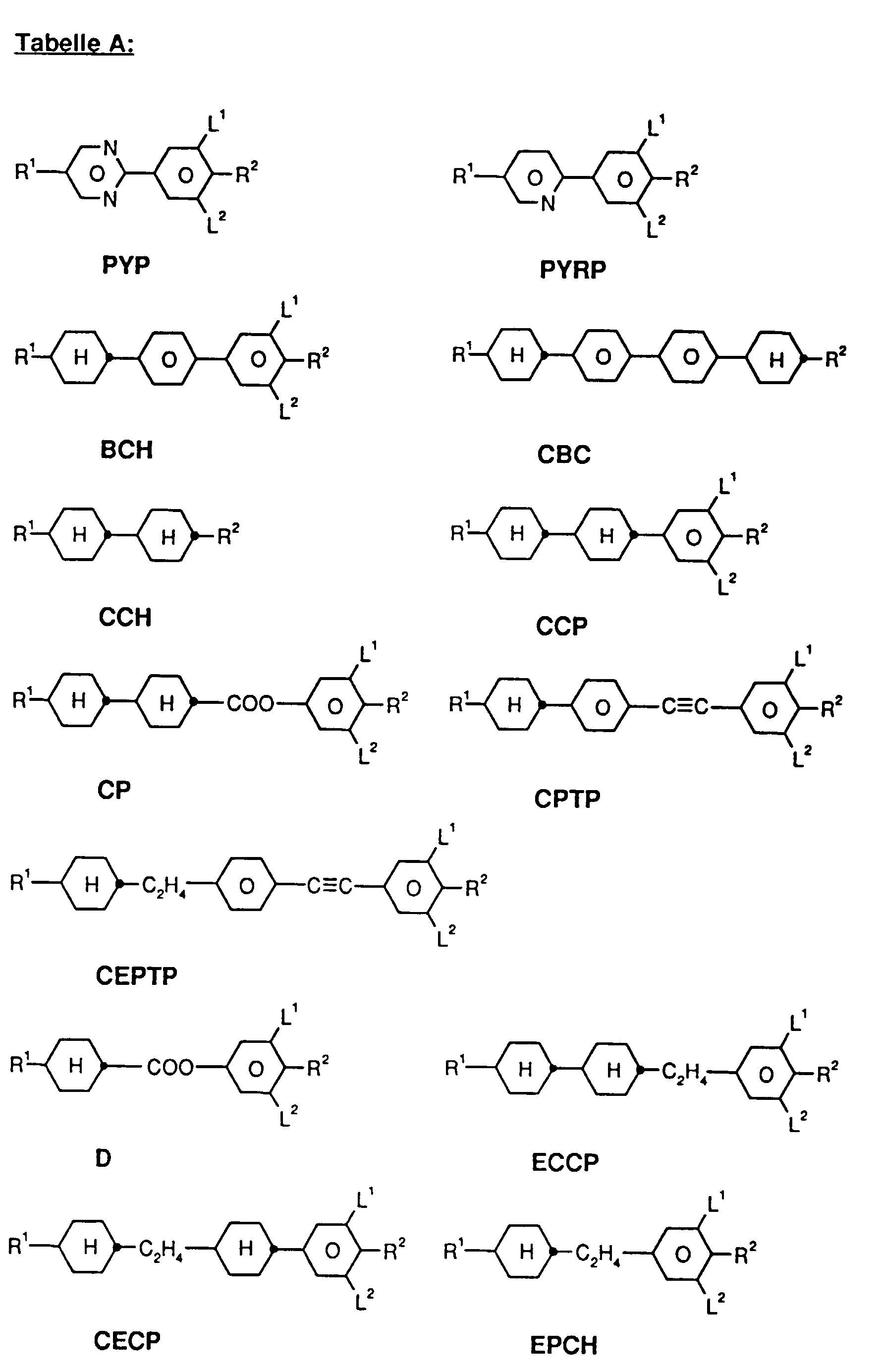

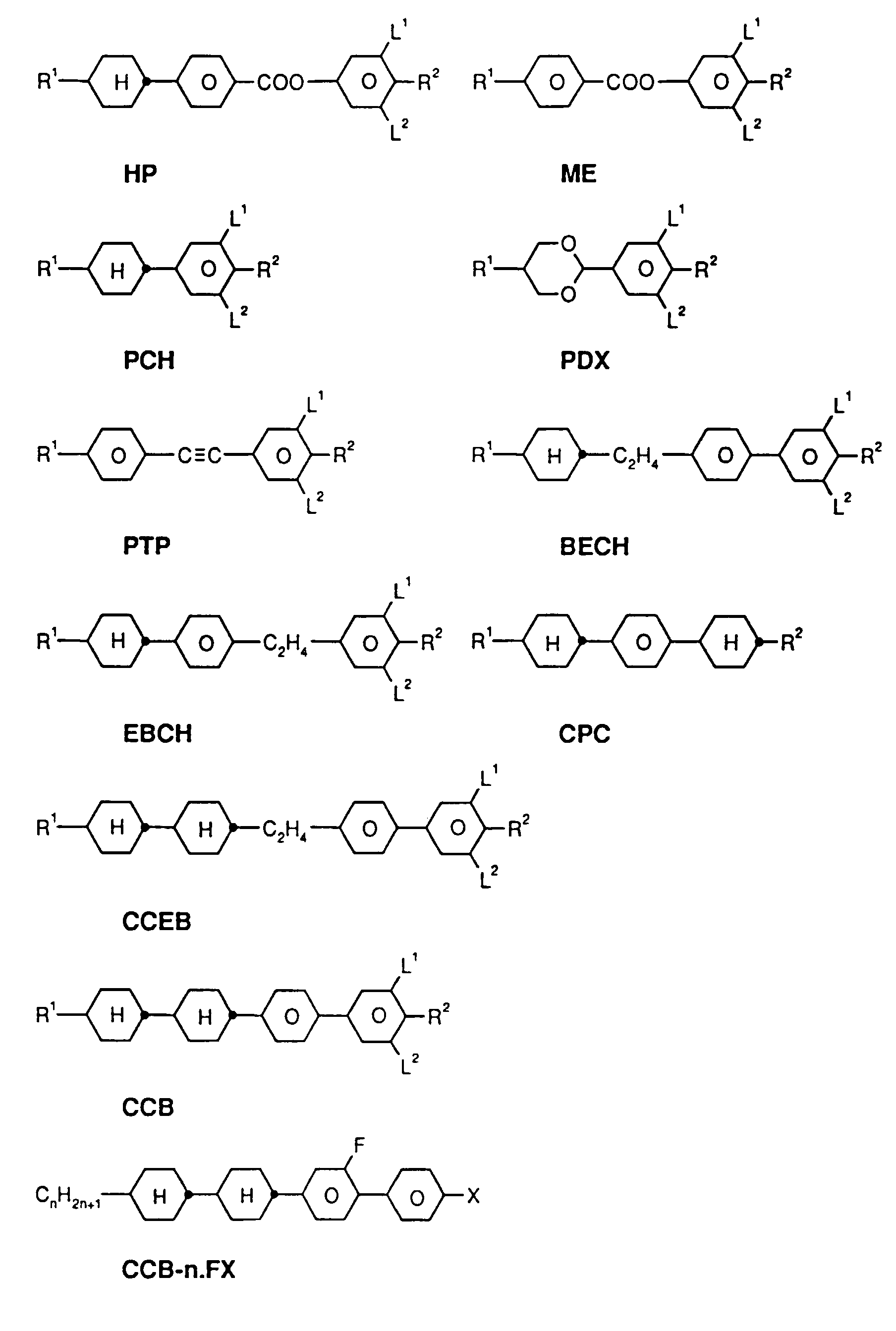

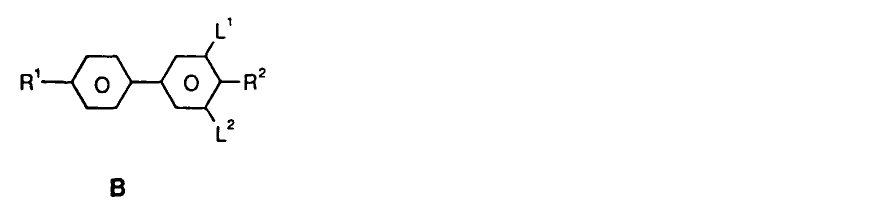

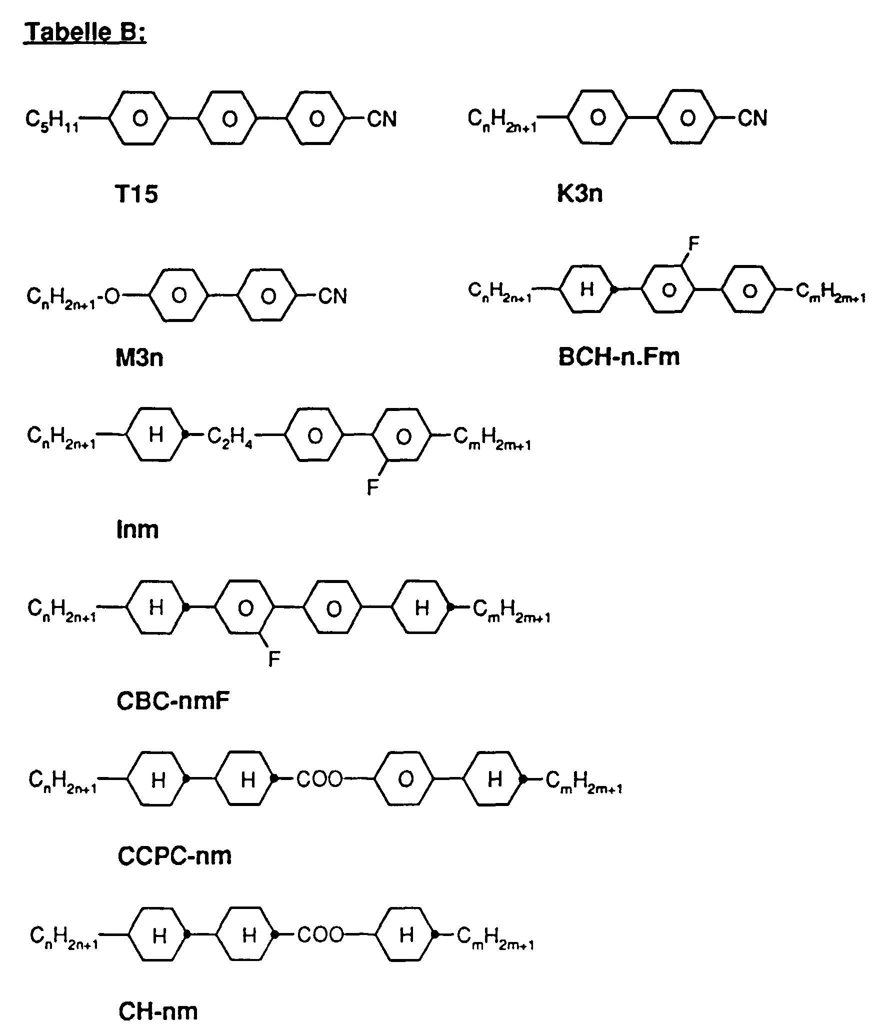

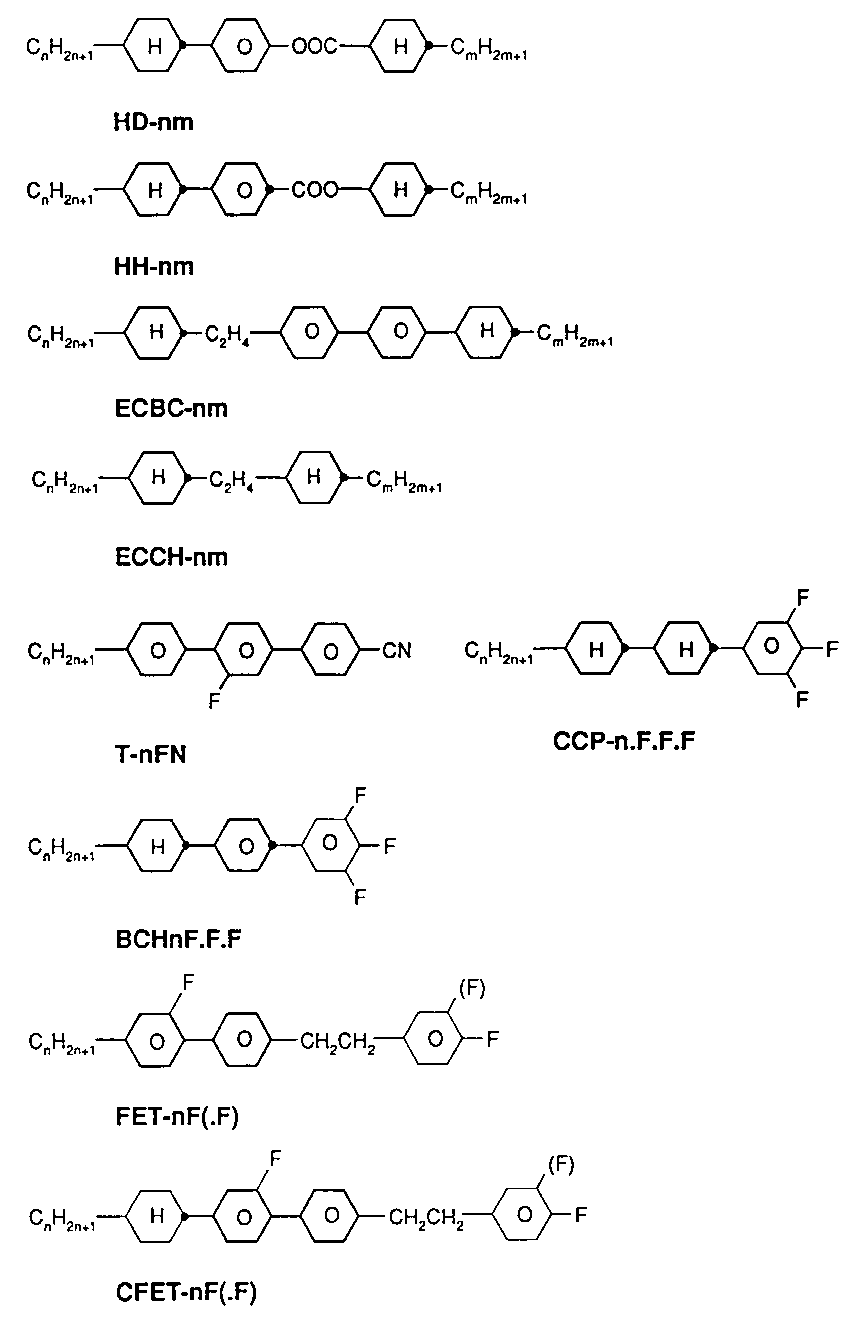

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. An bedeutet optische Anisotropie (589 nm, 20 °C) und die Viskosität (mm²/sec) wurde bei 20 °C bestimmt.

"Übliche Aufarbeitung" bedeutet: man gibt Wasser hinzu, extrahiert mit Dichlormethan, Methyl-tert.-Butylether oder Diethylether, trennt ab, trocknet die organische Phase, dampft ein und reinigt das Produkt durch Kristallisation und/oder Chromatographie.

| | |
|---|---|
| DAST | Diethylaminoschwefeltrifluorid |
| DCC | Dicyclohexylcarbodiimid |
| DDQ | Dichlordicyanobenzochinon |
| DIBALH | Diisobutylaluminiumhydrid |
| DMSO | Dimethylsulfoxid |
| KOT | Kalium-tertiär-butanolat |
| THF | Tetrahydrofuran |
| pTsOH | p-Toluolsulfonsäure |

### Beispiel 1

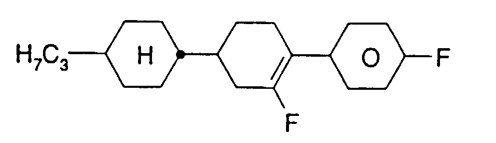

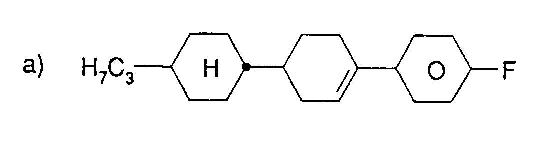

Zu einer Suspension von 1 mol Magnesiumspänen in 250 ml THF gibt man einige Tropfen Brom. Anschließend wird eine Lösung von 1 mol 1-Brom-4-fluorbenzol in 250 ml THF portionsweise zugetropft. Man kocht 1,5 h unter Rückfluß und tropft in der Siedehitze 0,9 mol 4-trans-4-Propylcyclohexylcyclohexanon in 500 ml THF zu dem Grignard-Reagenz. Es wird 1,5 h unter Rückfluß gekocht und bei 30 °C mit 1,5 l Wasser und 200 ml konz. HCI versetzt. Man rührt 1/4 h nach, trennt die organische Phase ab, extrahiert die wäßrige Phase mit Methyl-tert.-Butylether und arbeitet die vereinigten organischen Phasen wie üblich auf. 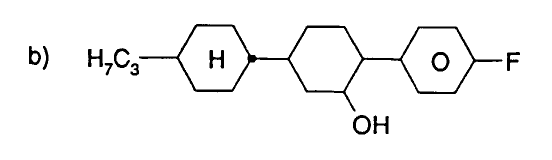 0,2 mol des Cyclohexenderivats aus 1a) werden in 500 ml THF gelöst und bei +2 °C mit 240 ml BH₃ THF-Komplex innerhalb 0,5 h versetzt. Man rührt 1 h bei +2 °C nach, läßt auf Raumtemperatur erwärmen und rührt eine weitere Stunde. Nach Zugabe von 60 ml Ethanol wird auf 30 °C erwärmt und zunächst mit 40 ml 6N NaOH und dann mit 80 ml 30%iger H₂O₂ versetzt. Man kocht 2 h unter Rückfluß, läßt auf Raumtemperatur abkühlen und hydrolysiert. Die wäßrige Phase wird abgetrennt und die organische Phase mit 250 ml Methyl-tert.-Butylether verdünnt. Die organische Phase wird mit 5%iger NaHSO₃-Lösung und Wasser gewaschen und über Na₂SO₄ getrocknet. Das Lösungsmittel wird am Rotavapor entfernt und der Rückstand wird in n-Hexan umkristallisiert. 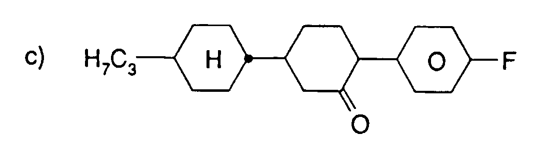 In einer Stickstoffatmosphäre werden 0,094 mol des Cyclohexanolderivats aus b) in 250 ml Dichlormethan gelöst und mit 0,14 mol Pyridiniumchlorochromat versetzt. Das Reaktionsgemisch wird 2 h unter Rückfluß gekocht und eingeengt. Der Rückstand wird über eine Al₂O₃Kieselgel-Säule gereinigt. 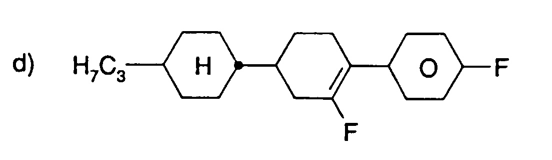 0,0382 mol DAST in 15 ml Dichlormethan werden bei Raumtemperatur mit dem Cyclohexanonderivat (0,02 mol) aus c) in 20 ml Dichlormethan versetzt. Man kocht 4 h unter Rückfluß und läßt die Reaktionslösung über Nacht auf Raumtemperatur abkühlen. Unter Eiskühlung wird hydrolysiert und anschließend wird wie üblich aufgearbeitet: 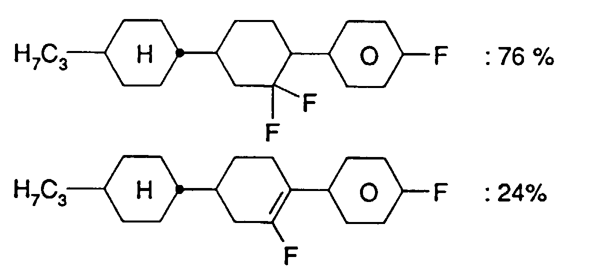 Das Produkt (0,01 mol) wird ohne weitere Aufreinigung in 50 ml THF gelöst, mit 0,03 mol Kalium-tert.-butylat versetzt und 11 h bei 50 °C in einer N₂-Atmosphäre gerührt. Man läßt auf Raumtemperatur abkühlen, versetzt mit Eiswasser und konz. HCI und extrahiert mit Methyl-tert.-butylether. Anschließend wird wie üblich aufgearbeitet.
K 40 N 124,5 l; Δn = +0,106; Δε = 6,76

Analog werden die folgenden Verbindungen der Formel 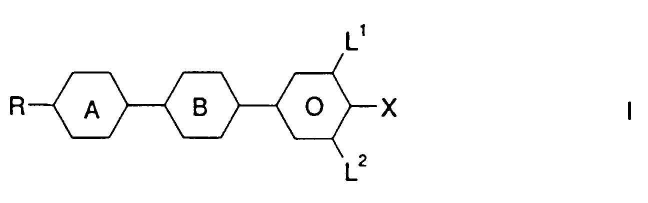 hergestellt:

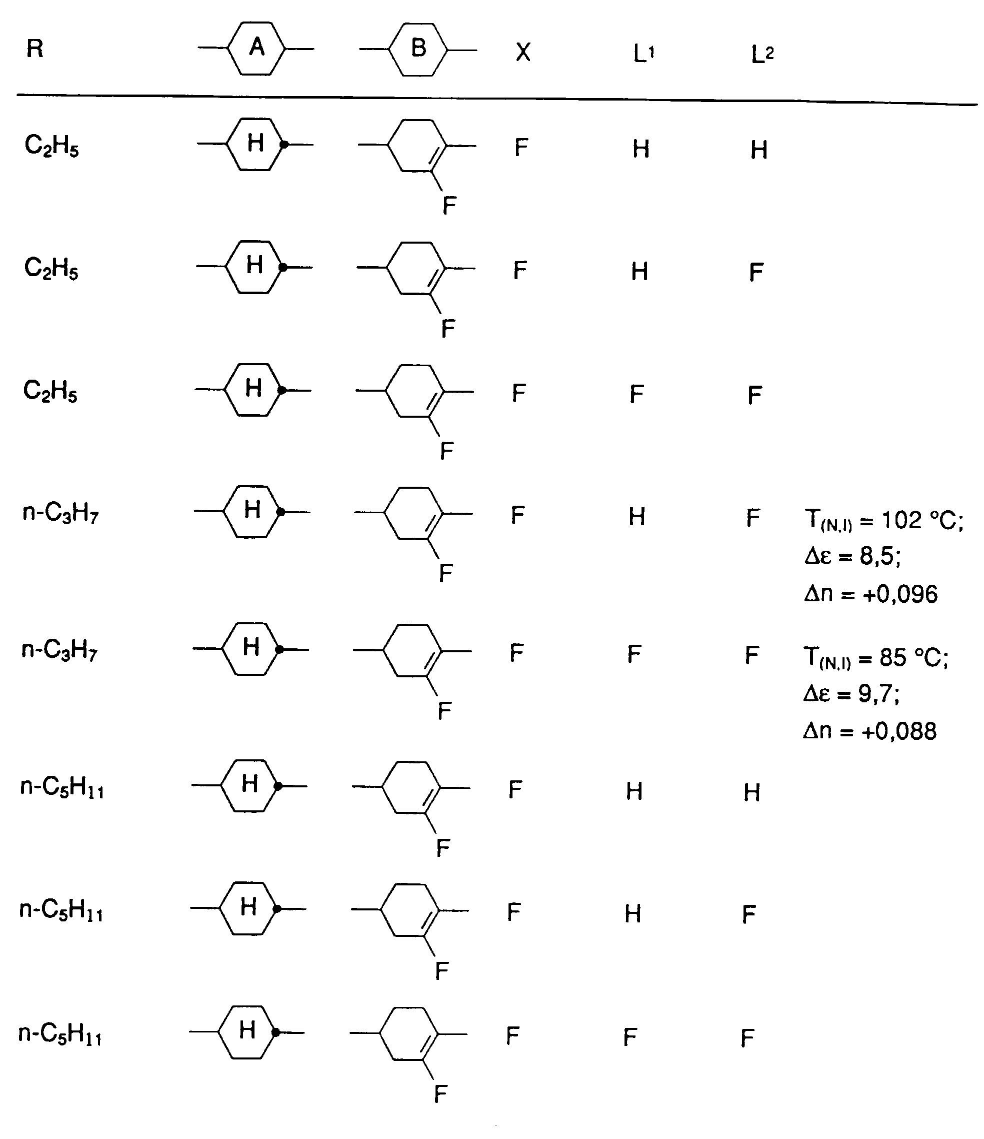

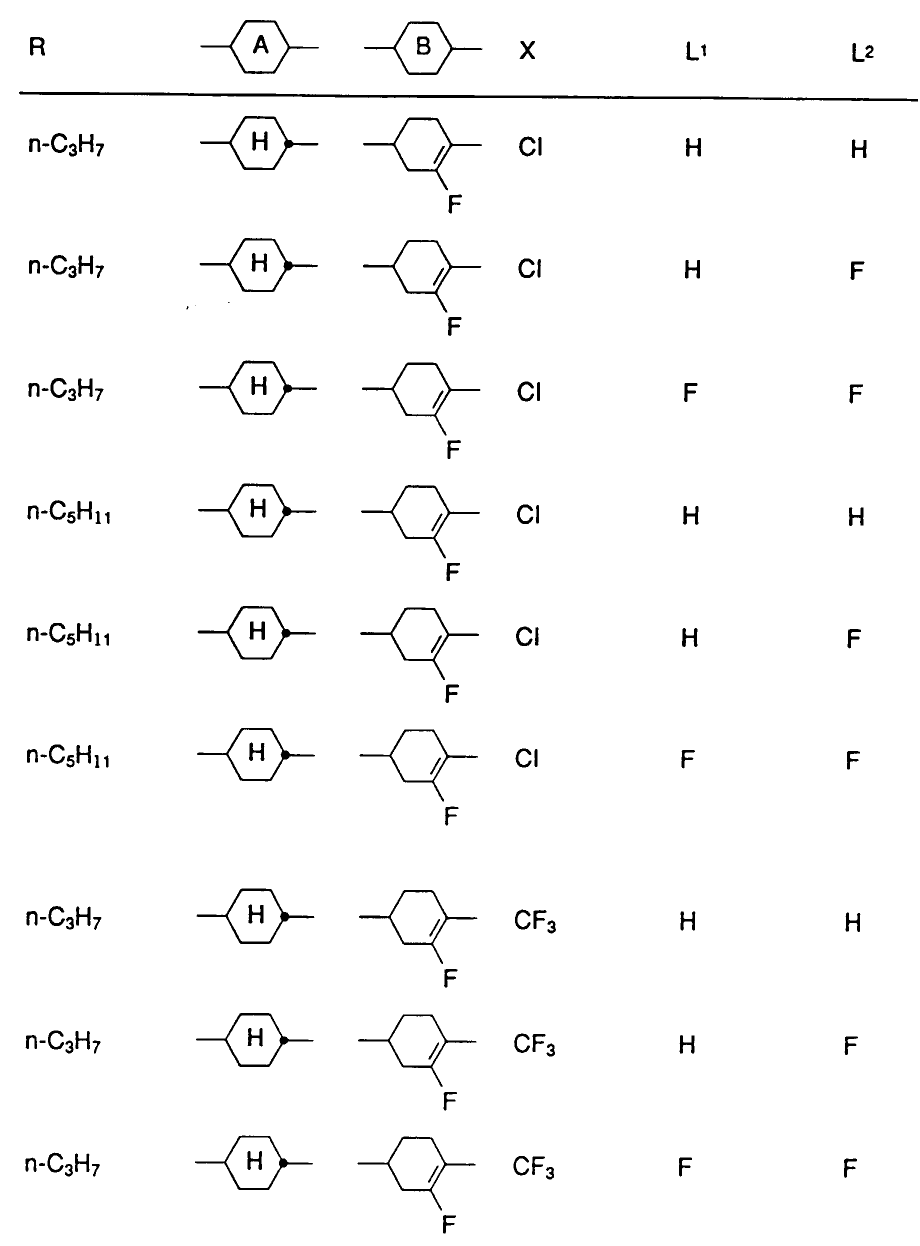

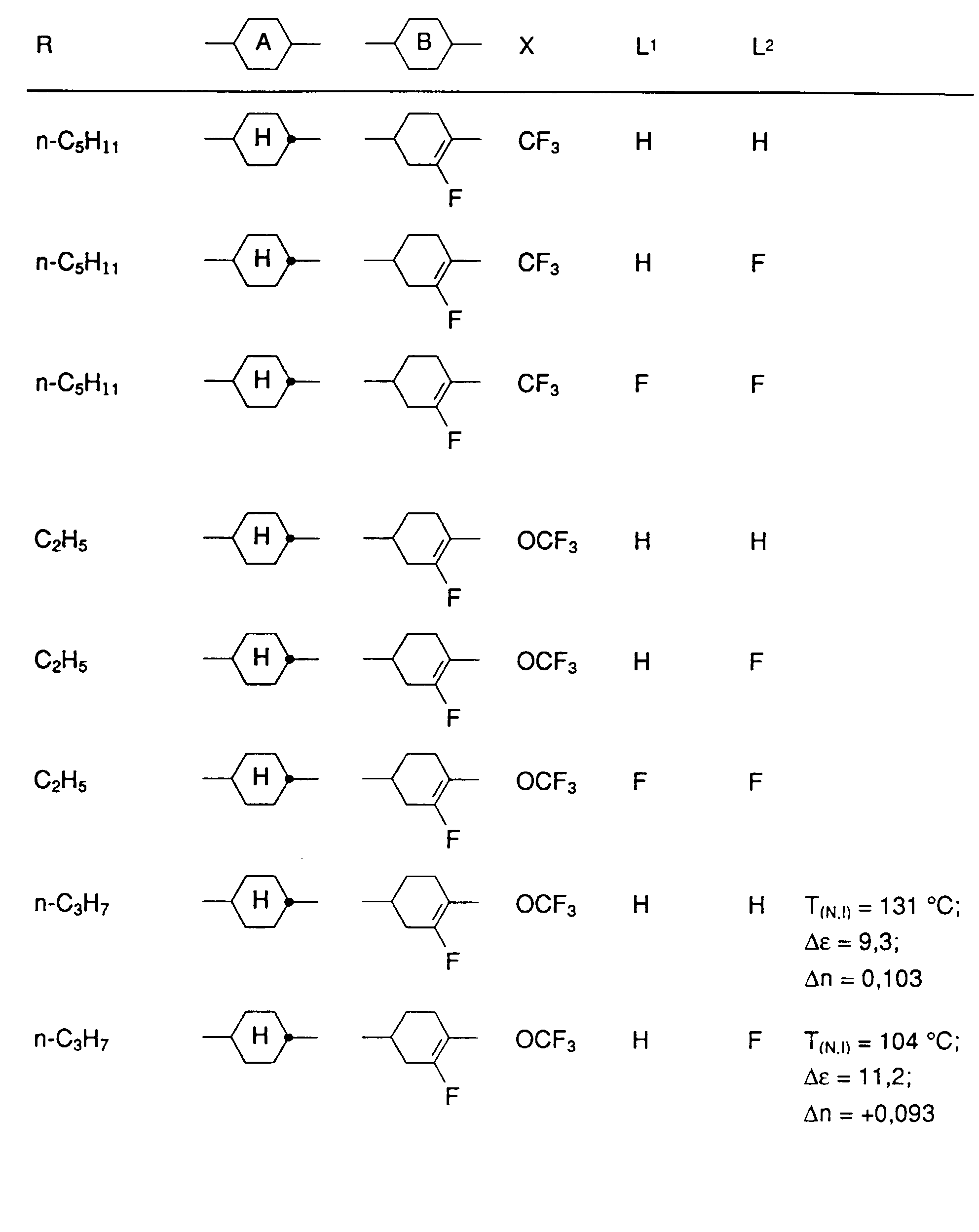

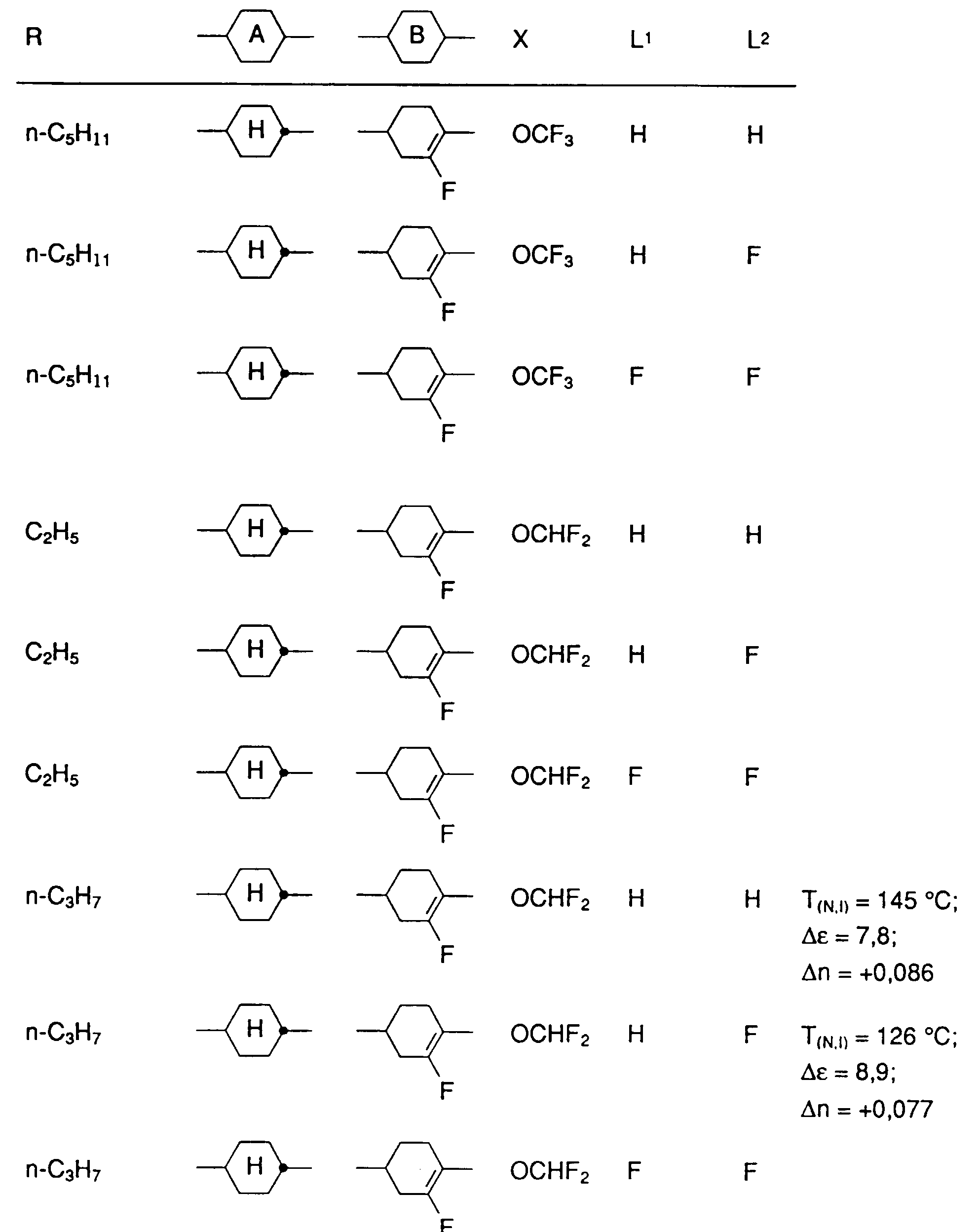

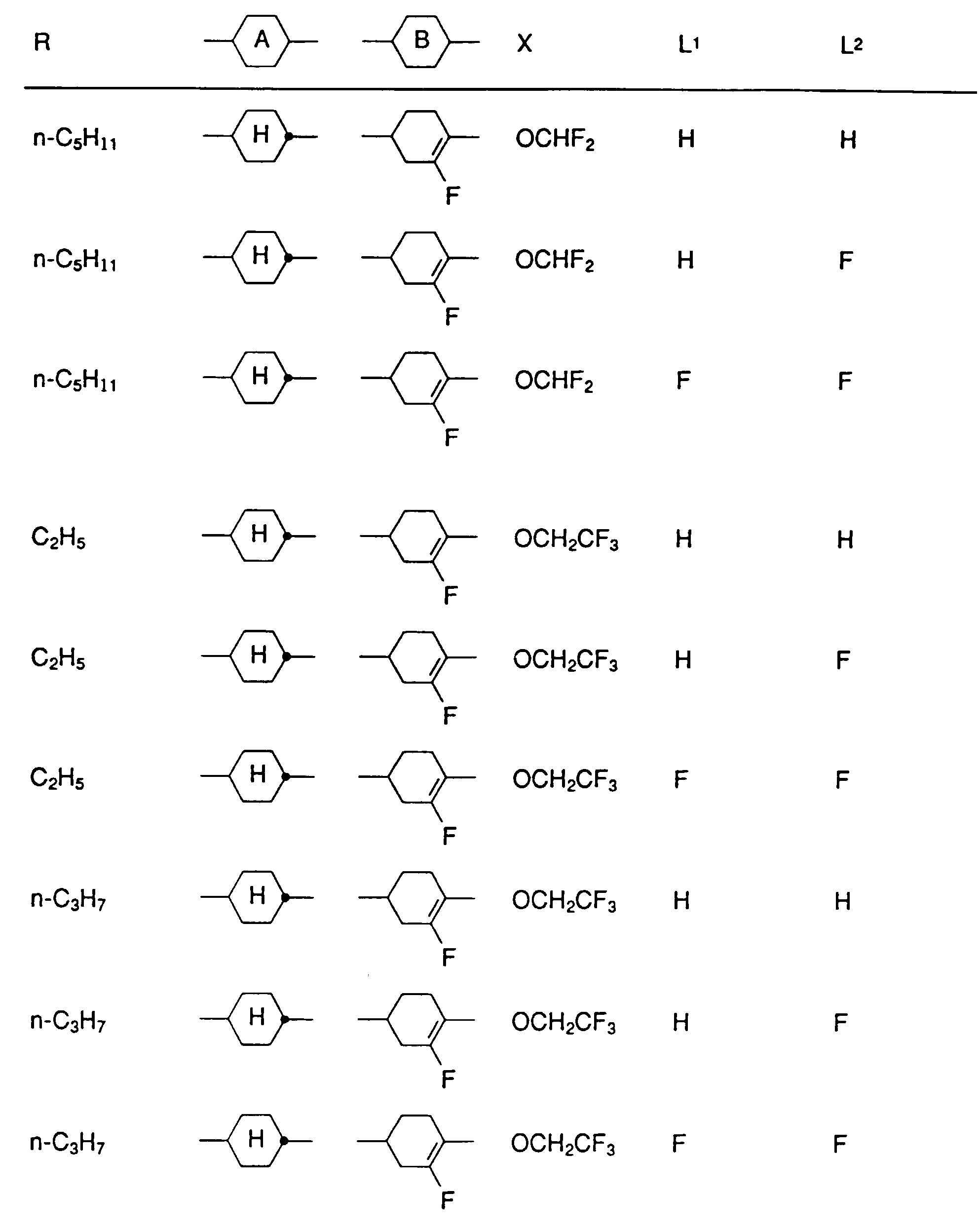

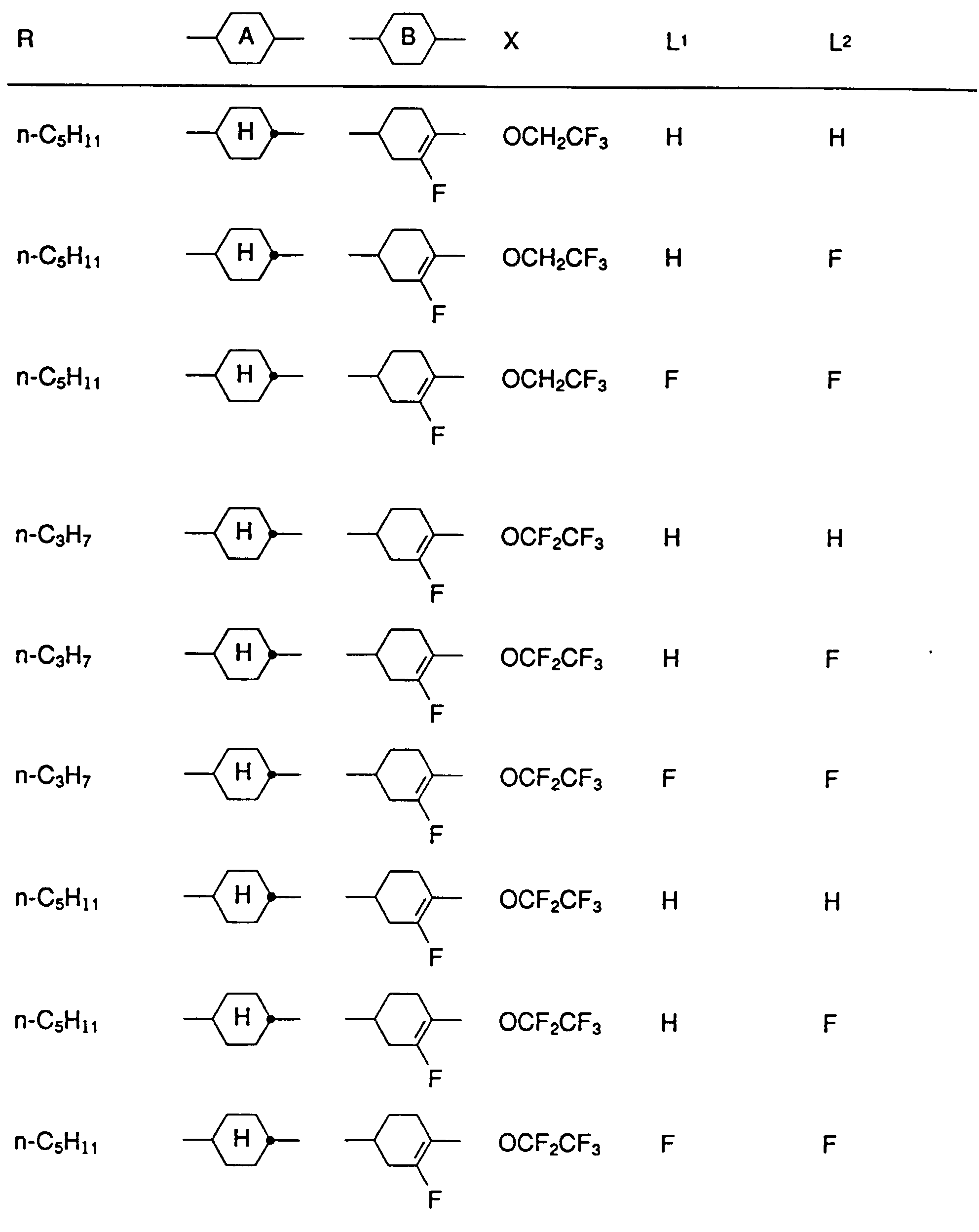

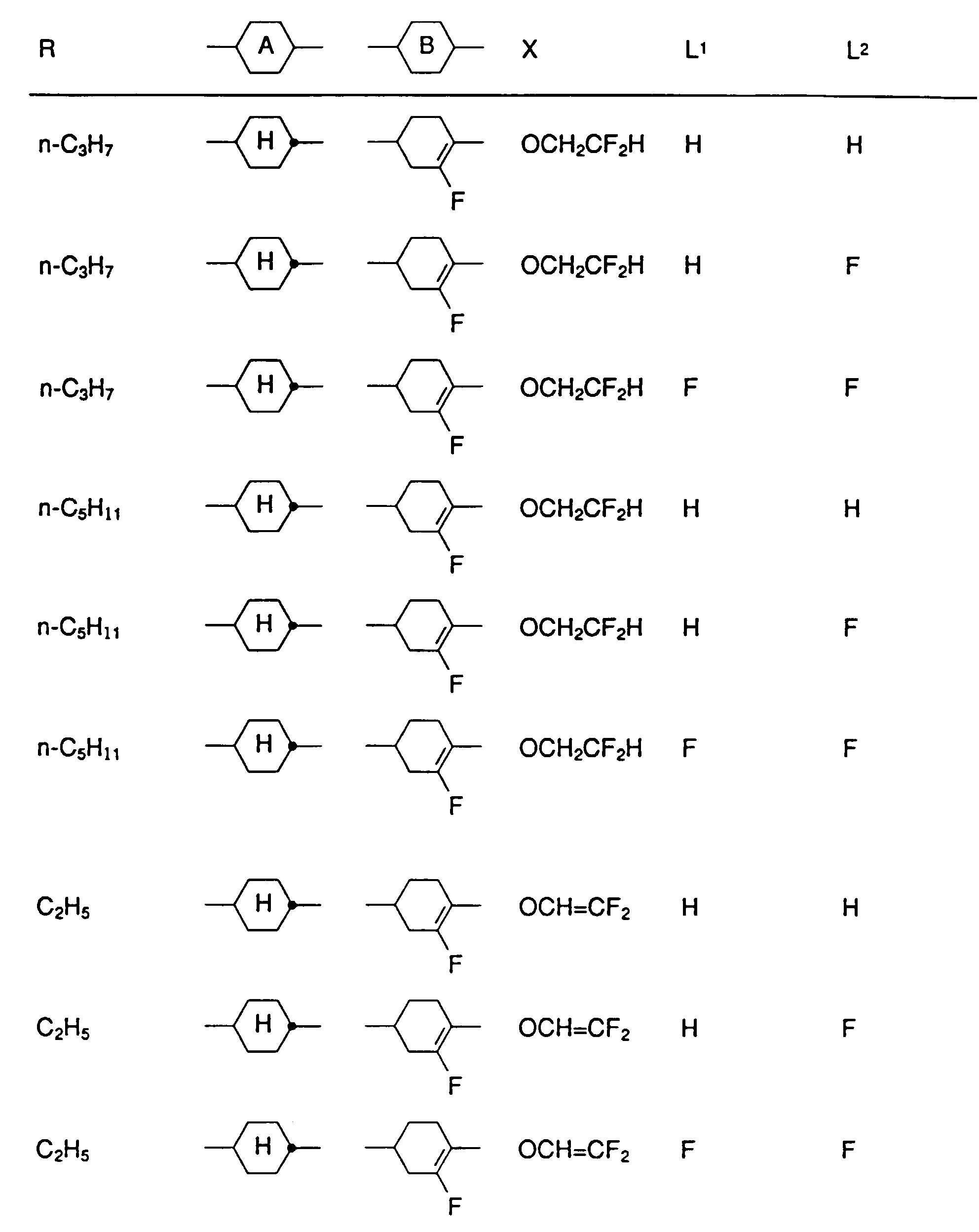

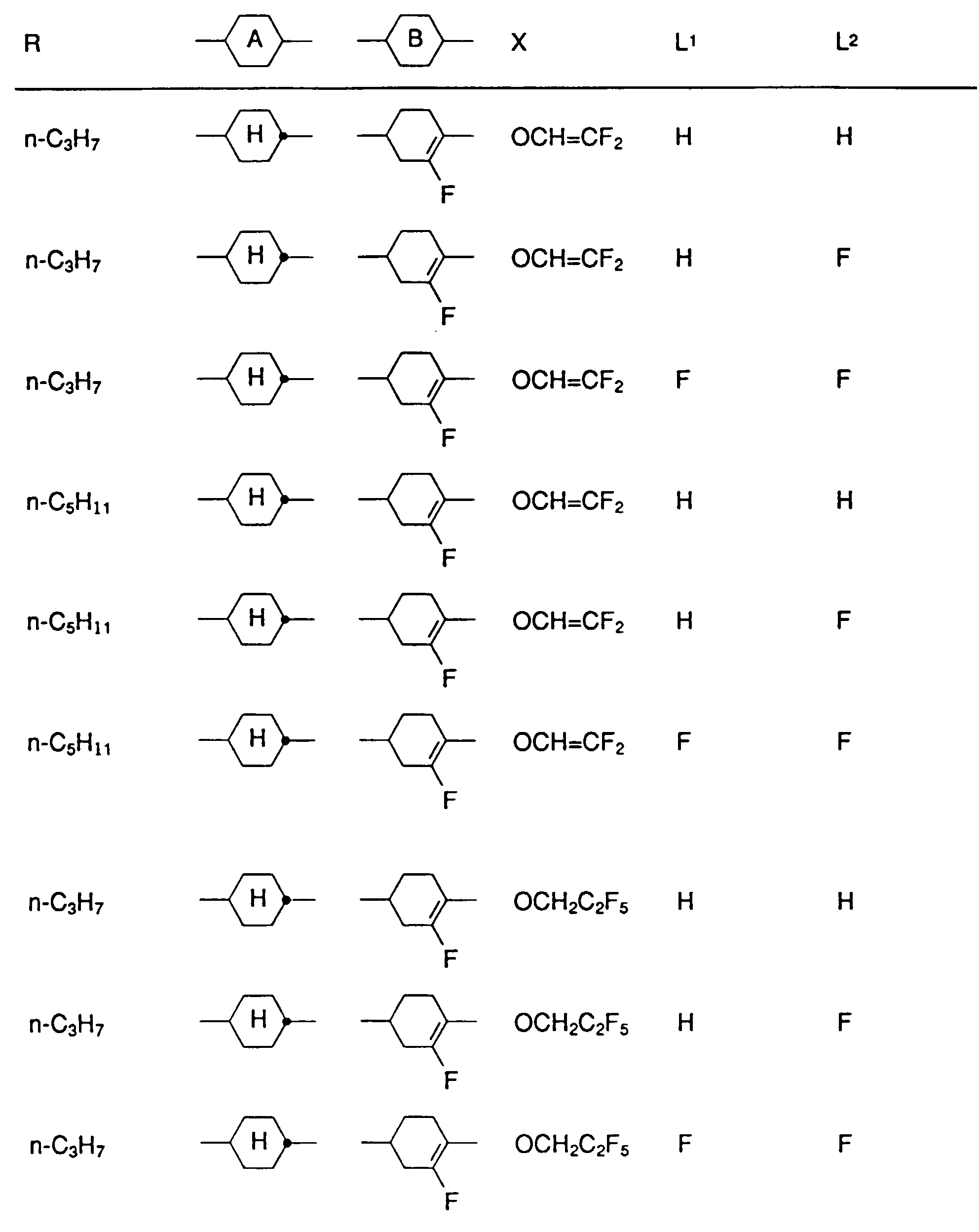

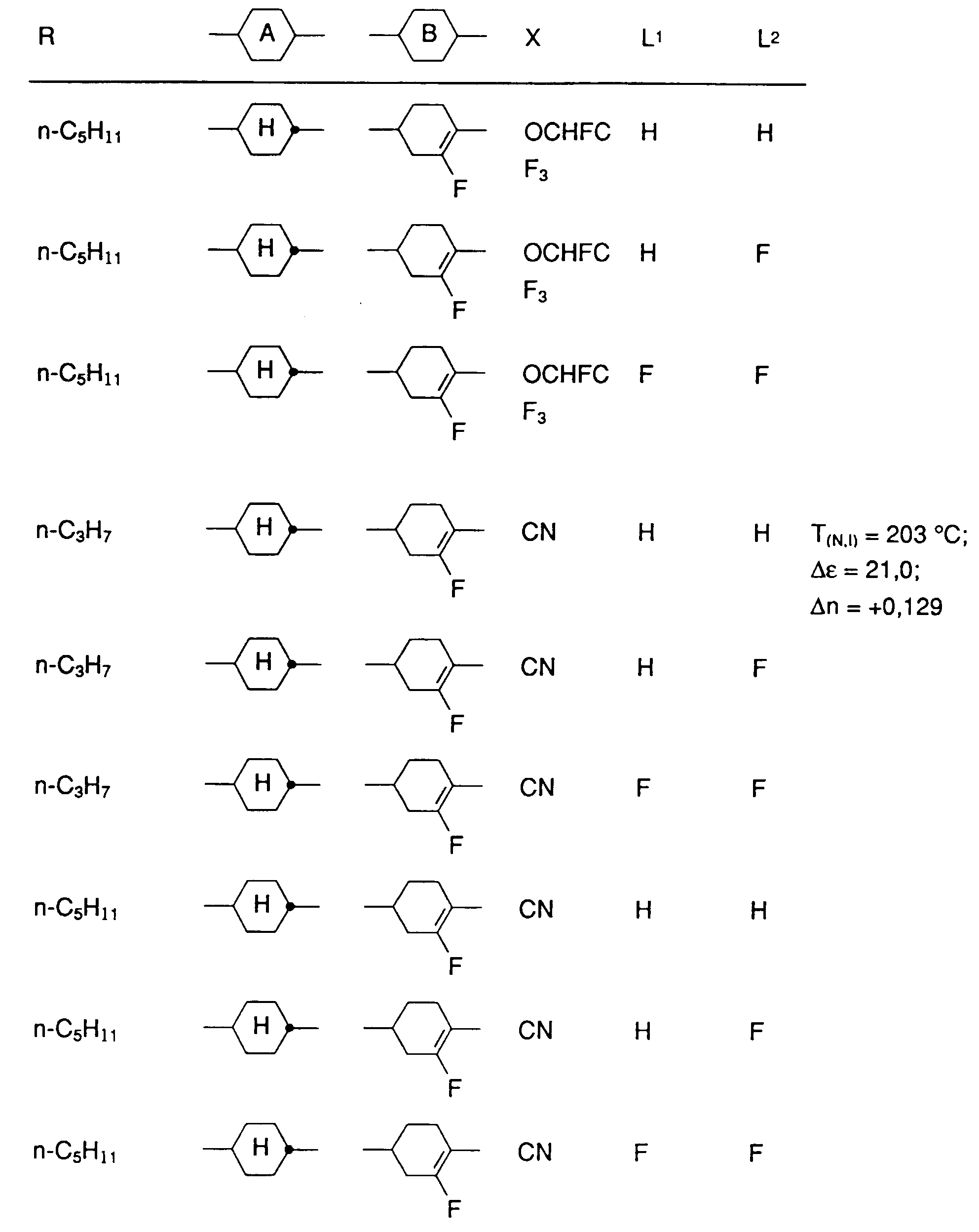

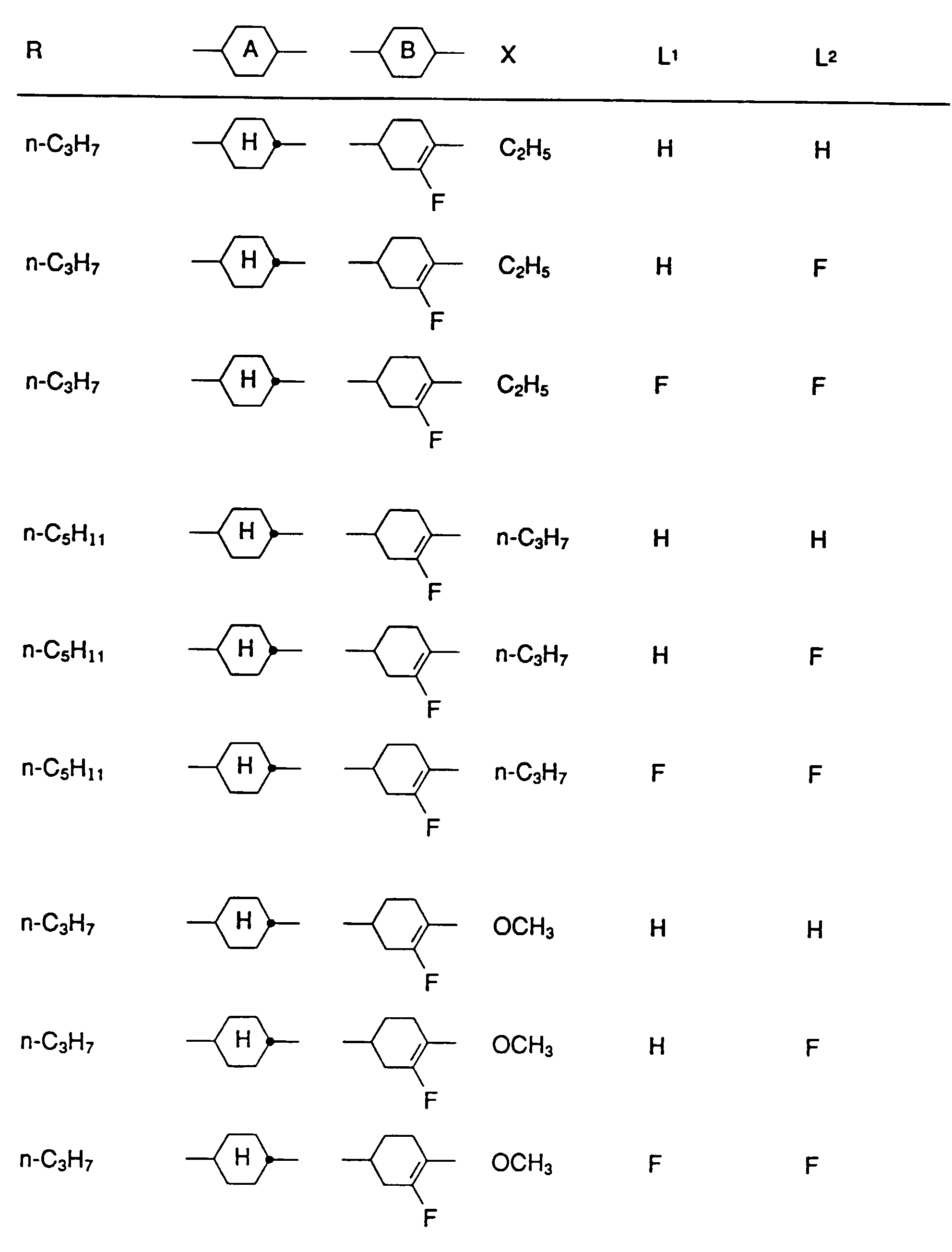

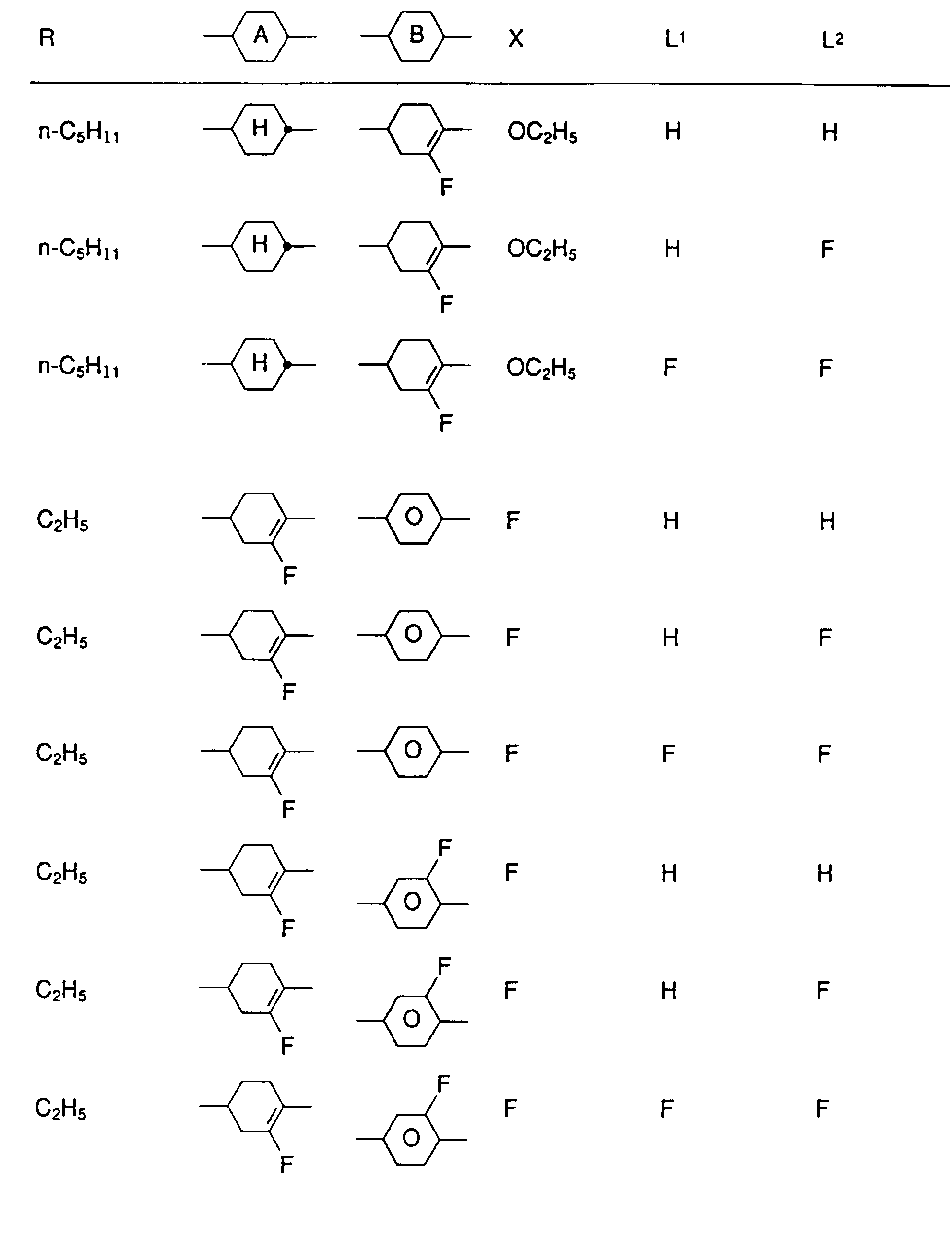

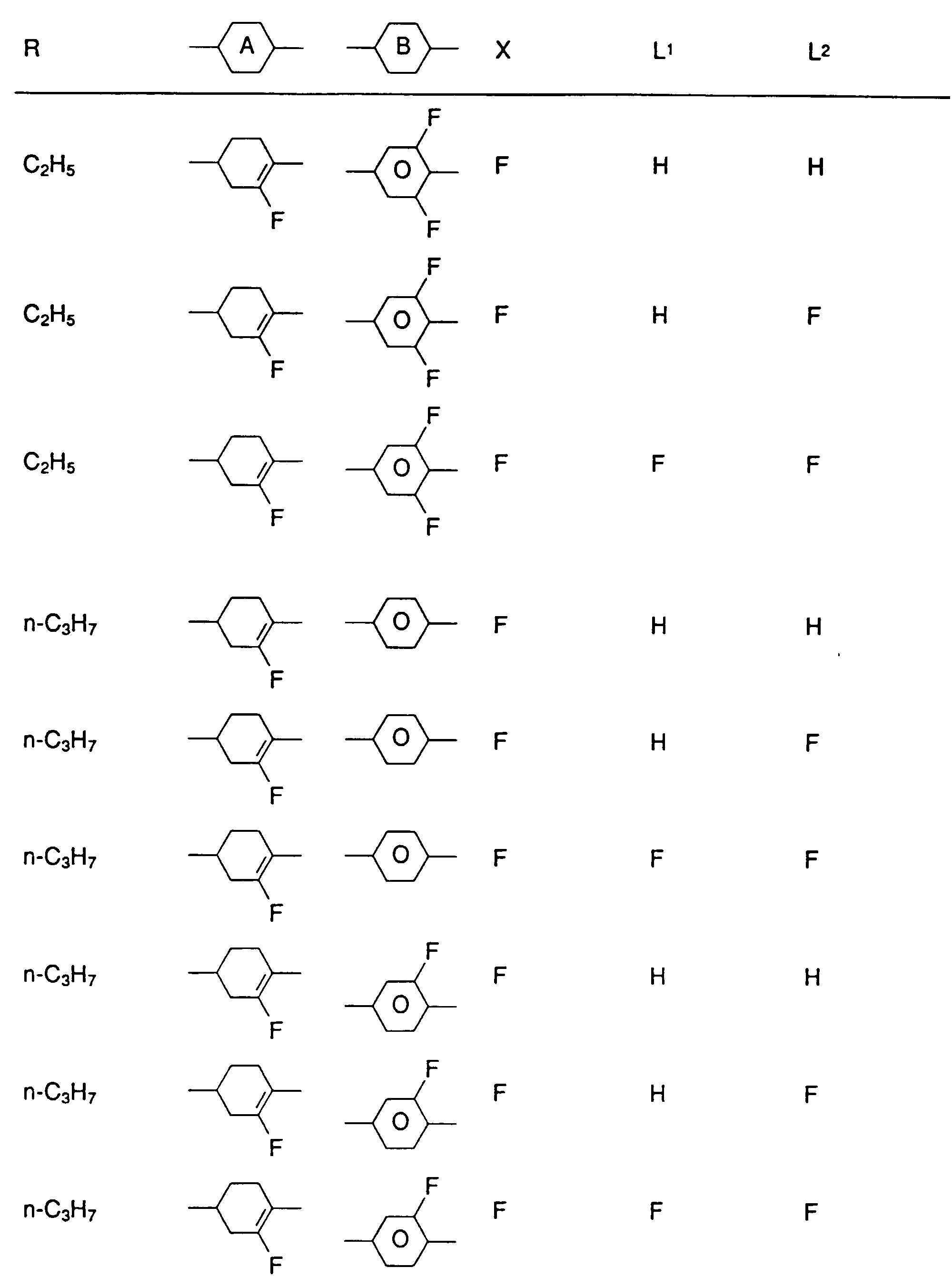

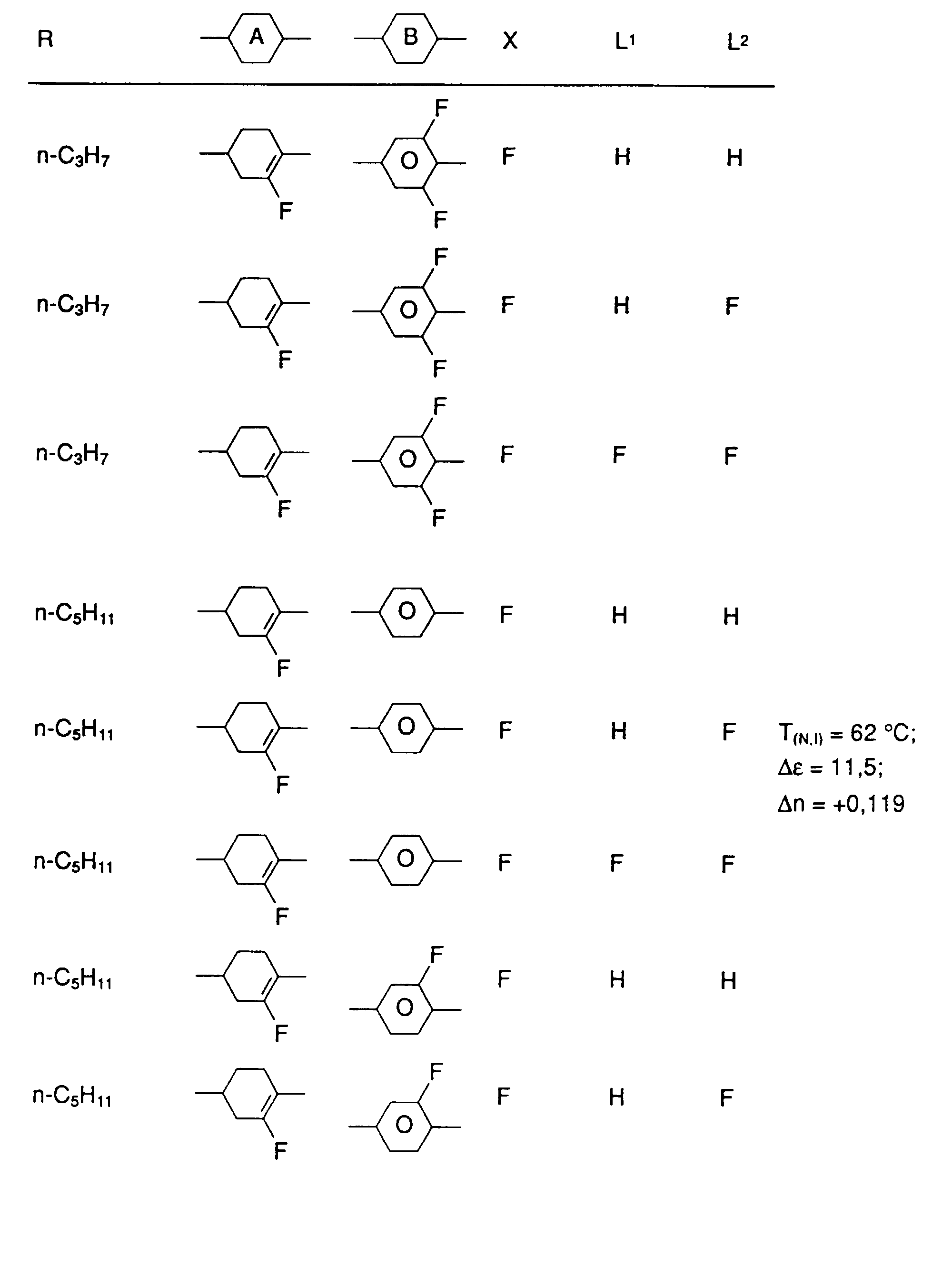

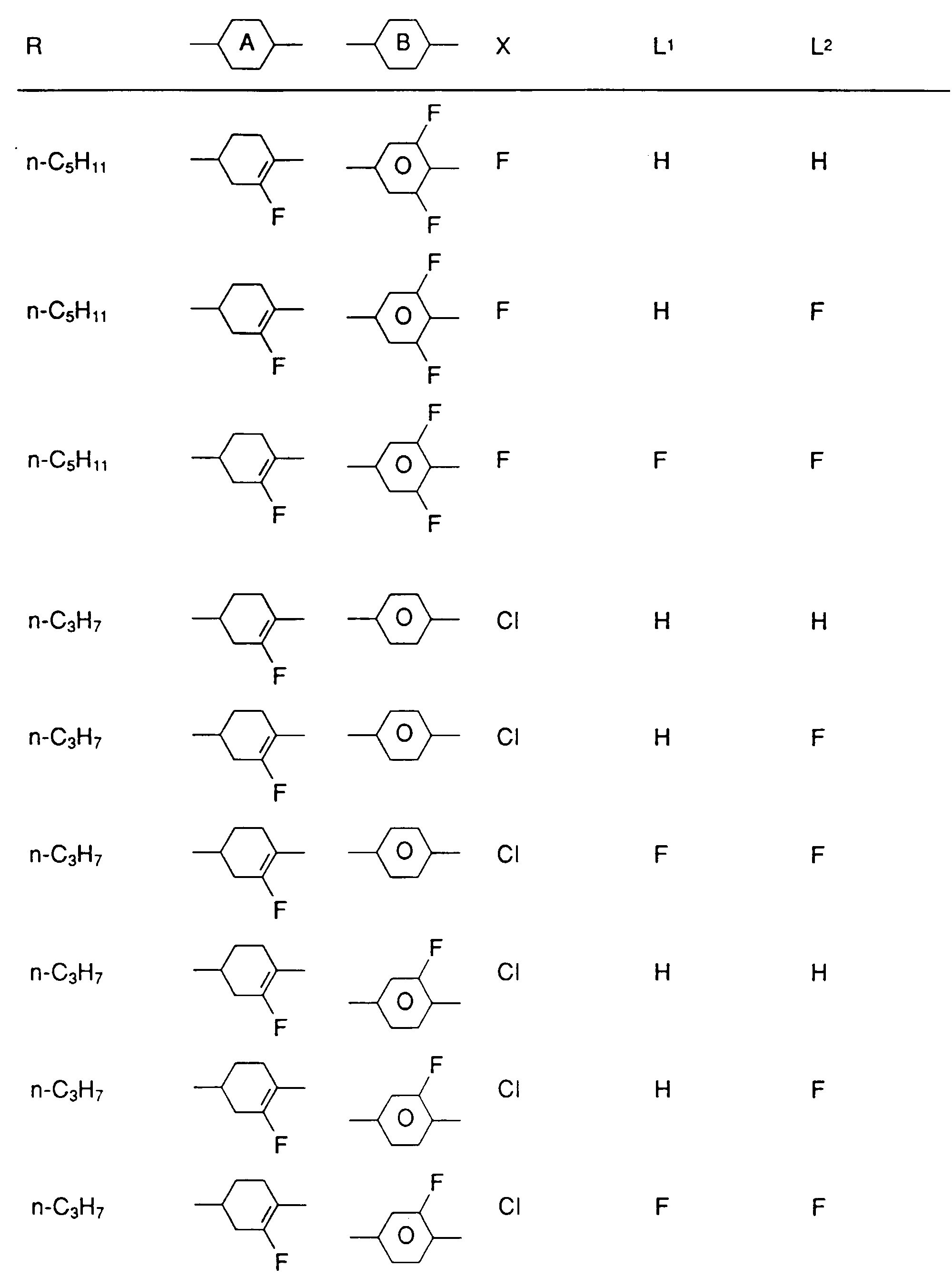

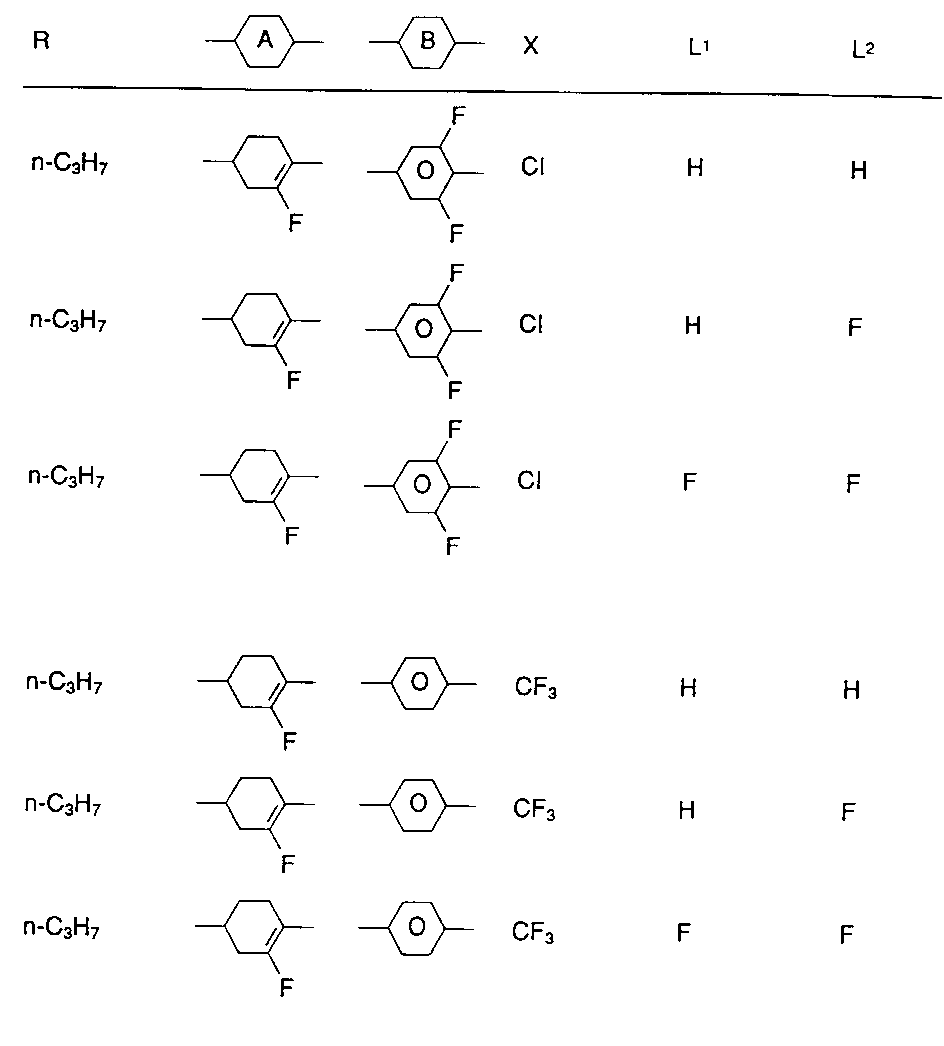

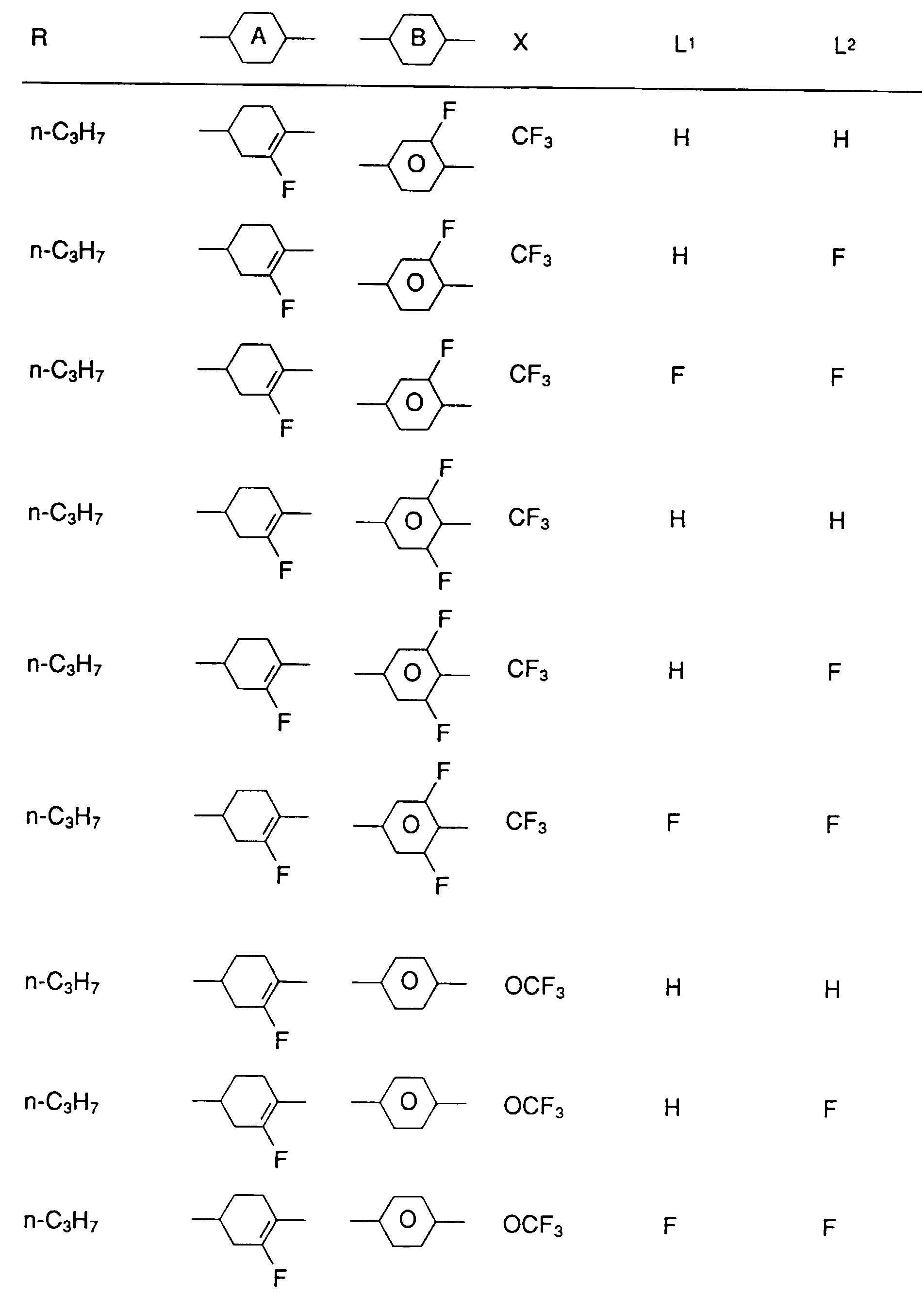

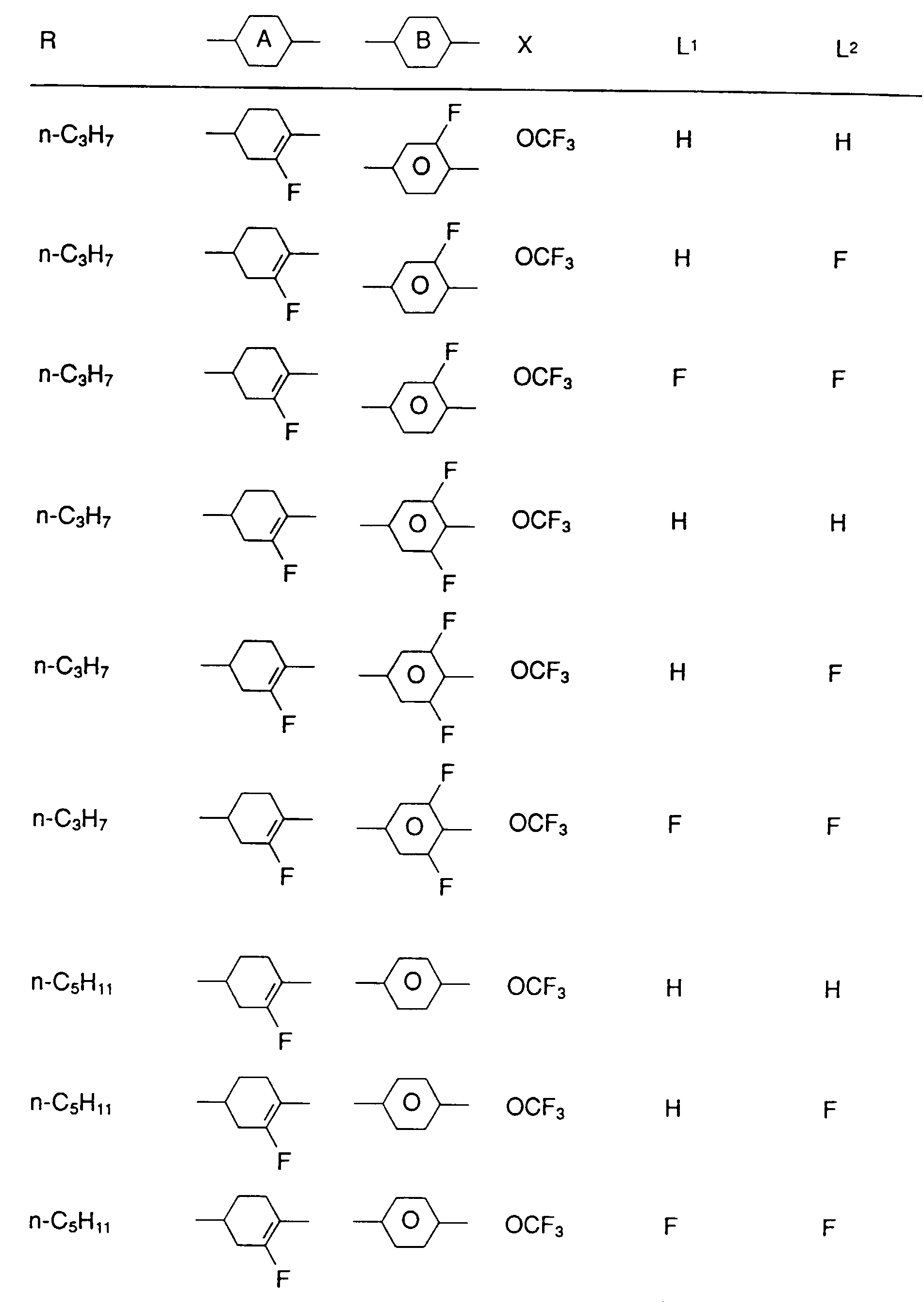

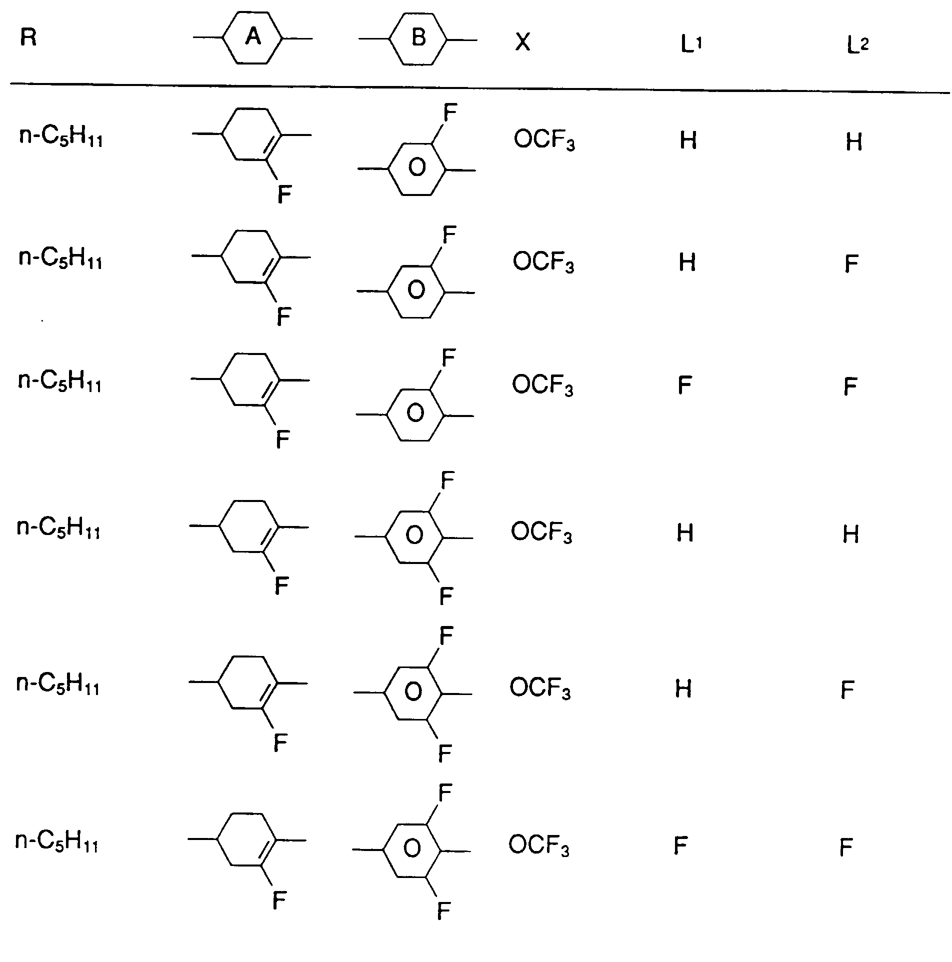

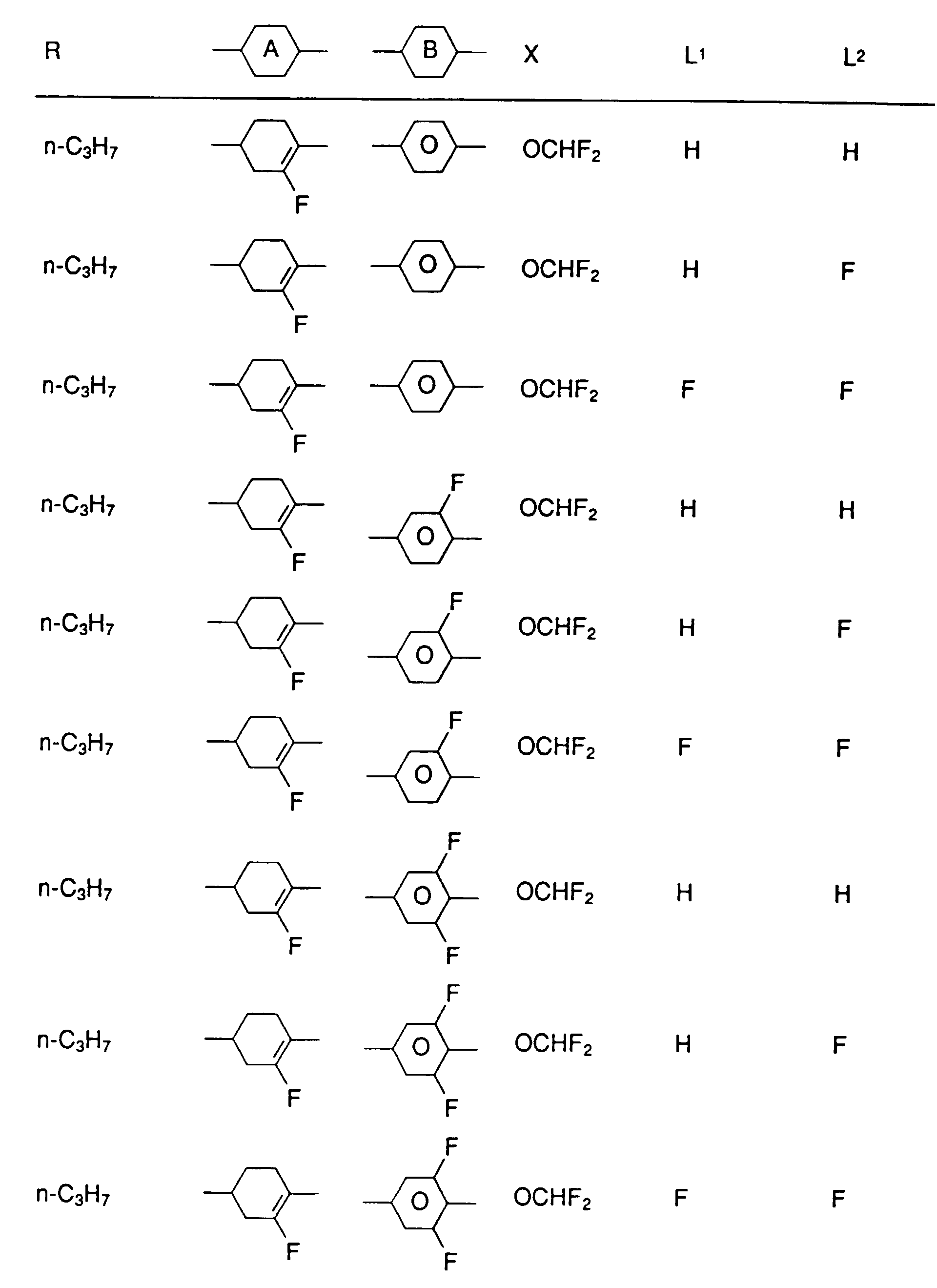

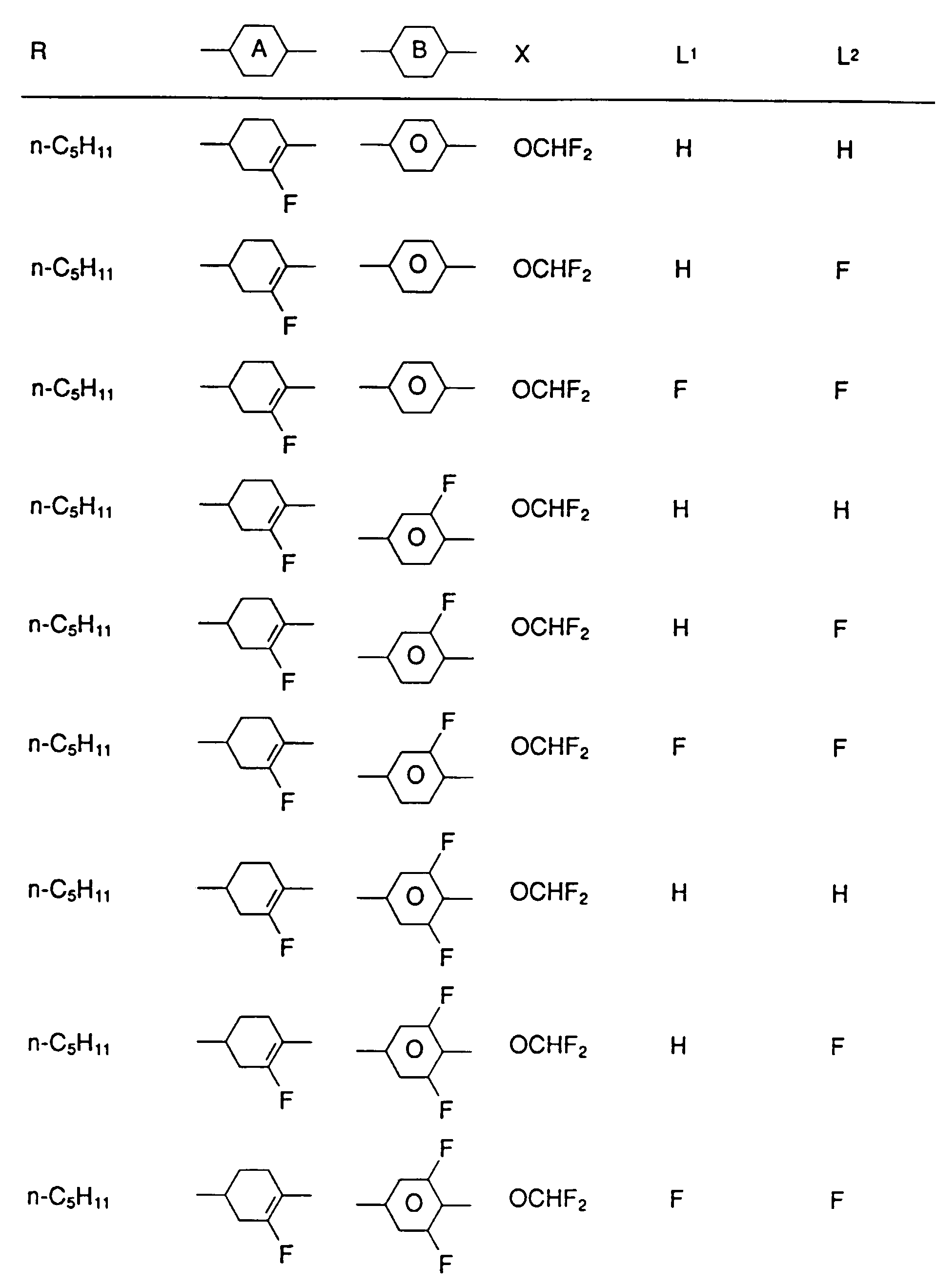

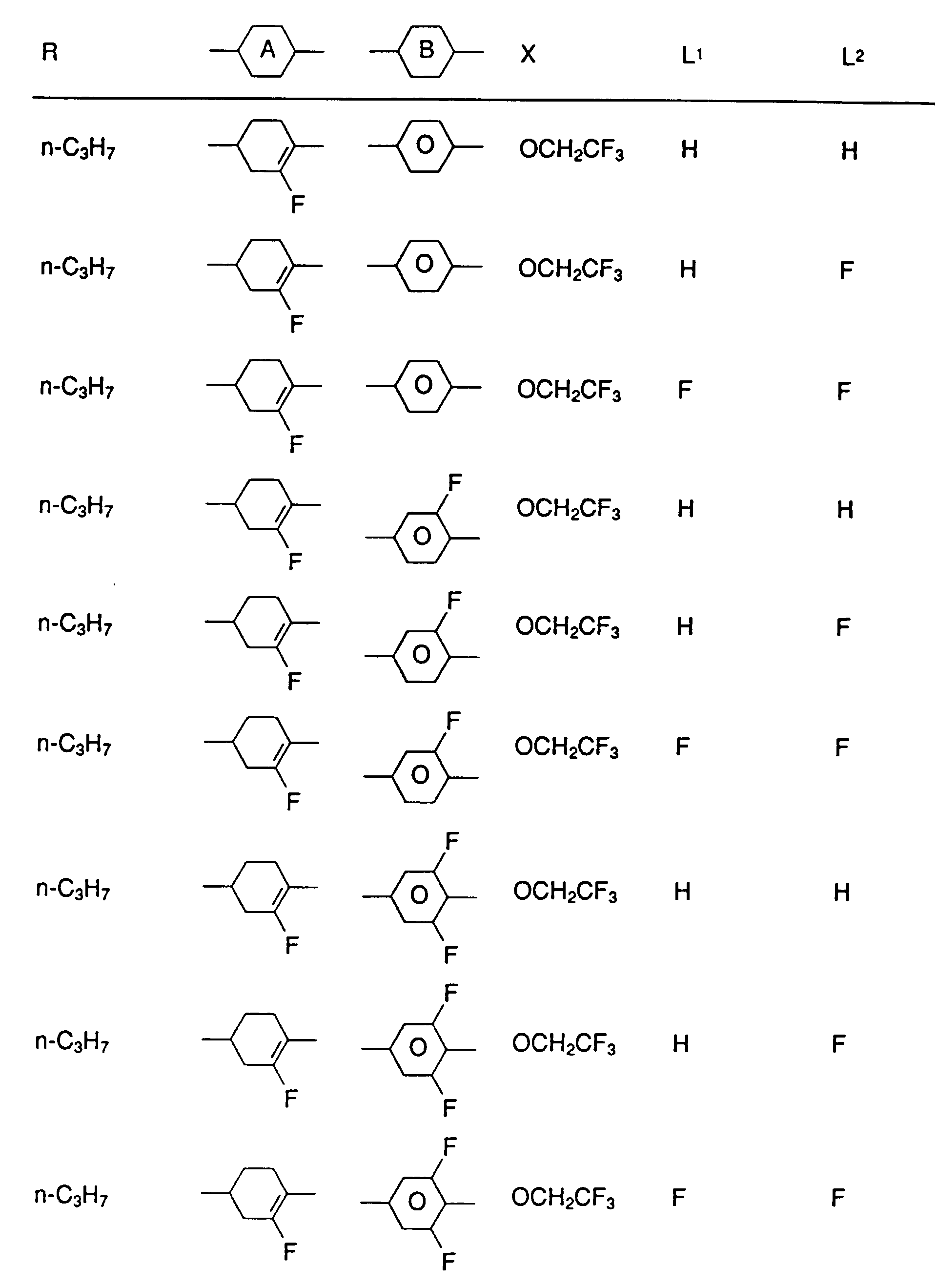

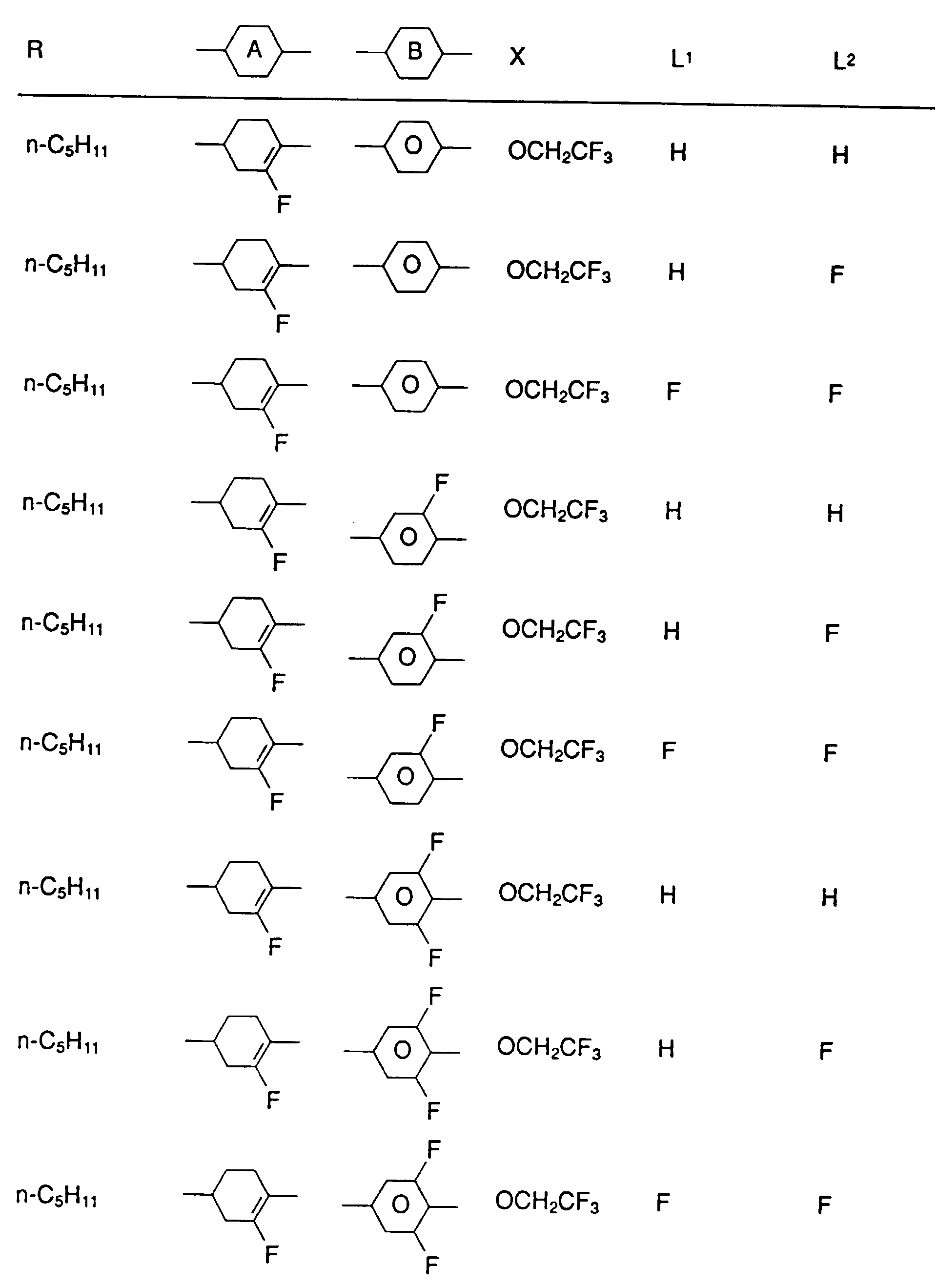

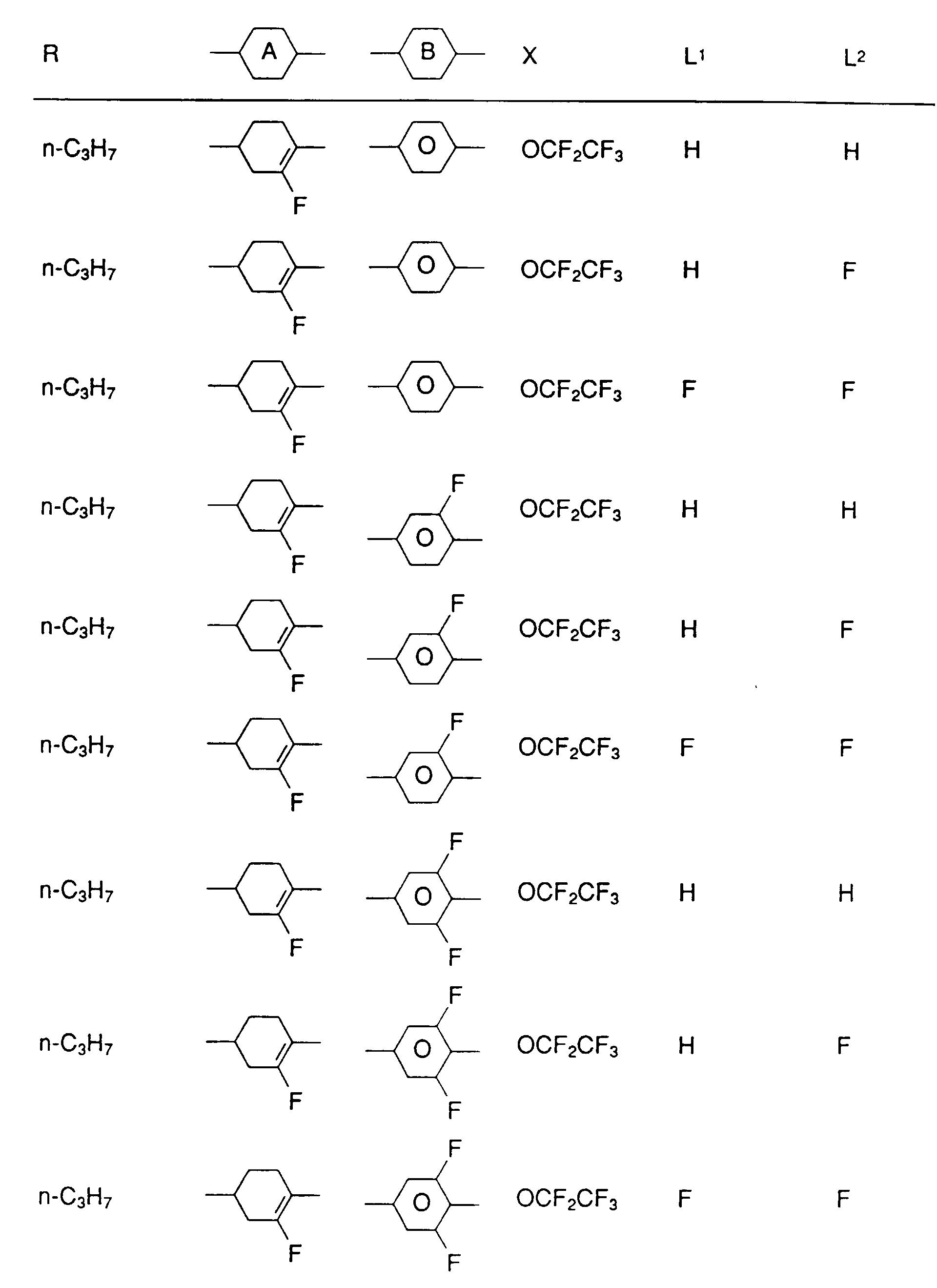

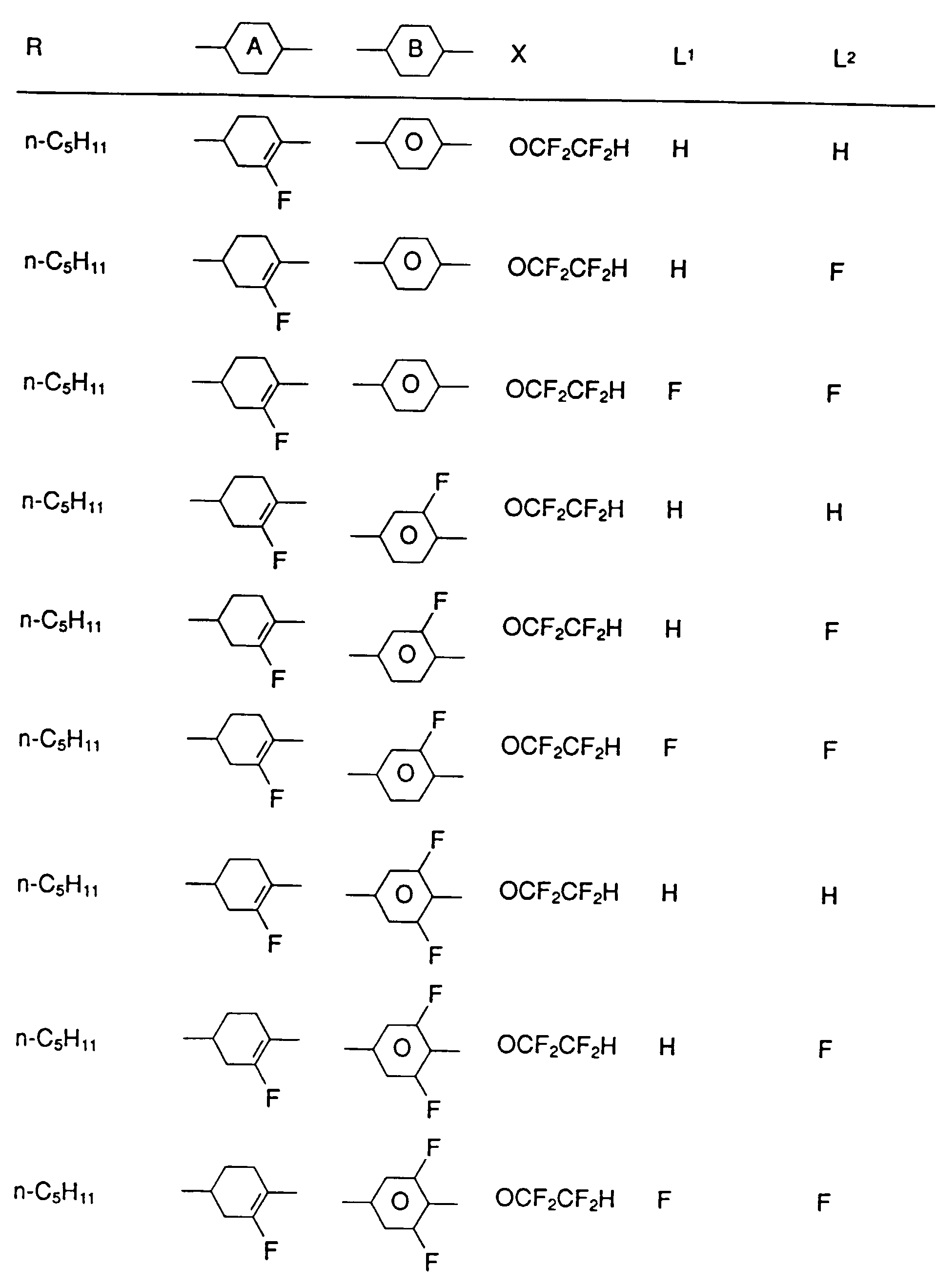

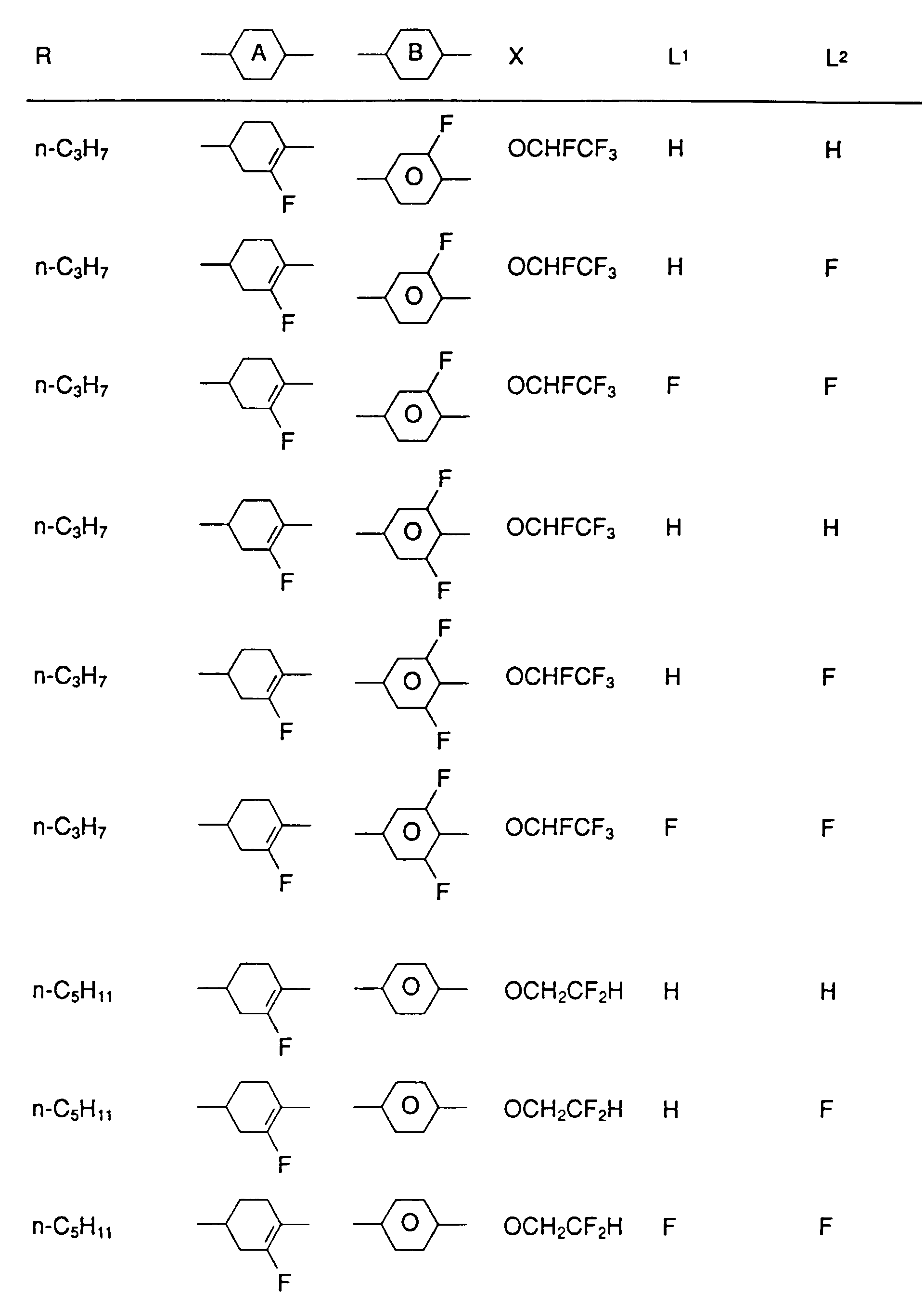

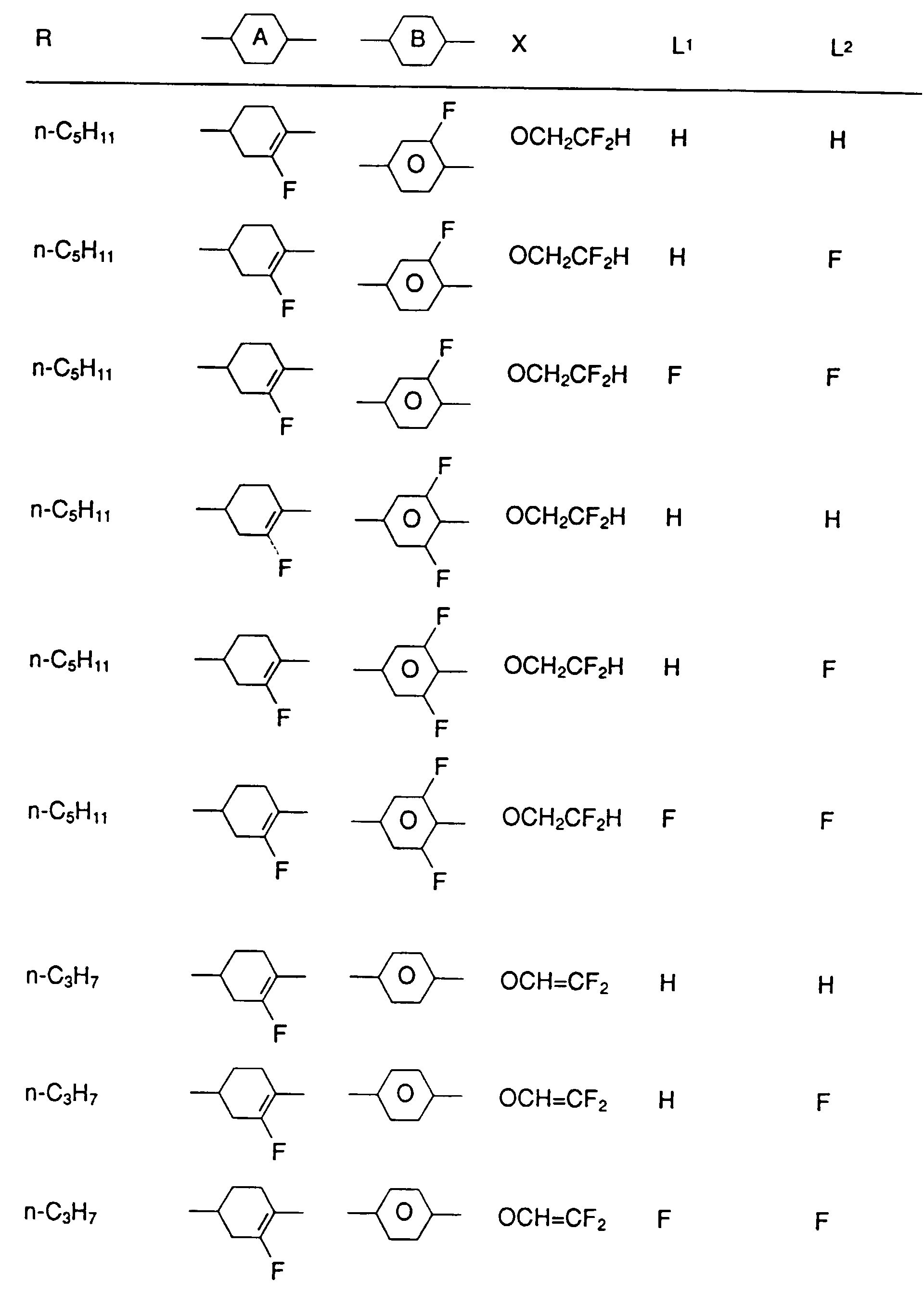

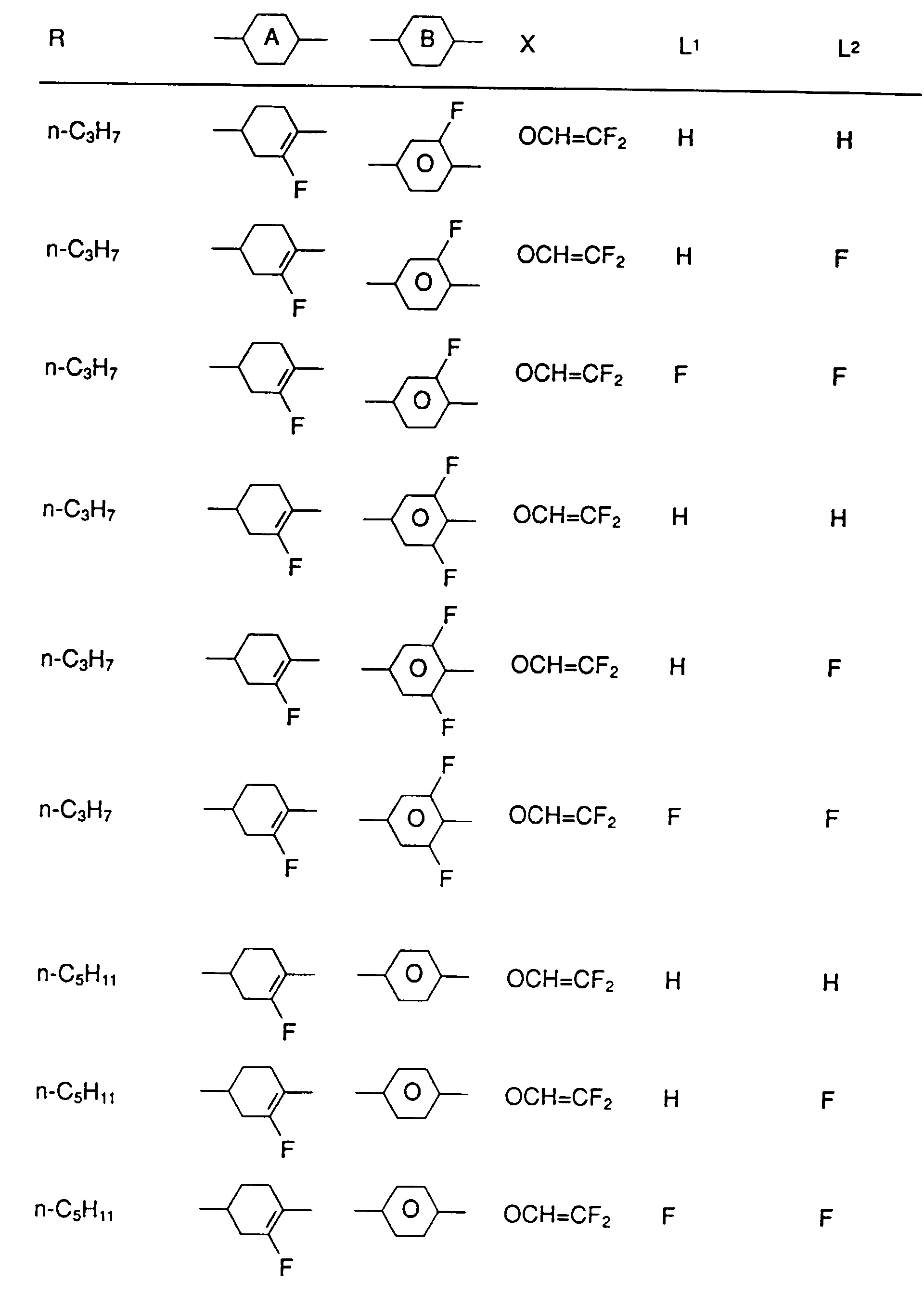

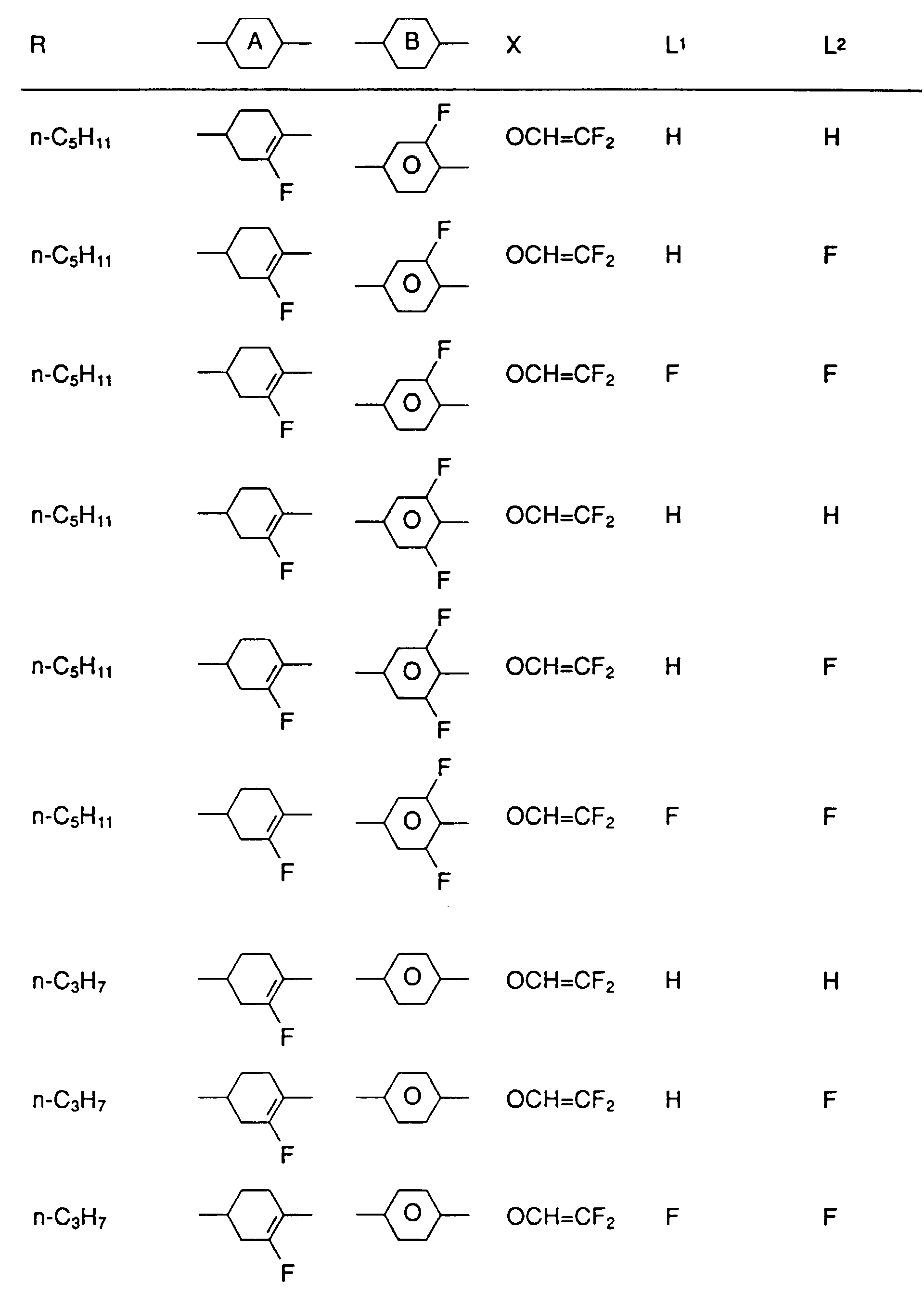

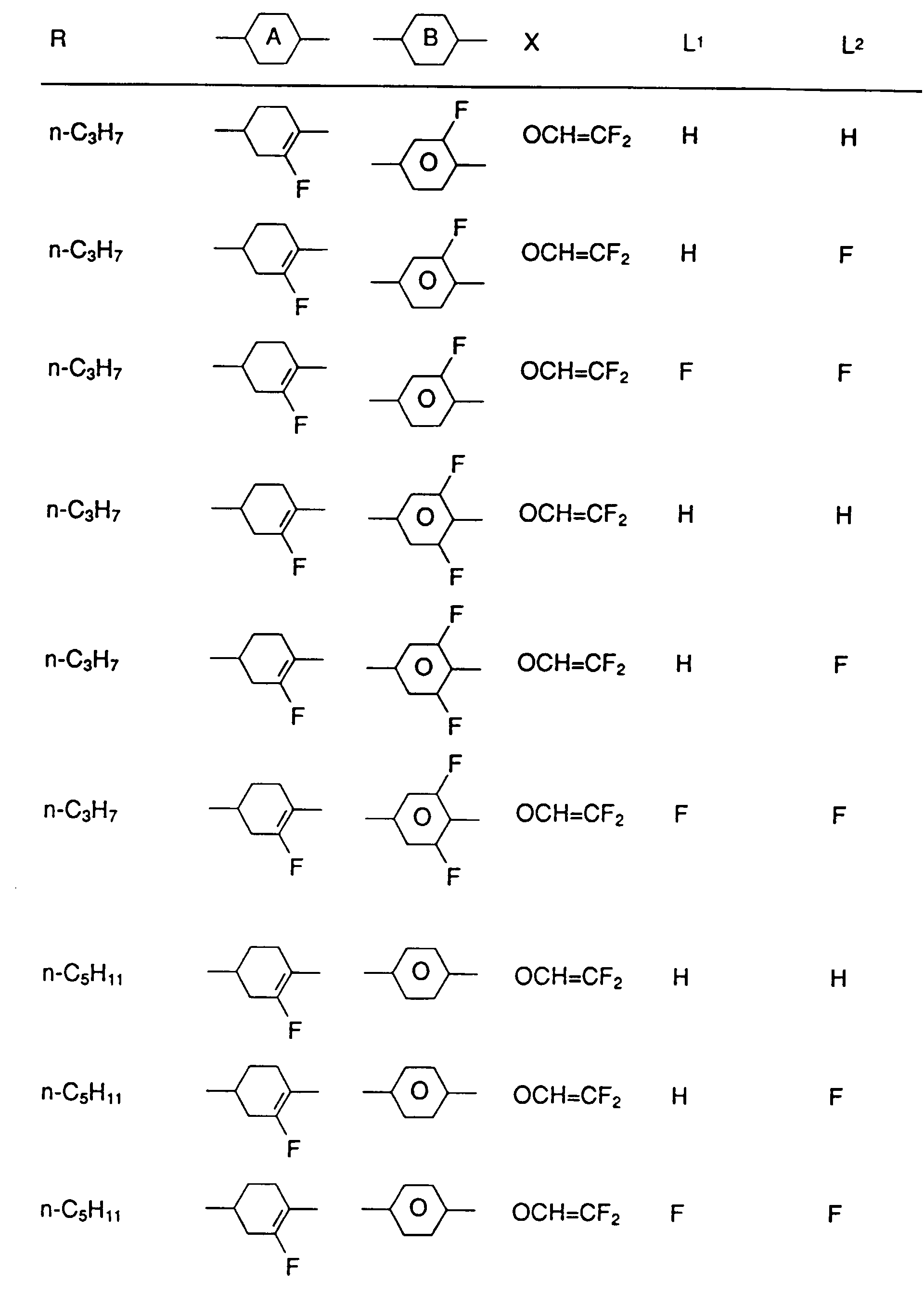

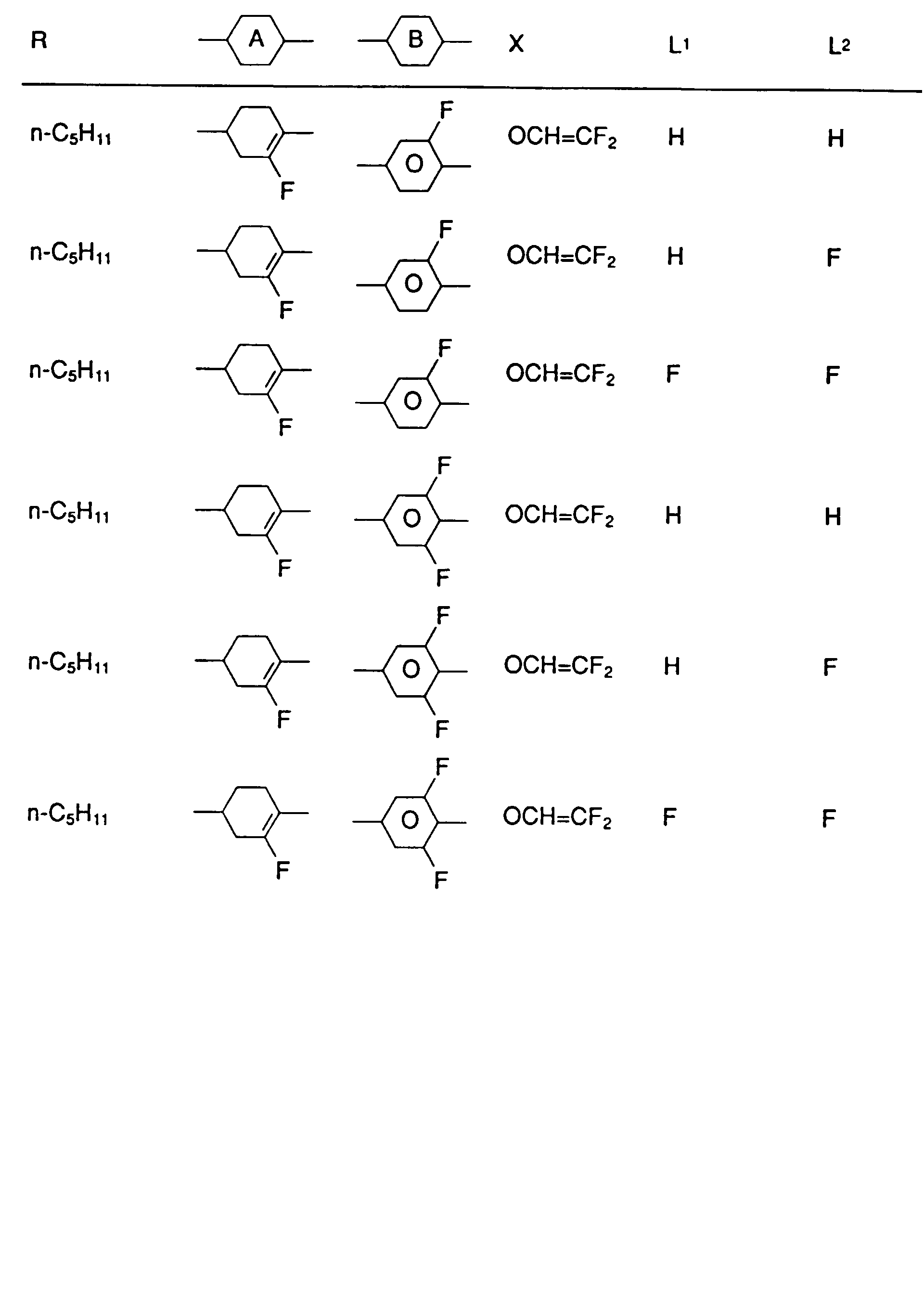

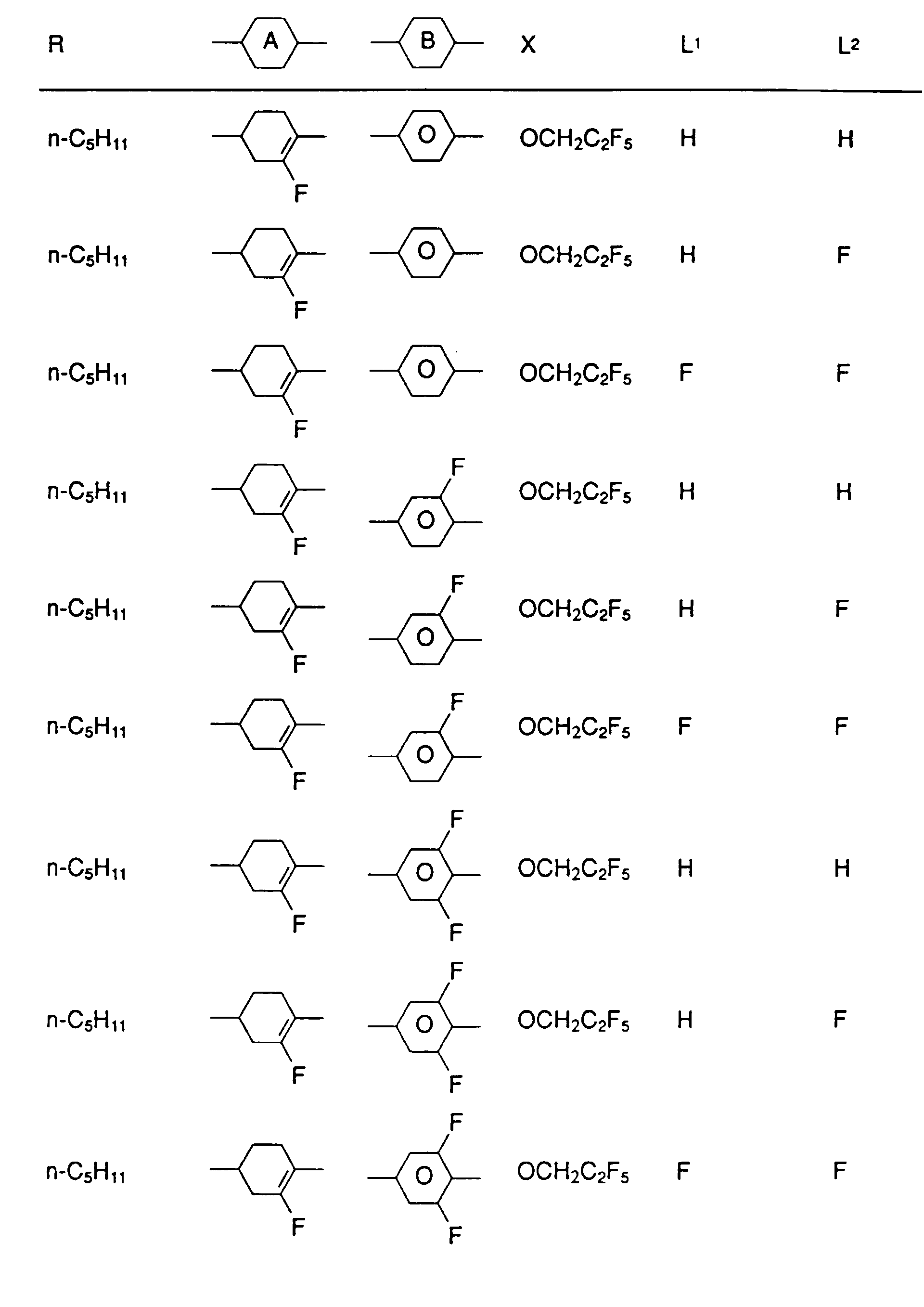

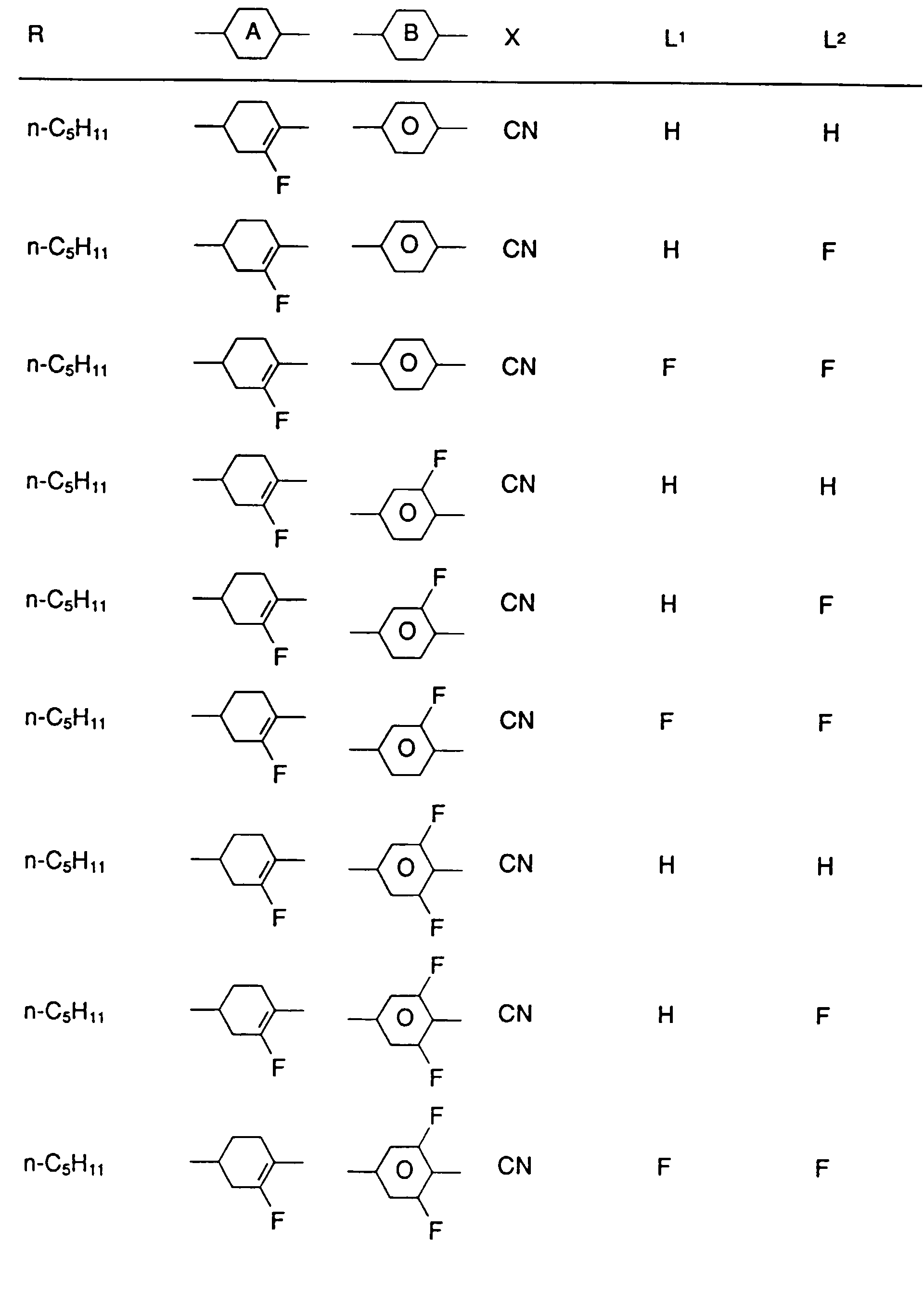

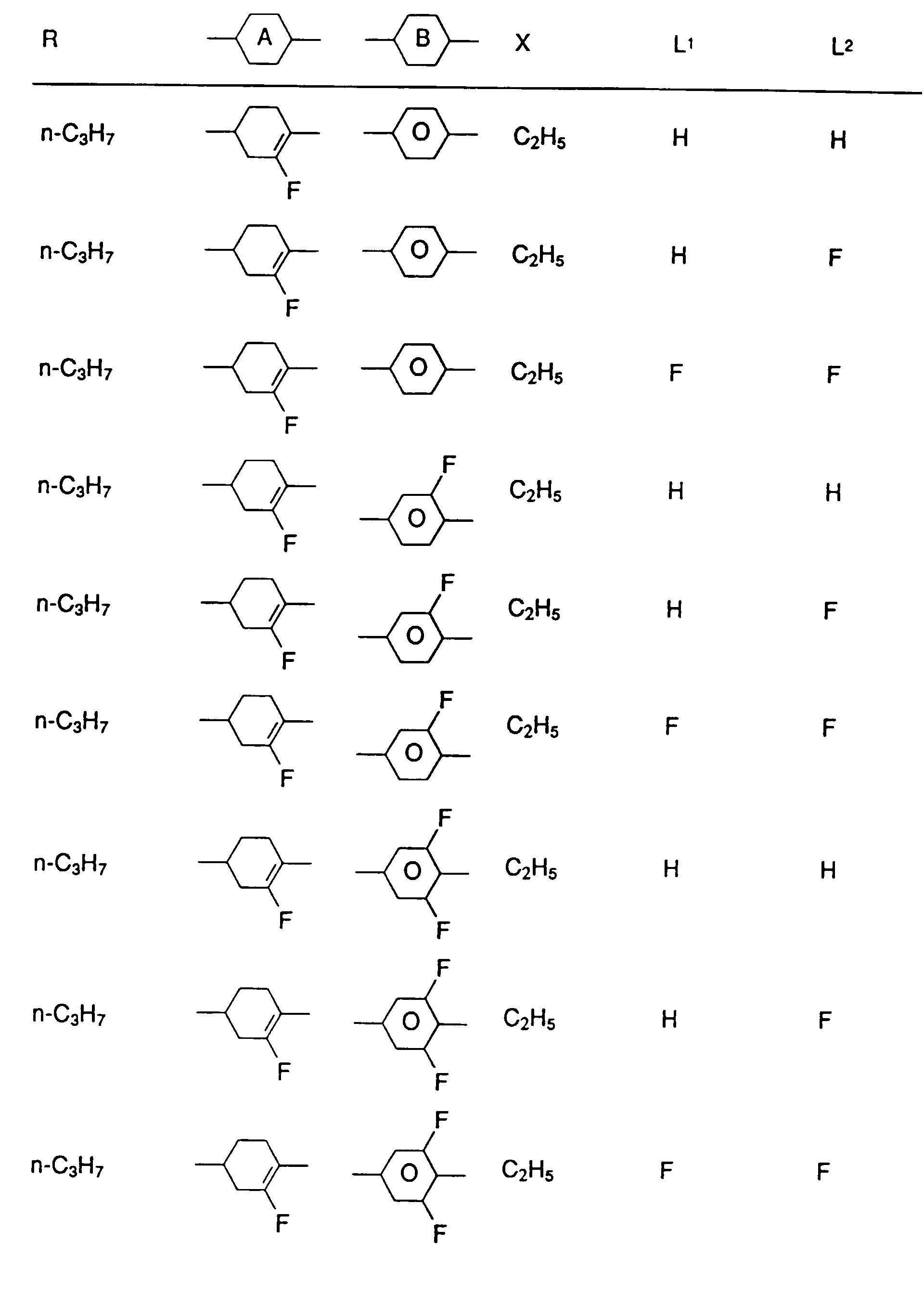

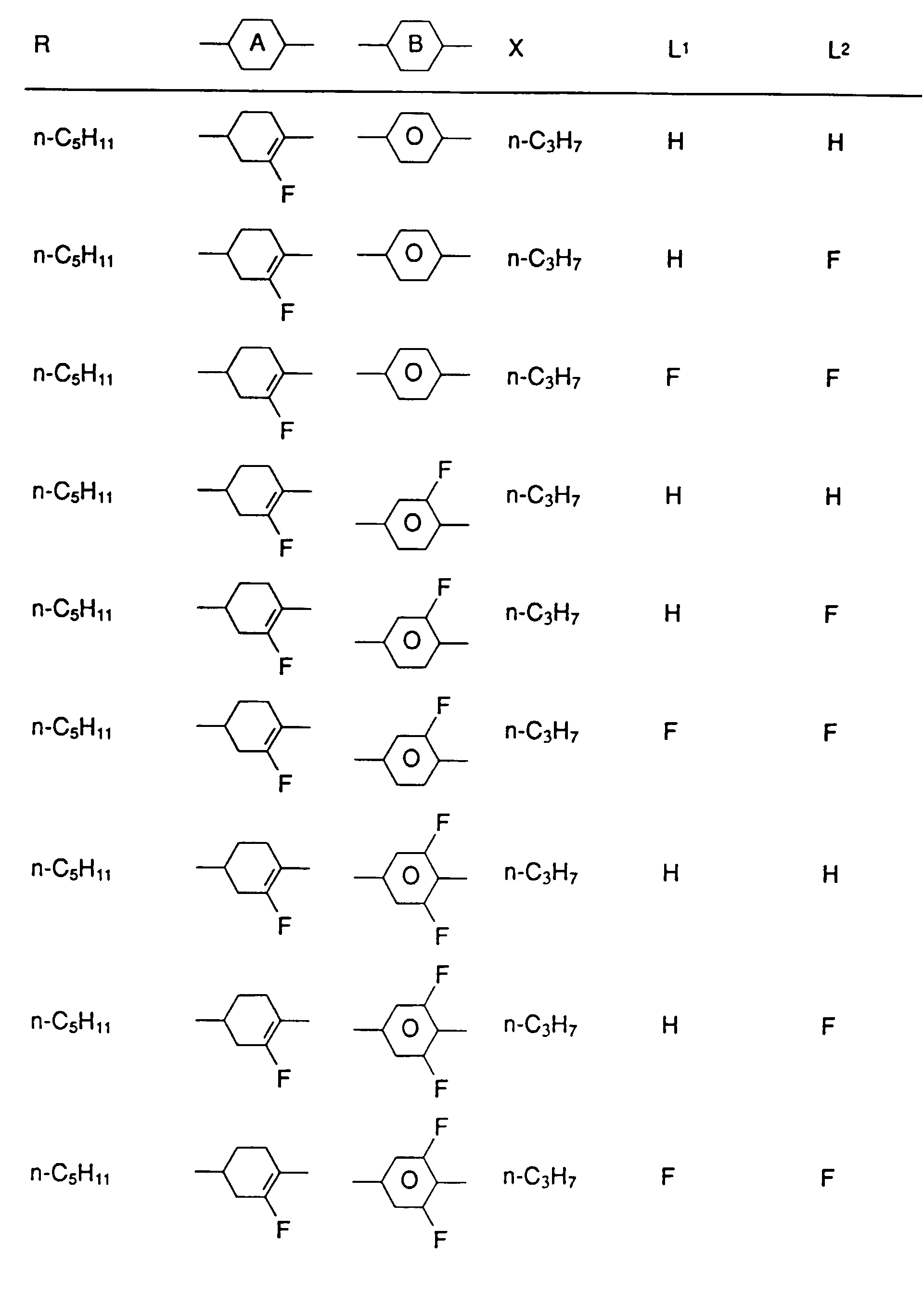

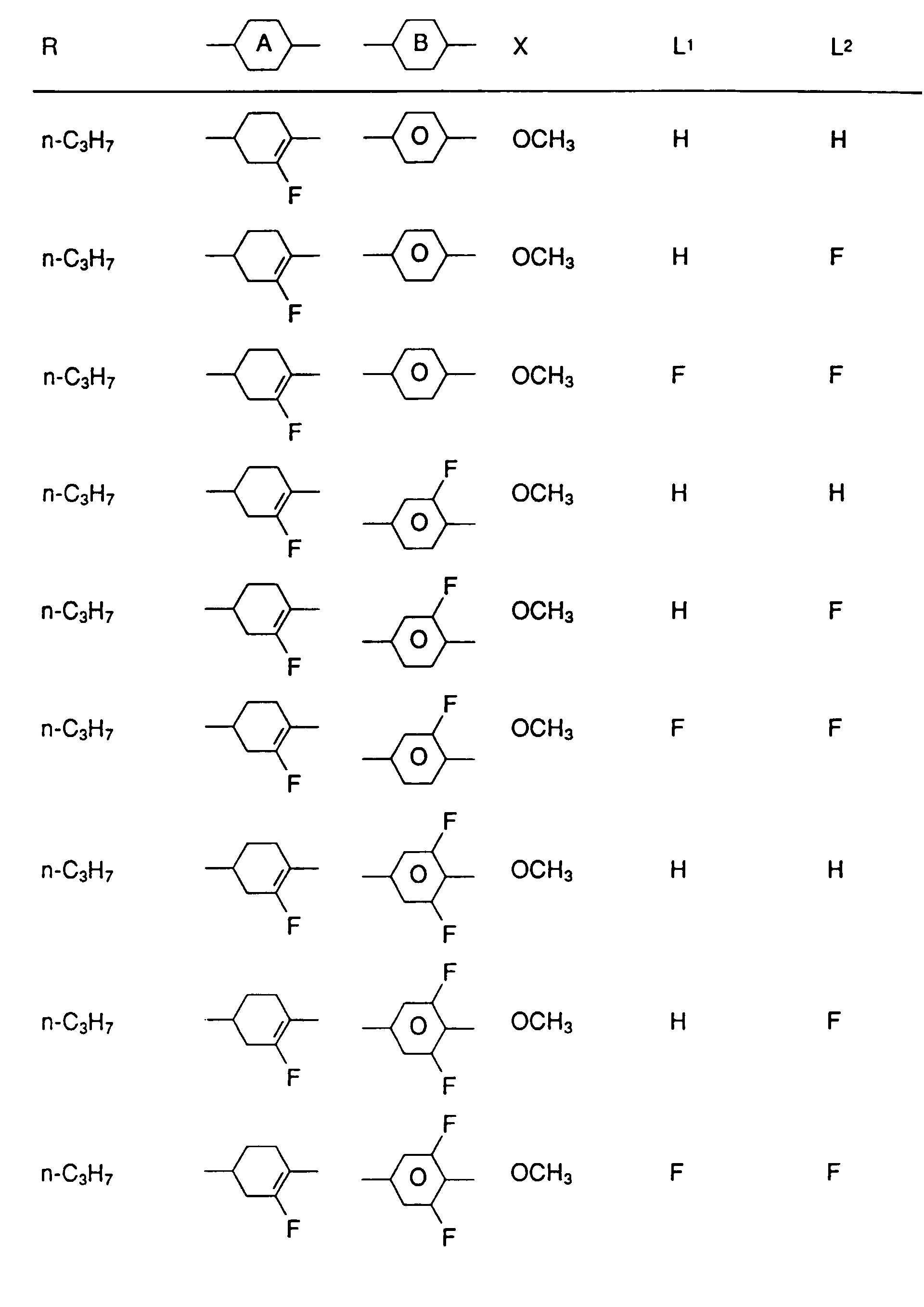

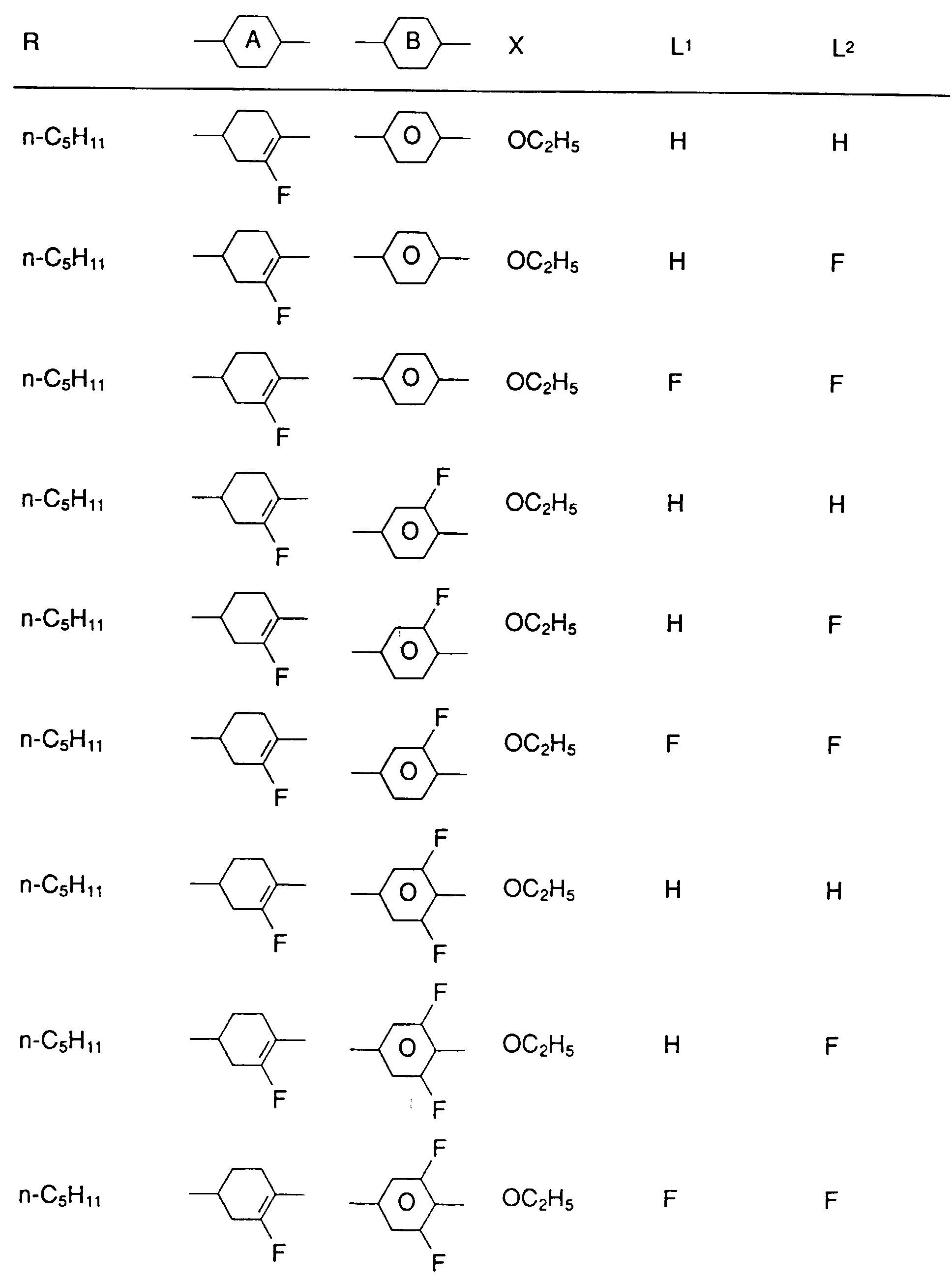

### Beispiel 2

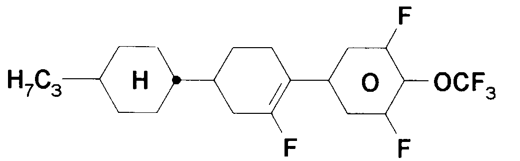

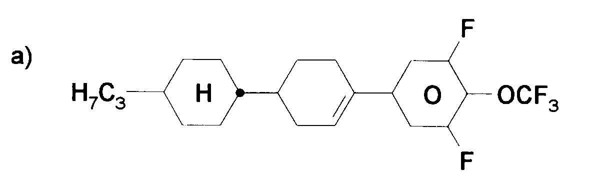

Zu einer Suspension von 0,25 mol Magnesiumspänen in 60 ml Ether gibt man zwei Tropfen Brom. Anschließend wird eine Lösung von 0,2 mol 3,5-Difluor-4-trifluormethoxybrombenzol in 60 ml Ether zugetropft. Man rührt 0,5 h nach und tropft dann bei 20-25 °C eine Lösung von 0,2 mol 4-trans-4-Propylcyclohexylcyclohexanon in 50 ml Ether zum Grignard-Reagenz. Es wird 2 h nachgerührt, auf 500 ml Wasser gegossen, angesäuert und mit Ether ausgeschüttelt. Die organische Phase wird zum Rückstand eingedampft. Der Rückstand wird in Toluol gelöst und mit p-Toluolsulfonsäure 5 Stunden unter Rückfluß am Wasserabscheider gekocht. Anschließend läßt man auf Raumtemperatur abkühlen, neutralisiert und arbeitet wie üblich auf. 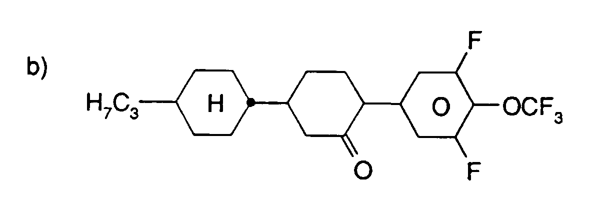 0,2 mol des Cyclohexenderivats aus Beispiel 1a) werden in 200 ml THF gelöst und mit 0,2 g Natriumborhydrid versetzt. Zu dem Gemisch werden 0,24 mol BF₃-Etherat zugetropft, wobei die Temperatur des Reaktionsgemisches 10 °C nicht übersteigen sollte. Anschließend wird 2 h bei Raumtemperatur nachgerührt. Nach der Hydrolyse wird das Reaktionsgemisch ohne weitere Aufarbeitung direkt mit schwefelsaurer Natriumdichromatlösung versetzt. Bei der Oxidation sollte die Reaktionstemperatur nicht höher als 15 °C sein. Man rührt eine Stunde bei Raumtemperatur nach und weitere 0,5 h bei 30 °C. Man filtriert ab, extrahiert mit Ether, wäscht mit Wasser und entfernt das Lösungsmittel im Vakuum. Der Rückstand wird chromatographisch gereinigt. 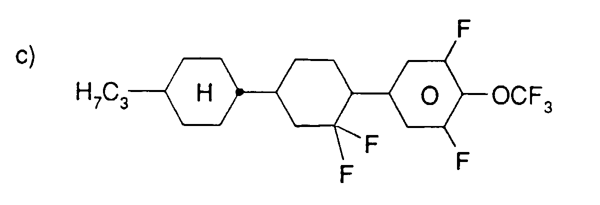 0,01 mol des Ketons aus b) werden mit 0,025 mol Diethylaminoschwefeltrifluorid über 15 h bei 60 °C behandelt. Danach wird die Mischung unter Rühren in Eiswasser eingetropft. Nach Zugabe von Ether wird die organische Phase abgetrennt und wie üblich aufgearbeitet.

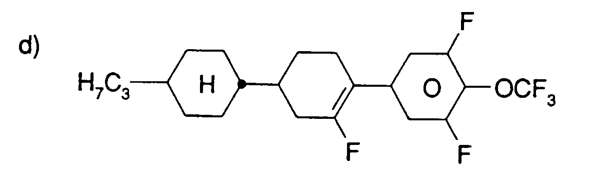

Zu 13,5 mol BuLi (15 % in n-Hexan) in 10 ml THF werden bei -20 °C 13,5 mol Diisopropylamin zugetropft. Anschließend wird die Lösung 10 min gerührt und zu einem Gemisch bestehend aus 10 ml THF und 13,5 mol der geminalen Difluorverbindung aus c) unter Schutzgas bei -40 °C zugetropft.

Das Gemisch wird 0,5 h zunächst bei -40 °C und anschließend über Nacht bei Raumtemperatur gerührt. Nach der Hydrolyse wird wie üblich aufgearbeitet.

## Patentansprüche

1. 2-Fluorcyclohexen-Derivate der Formel I, 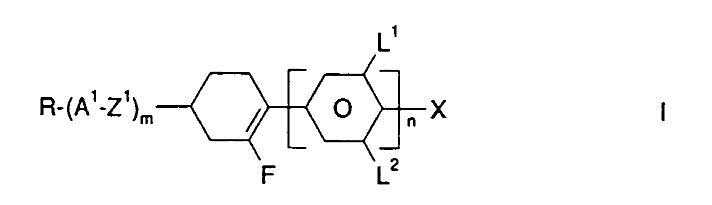 wobei
R ein unsubstituierter oder mit CN oder mit mindestens einem Halogen substituierter Alkyl- oder Alkenylrest mit bis zu 18 C-Atomen, worin eine oder mehrere nicht benachbarte CH₂-Gruppen durch einen Rest ausgewählt aus der Gruppe -O-, -S-, -CO-, -O-CO-, -CO-O- und -C≡C- ersetzt sein können,
A¹
a) einen 1,4-Phenylenrest, worin eine oder zwei CH-Gruppen durch N ersetzt sein können,
b) einen 1,4-Cyclohexylenrest, worin eine oder zwei nicht benachbarte CH₂-Gruppen durch -O- oder -S- ersetzt sein können,
c) einen 1,4-Cyclohexenylen-, einen Piperidin-1,4-diyl-, einen 1 ,4-Bicyclo[2,2,2]-octylen- oder einen Naphthalin-2,6-diylrest
wobei die Reste a) und b) ein oder mehrfach durch Halogenatome, Cyano- und/oder Methylgruppen substituiert sein können,
Z¹ -CH₂CH₂-, -CO-O-, -O-CO-, -CH=CH-, -OCH₂-, -CH₂O-, -C≡C-, -(CH₂)₄-, -CH=CH-CH₂CH₂- oder eine Einfachbindung,
X Alkyl oder Alkoxy mit 1-12 C-Atomen, NCS oder Q-Y, wobei Q -O-, -S- oder eine Einfachbindung und Y CN, F, Cl oder halogeniertes Alkyl oder Alkenyl mit 1-5 C-Atomen ist,
L¹ und L² jeweils unabhängig voneinander H oder F,
m 0, 1 oder 2 und
n 0, 1 oder 2, wobei m + n ≥ 1 ist,
bedeuten, mit der Maßgabe, daß im Fall m = 0, n = 1 und X = Alkyl, Alkoxy, F, Cl oder CN L¹ und L² Fluor bedeuten.

2. 2-Fluorcyclohexen-Derivate nach Anspruch 1 der Formel 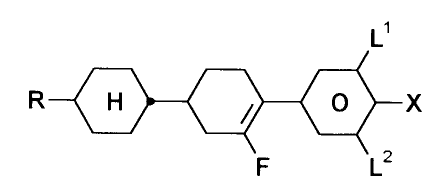 worin R, X, L¹ und L² die in Anspruch 1 angegebene Bedeutung haben.

3. 2-Fluorcyclohexen-Derivate nach Anspruch 1 der Formel 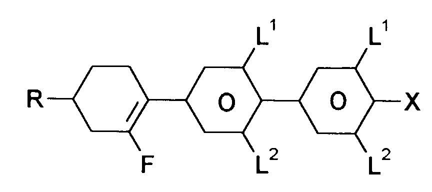 worin R, X, L¹ und L² die in Anspruch 1 angegebene Bedeutung haben.

4. 2-Fluorcyclohexen-Derivate nach Anspruch 1 der Formel 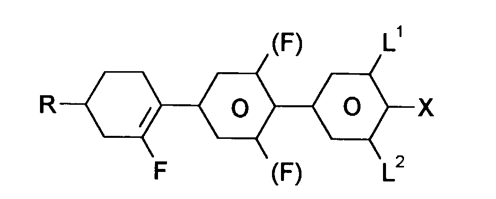 worin R, X, L¹ und L² die in Anspruch 1 angegebene Bedeutung haben.

5. Verbindungen der Formel I nach Anspruch 1, worin L¹ und L² Fluor bedeuten.

6. Verbindungen der Formel I nach Anspruch 1, worin X CN, F, Cl, OCF₃, OCHF₂, CF₃, OCHFCF₃, OCH₂CF₃, OCH=CF₂ oder OCF=CF₂ bedeuten.

7. Verbindungen der Formel I nach Anspruch 1, worin m + n = 2 ist.

8. Verbindungen der Formel II 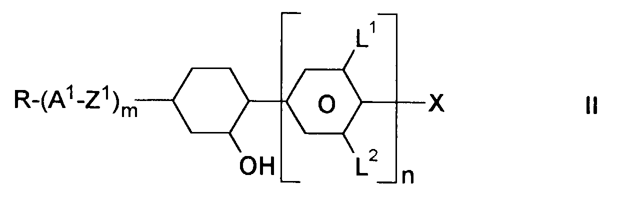 worin R, A¹, Z¹, X und m die in Anspruch 1 angegebene Bedeutung haben, n 1 oder 2 und L¹ und L² Fluor bedeuten.

9. Verbindungen nach Anspruch 8 der Formel 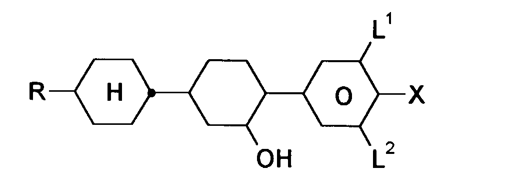 worin R und X die in Anspruch 1 angegebenen Bedeutungen haben und L¹ und L² Fluor bedeuten.

10. Verbindungen nach Anspruch 8 der Formel 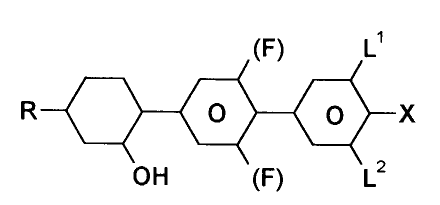 worin R und X die in Anspruch 1 angegebenen Bedeutungen haben und L¹ und L² Fluor bedeuten.

11. Verbindungen der Formel III 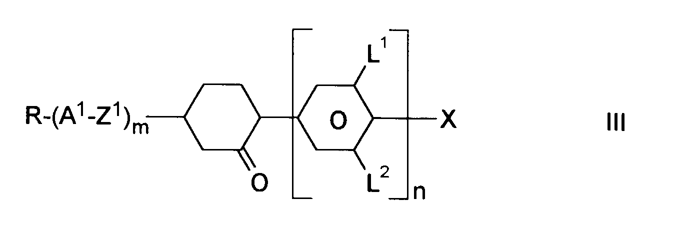 worin R, A¹, Z¹, X, L¹, L² und m die in Anspruch 1 angegebene Bedeutung haben und n 1 oder 2 bedeutet.

12. Verwendung der Verbindungen der Formel I nach Anspruch 1 als Komponenten flüssigkristalliner Medien für elektrooptische Anzeigeelemente.

13. Flüssigkristallines Medium mit mindestens zwei Komponenten, dadurch gekennzeichnet, daß eine oder mehrere Komponenten eine Verbindung der Formel I nach Anspruch 1 sind.

14. Elektrooptisches Anzeigeelement, dadurch gekennzeichnet, daß es als Dielektrikum ein flüssigkristallines Medium nach Anspruch 13 enthält.

15. Matrix-Flüssigkristallanzeigeelement nach Anspruch 14, dadurch gekennzeichnet, daß es als Dielektrikum ein flüssigkristallines Medium nach Anspruch 13 enthält.

## Claims

1. 2-Fluorocyclohexene derivatives of the formula I 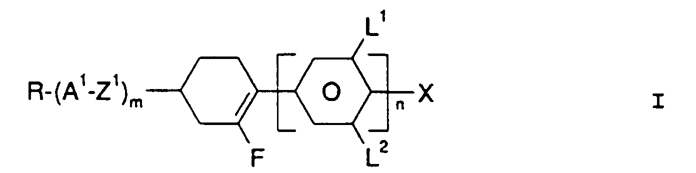 where
R is an alkyl or alkenyl radical having up to 18 carbon atoms which is unsubstituted or substituted by CN or by at least one halogen and in which one or more non-adjacent CH₂ groups can be replaced by a radical selected from the group consisting of -O-, -S-, -CO-, -O-CO-, -CO-O- and
A¹
a) is a 1,4-phenylene radical, in which one or two CH groups can be replaced by N,
b) is a 1,4-cyclohexylene radical, in which one or two non-adjacent CH₂ groups can be replaced by -O- or -S-,
c) is a 1,4-cyclohexenylene, piperidine-1,4-diyl, 1,4-bicyclo[2.2.2]octylene or naphthalene-2,6-diyl radical,
where the radicals a) and b) can be monosubstituted or polysubstituted by halogen atoms or cyano and/or methyl groups,
Z¹ is -CH₂CH₂-, -CO-O-, -O-CO-, -CH=CH-, -OCH₂-, -CH₂O-, -C≡C-, -(CH₂)₄-, -CH=CH-CH₂CH₂- or a single bond,
X is alkyl or alkoxy having 1 to 12 carbon atoms, NCS or Q-Y, where Q is -O-, -S- or a single bond, and Y is CN, F, Cl or halogenated alkyl or alkenyl having 1 to 5 carbon atoms,
L¹ and L² are each, independently of one another, H or F,
m is 0, 1 or 2, and
n is 0, 1 or 2, where m + ≥ 1,
with the proviso that, in the case where m = 0, n = 1 and X = alkyl, alkoxy, F, Cl or CN, L¹ and L² are fluorine.

2. 2-Fluorocyclohexene derivatives according to Claim 1 of the formula 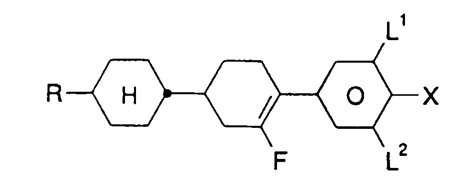 in which R, X, L¹ and L² are as defined in Claim 1.

3. 2-Fluorocyclohexene derivatives according to Claim 1 of the formula 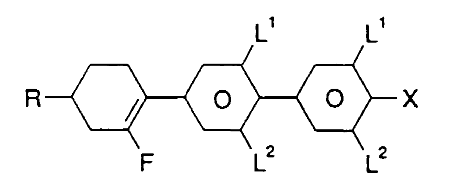 in which R, X, L¹ and L² are as defined in Claim 1.

4. 2-Fluorocyclohexene derivatives according to Claim 1 of the formula 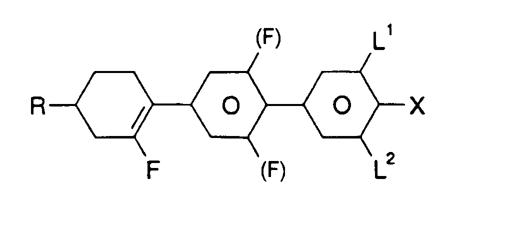 in which R, X, L¹ and L² are as defined in Claim 1.

5. Compounds of the formula I according to Claim 1 in which L¹ and L² are fluorine.

6. Compounds of the formula I according to Claim 1 in which X is CN, F, Cl, OCF₃, OCHF₂, CF₃, OCHFCF₃, OCH₂CF₃, OCH=CF₂ or OCF=CF₂.

7. Compounds of the formula I according to Claim 1 in which m + n = 2.

8. Compounds of the formula II 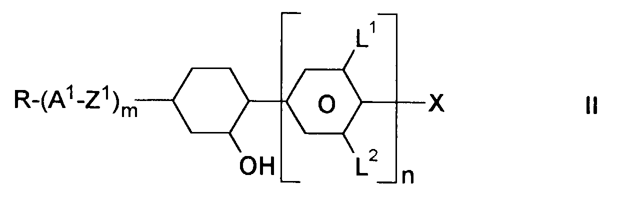 in which R, A¹, Z¹, X and m are as defined in Claim 1, n is 1 or 2, and L¹ and L² are fluorine.

9. Compounds according to Claim 8, of the formula 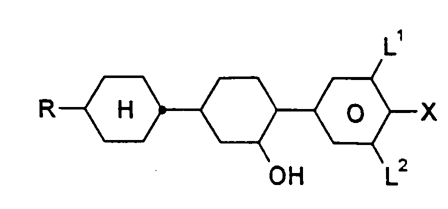 in which R and X are as defined in Claim 1, and L¹ and L² are fluorine.

10. Compounds according to Claim 8, of the formula 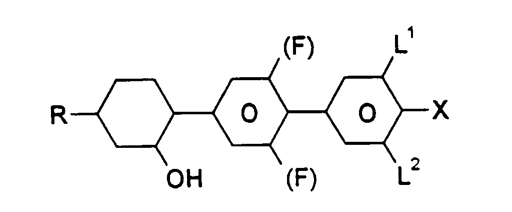 in which R and X are as defined in Claim 1, and L¹ and L² are fluorine.

11. Compounds of the formula III 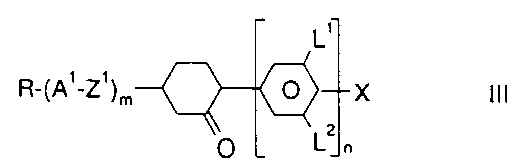 in which R, A¹, Z¹, X, L¹, L² and m are as defined in Claim 1, and n is 1 or 2.

12. Use of the compounds of the formula I according to Claim 1 as components of liquid-crystalline media for electro-optical display elements.

13. Liquid-crystalline medium having at least two components, characterized in that one or more components are a compound of the formula I according to Claim 1.

14. Electro-optical display element, characterized in that it contains, as dielectric, a liquid-crystalline medium according to Claim 13.

15. Matrix liquid-crystal display element according to Claim 14, characterized in that it contains, as dielectric, a liquid-crystalline medium according to Claim 13.

## Revendications

1. Dérivés de 2-fluorocyclohexène de formule I, 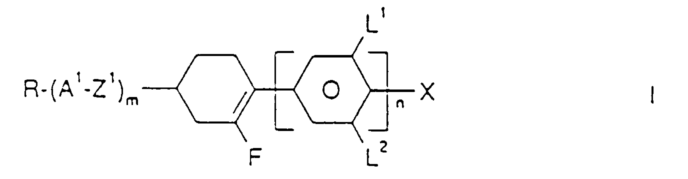 dans laquelle
R représente un radical alkyle ou alcényle non substitué ou substitué par CN ou par au moins un halogène et ayant jusqu'à 18 atomes de carbone, dans lequel un ou plusieurs groupes CH₂ non voisins peuvent être remplacés par un radical choisi dans le groupe -O-, -S-, -CO-, -O-CO-, -CO-O- et -C≡C-,
A¹ représente
a) un radical 1,4-phénylène, dans lequel un ou deux groupes CH peuvent être remplacés par N,
b) un radical 1,4-cyclohexylène, dans lequel un ou deux groupes CH₂ non voisins peuvent être remplacés par -O- ou -S-,
c) un radical 1,4-cyclohexénylène, pipéridine-1,4-diyle, 1,4-bicyclo[2,2,2]-octylène ou naphtalène-2,6-diyle, les radicaux a) et b) pouvant être mono- ou polysubstitués par des atomes d'halogène, des groupes cyano et/ou méthyle,
Z¹ représente -CH₂CH₂-, -CO-O-, -O-CO-, -CH=CH-, -OCH₂-, -CH₂O-, -C≡C-, -(CH₂)₄-, -CH=CH-CH₂CH₂- ou une liaison simple,
X représente r un alkyle ou un alcoxy en C₁ à C₁₂, , NCS ou Q-Y, où Q représente -O-, -S- ou une liaison simple et Y représente CN, F, Cl ou un alkyle ou alcényle halogéné en C₁ à C₅ ,
L¹ et L² représentent chacun indépendamment l'un de l'autre H ou F,
m vaut 0, 1 ou 2 et
n vaut 0, 1 ou 2 , avec m + n ≥ 1,
sous réserve que dans le cas où m = 0, n = 1 et X = alkyle, alcoxy, F, Cl ou CN, L¹ et L² représentent un fluor.

2. Dérivés de 2-fluorocyclohexène selon la revendication 1 de formule 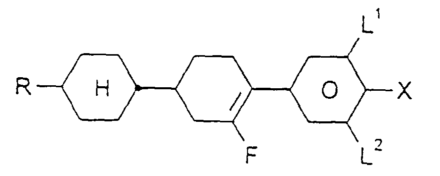 dans laquelle R, X, L¹ et L², ont la signification donnée dans la revendication 1.

3. Dérivés de 2-fluorocyclohexène selon la revendication 1 de formule 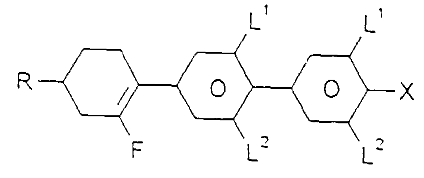 dans laquelle R, X, L¹ et L² ont la signification donnée dans la revendication 1.

4. Dérivés de 2-fluorocyclohexène selon la revendication 1 de formule 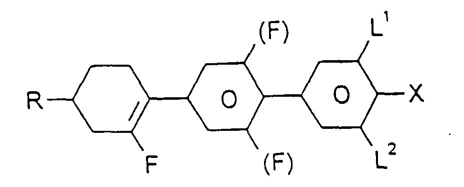 dans laquelle R, X, L¹ et L² ont la signification donnée dans la revendication 1.

5. Composés de formule I selon la revendication 1, dans laquelle L¹ et L² représentent un fluor.

6. Composés de formule I selon la revendication 1, dans laquelle X représente CN, F, Cl, OCF₃, OCHF₂, CF₃, OCHFCF₃, OCH₂CF₃, OCH=CF₂ ou OCF=CF₂.

7. Composés de formule I selon la revendication 1, dans laquelle m + n = 2.

8. Composés de formule II 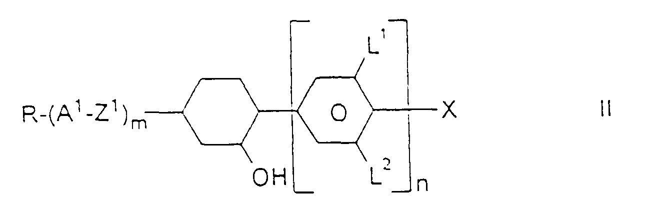 dans laquelle R, A¹, Z¹, X et m ont la signification donnée dans la revendication 1, n vaut 1 ou 2 et L¹ et L² représentent un fluor.

9. Composés selon la revendication 8 de formule 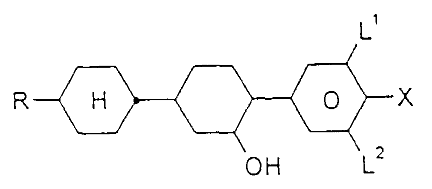 dans laquelle R et X ont les significations données dans la revendication 1 et L¹ et L² représentent un fluor.

10. Composés selon la revendication 8 de formule 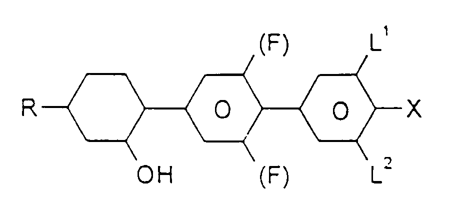 dans laquelle R et X ont les significations données dans la revendication 1 et L¹ et L² représentent un fluor.

11. Composés de formule III 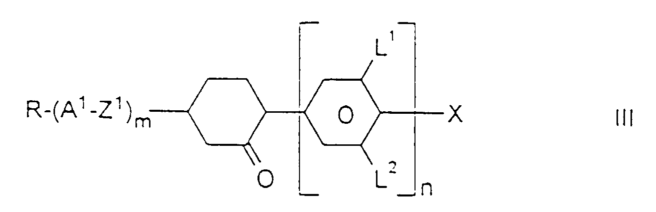 dans laquelle R, A¹, Z¹, X, L¹ et L² ont la signification donnée dans la revendication 1 et n vaut 1 ou 2.

12. Utilisation des composés de formule I selon la revendication 1 comme composants de milieux à cristaux liquides pour éléments d'affichage électro-optiques.

13. Milieu à cristaux liquides ayant au moins deux composants, caractérisé en ce qu'un ou plusieurs composants représente(nt) un composé de formule I selon la revendication 1.

14. Elément d'affichage électro-optique, caractérisé en ce qu'il contient comme diélectrique un milieu à cristaux liquides selon la revendication 13.

15. Elément d'affichage à cristaux liquides à matrice selon la revendication 14, caractérisé en ce qu'il contient comme diélectrique un milieu à cristaux liquides selon la revendication 13.
